# EUROPÄISCHE PATENTSCHRIFT

(11) **EP 1 981 903 B1**
(45) Veröffentlichungstag und Bekanntmachung des Hinweises auf die Patenterteilung: **21.10.2009**
(21) Anmeldenummer: 07703035.1
(22) Anmeldetag: 25.01.2007
(51) Int. Cl.: C07K 11/02, A61K 38/15

(54) **ASPARAGIN-10-SUBSTITUIERTE NONADEPSIPEPTIDE**
ASPARAGINE-10-SUBSTITUTED NONADEPSIPEPTIDES
NONADEPSIPEPTIDE 10 SUBSTITUE PAR DE L'ASPARAGINE

(30) Priorität: 25.01.2006 DE 102006003443
(43) Veröffentlichungstag der Anmeldung: 22.10.2008
(73) Patentinhaber: AiCuris GmbH & Co. KG, 42117 Wuppertal (DE)
(72) Erfinder: VON NUSSBAUM, Franz, 61462 Königstein (DE); BRUNNER, Nina, 45279 Essen (DE); ENDERMANN, Rainer, 42113 Wuppertal (DE); TELSER, Joachim, 42115 Wuppertal (DE); SCHUHMACHER, Joachim, 42113 Wuppertal (DE); ANLAUF, Sonja, 42115 Wuppertal (DE)
(74) Vertreter: Scheuermann, Erik
(86) Internationale Anmeldenummer: PCT/EP2007/000645
(87) Internationale Veröffentlichungsnummer: WO 2007/085456

(56) Entgegenhaltungen:
- WO-A-20/04099239
- BLACKBURN R K ET AL: "APPLICATION OF AN IMMOBILIZED DIGESTIVE ENZYME ASSAY TO MEASURE CHEMICAL AND ENZYMATIC HYDROLYSIS OF THE CYCLIC PEPTIDE ANTIBIOTIC LYSOBACTIN" DRUG METABOLISM AND DISPOSITION, WILLIAMS AND WILKINS., BALTIMORE, MD, US, Bd. 21, Nr. 4, 1993, Seiten 573-579, XP009059530 ISSN: 0090-9556
- PALOMO C ET AL: "A concise synthesis of alpha-amino acid N-carboxy anhydrides of (2S,3S)-beta-substituted serines" TETRAHEDRON LETTERS, ELSEVIER, AMSTERDAM, NL, Bd. 42, Nr. 51, 17. Dezember 2001 (2001-12-17), Seiten 8955-8957, XP004323292 ISSN: 0040-4039
- EGNER B J ET AL: "Monitoring the solid phase synthesis of analogues of lysobactin and the katanosins using in situ MALDI-TOF MS" TETRAHEDRON, ELSEVIER SCIENCE PUBLISHERS, AMSTERDAM, NL, Bd. 53, Nr. 41, 13. Oktober 1997 (1997-10-13), Seiten 14021-14030, XP004618631 ISSN: 0040-4020
- MAKI H ET AL.: "Katanosin B and Lusbacin A3, Inhibitors of Peptidoglycan Synthesis in Methicillin-Resistant Staphylococcus aureus" ANTIMICROBIAL AGENTS AND CHEMOTHERAPY, Bd. 45, Nr. 6, Juni 2001 (2001-06), Seiten 1823-1827, XP002430697

## Beschreibung

Die Erfindung betrifft Nonadepsipeptide und Verfahren zu ihrer Herstellung sowie ihre Verwendung zur Herstellung von Arzneimitteln zur Behandlung und/oder Prophylaxe von Krankheiten, insbesondere bakteriellen Infektionskrankheiten.

Die bakterielle Zellwand wird durch eine Reihe von Enzymen synthetisiert (Zellwandbiosynthese) und ist essentiell für das Überleben bzw. die Vermehrung von Mikroorganismen. Die Struktur dieses Makromoleküls ebenso wie die an ihrer Synthese beteiligten Proteine sind innerhalb der Bakterien stark konserviert. Aufgrund ihrer essentiellen Natur und Einheitlichkeit ist die Zellwandbiosynthese ein idealer Angriffspunkt für neue Antibiotika (D.W. Green, The bacterial cell wall as a source of antibacterial targets, Expert Opin. Ther. Targets, 2002, 6, 1-19).

Vancomycin und Penicilline sind Inhibitoren der bakteriellen Zellwandbiosynthese und stellen erfolgreiche Beispiele für die antibiotische Potenz dieses Wirkprinzips dar. Sie werden seit mehreren Jahrzehnten in der Klinik zur Behandlung von bakteriellen Infektionen, vor allem mit Gram-positiven Erregern eingesetzt. Durch das wachsende Auftreten von resistenten Keimen, z.B. Methicillin-resistenten Staphylokokken, Penicillin-resistenten Pneumokokken und Vancomycin-resistenten Enterokokken (F. Baquero, Gram-positive resistance: challenge for the development of new antibiotics, J. Antimicrob. Chemother., 1997, 39, Suppl A: 1-6; A.P. Johnson, D.M. Livermore, G.S. Tillotson, Antimicrobial susceptibility of Gram-positive bacteria: what's current, what's anticipated ?, J. Hosp. Infect., 2001, (49), Suppl A: 3-11) sowie jüngst auch erstmals Vancomycin-resistenten Staphylokokken (B. Goldrick, First reported case of VRSA in the United States, Am. J. Nurs., 2002, 102, 17) verlieren diese Substanzen zunehmend ihre therapeutische Wirksamkeit.

Die vorliegende Erfindung beschreibt eine neue Klasse von Zellwandbiosynthese-Inhibitoren ohne Kreuzresistenzen zu bekannten Antibiotika-Klassen, sowie Verfahren zu deren Herstellung.

Die semisynthetische Derivatisierung komplexer Naturstoffe setzt oft komplexe Schutzgruppenoperationen voraus (Haebich et al., Angew. Chem. Int. Ed., 2006, 45, 5072). Nur so gelingt die regio- und chemoselektive Adressierung der Derivatisierungsposition. In der vorliegenden Erfindung wurde überraschenderweise ein Verfahren gefunden, das ohne Schutzgruppenoperationen eine hoch regio- und chemoselektive Derivatisierung des komplexen Depsipeptids Lysobactin erlaubt.

Der Naturstoff Lysobactin und einige Derivate sind als antibakteriell wirksam beschrieben in US 4,754,018. Die Isolierung und antibakterielle Wirksamkeit von Lysobactin ist auch in EP-A-196 042 und JP 01132600 beschrieben. WO04/099239 beschreibt Derivate des Lysobactins mit antibakterieller Wirksamkeit.

Die antibakterielle Wirkung von Lysobactin und Katanosin A wird weiterhin beschrieben in O'Sullivan, J. et al., J. Antibiot. 1988, 41, 1740 - 1744, Bonner, D. P. et al., J. Antibiot. 1988, 41, 1745 - 1751, Shojl, J. et al., J. Antibiot. 1988, 41, 713 - 718 und Tymiak, A. A. et al., J. Org. Chem. 1989, 54, 1149 - 1157.

Eine Aufgabe der vorliegenden Erfindung ist es, alternative Verbindungen mit vergleichbarer oder verbesserter antibakterieller Wirkung, besserer Löslichkeit, höherer freier Fraktion im Blutplasma und besserer Verträglichkeit, z.B. geringerer Nephrotoxizität, zur Behandlung von bakteriellen Erkrankungen bei Menschen und Tieren zur Verfügung zu stellen.

Gegenstand der Erfindung sind Verbindungen der Formel 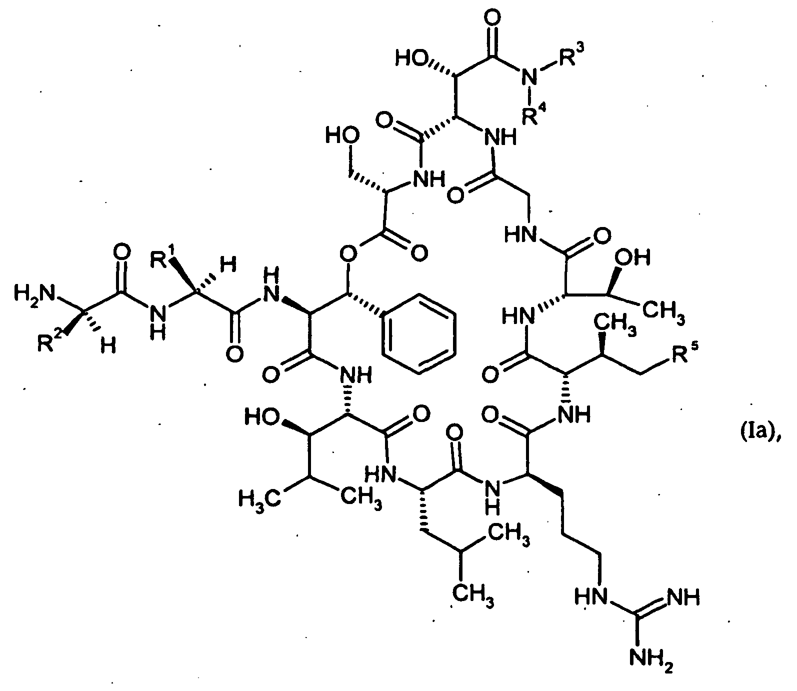 in welcher
- R¹: 2-Methylprop-1-yl, 2,2-Dimethylprop-1-yl, 1-Trimethylsilylmethyl oder 3-Pyridylmethyl bedeutet, wobei 3-Pyridylmethyl substituiert sein kann mit einem Substituenten Trifluormethyl,
- R²: 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
- R³: C₁-C₆-Alkyl bedeutet,
wobei Alkyl substituiert sein kann mit 1 bis 3 Substituenten, wobei die Substituenten unabhängig voneinander ausgewählt werden aus der Gruppe, bestehend aus Hydroxy, Amino, Hydroxycarbonyl, Aminocarbonyl, {[Bis(dimethylamino)methylen]amino}ethoxy, Phenyl, 5- oder 6-gliedriges Heterocyclyl, 5-oder 6-gliedriges Heteroaryl, Benzyloxycarbonyl und Benzyloxycarbonyl-amino,
- R⁴: Wasserstoff, C₁-C₄-Alkyl, Cyclopropyl oder Cyclopropylmethyl bedeutet,
- R⁵: Wasserstoff oder Methyl bedeutet,
und ihre Salze, ihre Solvate und die Solvate ihrer Salze.

Erfindungsgemäße Verbindungen sind die Verbindungen der Formeln (Ia), (I), (IIa) und (II) und deren Salze, Solvate, Solvate der Salze und Prodrugs, die von den Formeln (Ia), (I), (IIa) und (II) umfassten Verbindungen der nachfolgend genannten Formeln und deren Salze, Solvate, Solvate der Salze und Prodrugs sowie die von den Formeln (Ia), (I), (IIa) und (II) umfassten, nachfolgend als Ausführungsbeispiele genannten Verbindungen und deren Salze, Solvate, Solvate der Salze und Prodrugs, soweit es sich bei den von den Formeln (Ia), (I), (IIa) und (II) umfassten, nachfolgend genannten Verbindungen nicht bereits um Salze, Solvate, Solvate der Salze und Prodrugs handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können. Der Begriff Salze schließt auch Mischsalze der erfindungsgemäßen Verbindungen wie beispielsweise Mesylat-Trifluoracetat Salze ein.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl steht für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert*-Butyl, 2,2-Dimethylprop-1-yl, n-Pentyl und n-Hexyl.
Heterocyclyl steht für einen monocyclischen, heterocyclischen Rest mit 5 oder 6 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Beispielhaft und vorzugsweise seien genannt Tetrahydrofuran-2-yl, Tetrahydrofuran-3-yl, Pyrrolidin-2-yl, Pyrrolidin-3-yl, Pyrrolinyl, Piperidin-1-yl, Piperidin-2-yl, Piperidin-3-yl, Piperidin-4-yl, Tetrahydropyran-2-yl, Tetrahydropyran-3-yl, Tetrahydropyran-4-yl, Piperazin-1-yl, Piperazin-2-yl, Morpholin-2-yl, Morpholin-3-yl, Morpholin-4-yl, Thiomorpholin-2-yl, Thiomorpholin-3-yl und Thiomorpholin-4-yl.
Heteroaryl steht für einen aromatischen, monocyclischen Rest mit 5 oder 6 Ringatomen und bis zu 4, vorzugsweise bis zu 2 Heteroatomen aus der Reihe S, O und N, beispielhaft und vorzugsweise für Thien-2-yl, Thien-3-yl, Fur-2-yl, Fur-3-yl, Pyrrol-1-yl, Pyrrol-2-yl, Pyrrol-3-yl, Thiazol-2-yl, Thiazol-4-yl, Thiazol-5-yl, Oxazol-2-yl, Oxazol-4-yl, Oxazol-5-yl, Imidazol-1-yl, Imidazol-2-yl, Imidazol-4-yl, Imidazol-5-yl, Pyrid-2-yl, Pyrid-3-yl, Pyrid-4-yl, Pyrimid-2-yl, Pyrimid-4-yl, Pyrimid-5-yl, Pyrazin-2-yl, Pyrazin-3-yl, Pyridazin-3-yl und Pyridazin-4-yl.
Halogen steht für Fluor, Chlor, Brom und Jòd, bevorzugt Fluor und Chlor.

Bevorzugt sind Verbindungen der Formel (Ia), welche der Formel 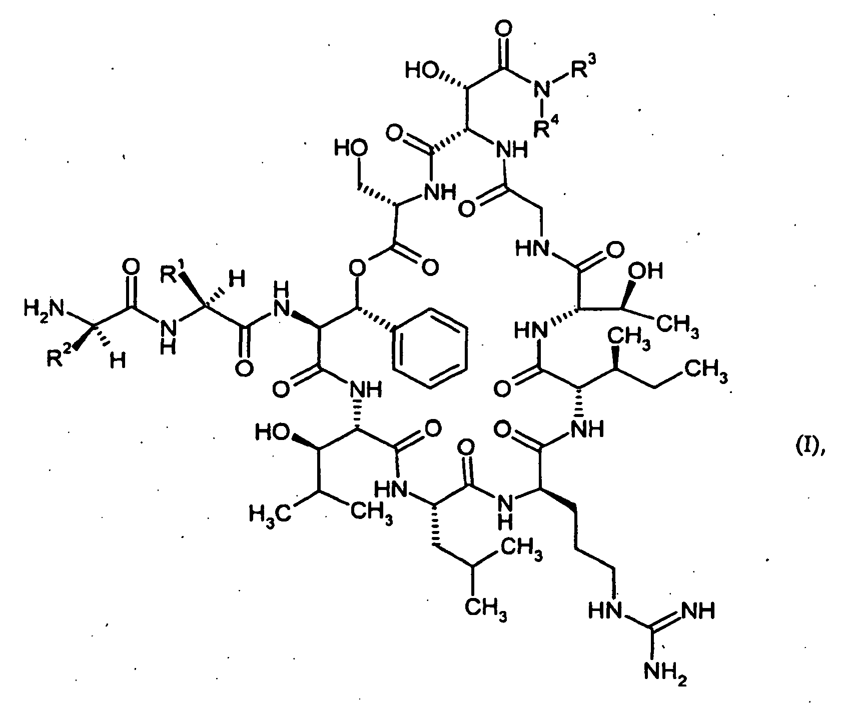 entsprechen,
in welcher
- R¹: 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
- R²: 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
- R³: C₁-C₆-Alkyl bedeutet,
wobei Alkyl substituiert sein kann mit 1 bis 3 Substituenten, wobei die Substituenten unabhängig voneinander ausgewählt werden aus der Gruppe, bestehend aus Hydroxy, Amino, Hydroxycarbonyl, Aminocarbonyl, {[Bis(dimethylamino)methylen]amino}ethoxy, Phenyl, 5- oder 6-gliedriges Heterocyclyl, 5-oder 6-gliedriges Heteroaryl, Benzyloxycarbonyl und Benzyloxycarbonylamino,
- R⁴: Wasserstoff, C₁-C₄-Alkyl, Cyclopropyl oder Cyclopropylmethyl bedeutet,
und ihre Salze, ihre Solvate und die Solvate ihrer Salze.

Bevorzugt sind auch Verbindungen der Formeln (la) und (I), in welchen
- R¹: 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
- R²: 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
- R³: C₁-C₄-Alkyl bedeutet,
wobei Alkyl substituiert sein kann mit 1 bis 2 Substituenten, wobei die Substituenten unabhängig voneinander ausgewählt werden aus der Gruppe, bestehend aus Hydroxy, Amino, Hydroxycarbonyl, Aminocarbonyl, {[Bis(dimethylamino)methylen]amino}ethoxy, Phenyl, Morpholin-4-yl, Pyrid-2-yl, Pyrid-3-yl, Pyrid-4-yl, Benzyloxycarbonyl und Benzyloxycarbonylamino,
- R⁴: Wasserstoff oder Methyl bedeutet,
und ihre Salze, ihre Solvate und die Solvate ihrer Salze.

Bevorzugt sind auch Verbindungen der Formeln (Ia) und (I), in welchen
- R¹: 2-Methylprop-1-yl bedeutet,
- R²: 2-Methylprop-1-yl bedeutet,
- R³: C₁-C₃-Alkyl bedeutet,
wobei Alkyl substituiert sein kann mit einem Substituenten, wobei der Substituent ausgewählt wird aus der Gruppe, bestehend aus Hydroxy, Amino, Hydroxycarbonyl, Aminocarbonyl, {[Bis(dimethylamino)methylen]amino}ethoxy, Phenyl, Morpholin-4-yl, Pyrid-3-yl, Benzyloxycarbonyl und Benzyloxycarbonylamino,
- R⁴: Wasserstoff oder Methyl bedeutet,
und ihre Salze, ihre Solvate und die Solvate ihrer Salze.

Bevorzugt sind auch Verbindungen der Formeln (Ia) und (I), in welchen
- R¹: 2,2-Dimethylprop-1-yl bedeutet,
- R²: 2,2-Dimethylprop-1-yl bedeutet,
- R³: C₁-C₃-Alkyl bedeutet,
wobei Alkyl substituiert sein kann mit einem Substituenten, wobei der Substituent ausgewählt wird aus der Gruppe, bestehend aus Hydroxy, Amino, Hydroxycarbonyl, Aminocarbonyl, {[Bis(dimethylamino)methylen]amino}ethoxy, Phenyl, Morpholin-4-yl, Pyrid-3-yl, Benzyloxycarbonyl und Benzyloxycarbonylamino,
- R⁴: Wasserstoff oder Methyl bedeutet,
und ihre Salze, ihre Solvate und die Solvate ihrer Salze.

Gegenstand der Erfindung sind ferner Verbindungen, welche der Formel 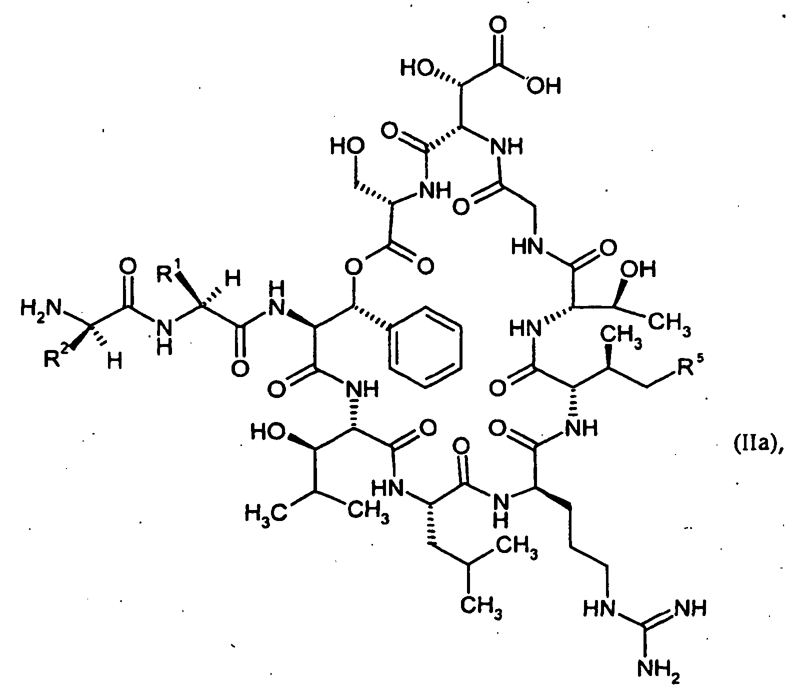 entsprechen,
in welcher
- R¹: 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
- R²: 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
- R⁵: Wasserstoff oder Methyl bedeutet,
und ihre Salze, ihre Solvate und die Solvate ihrer Salze.

Bevorzugt sind Verbindungen der Formel (IIa), welche der Formel 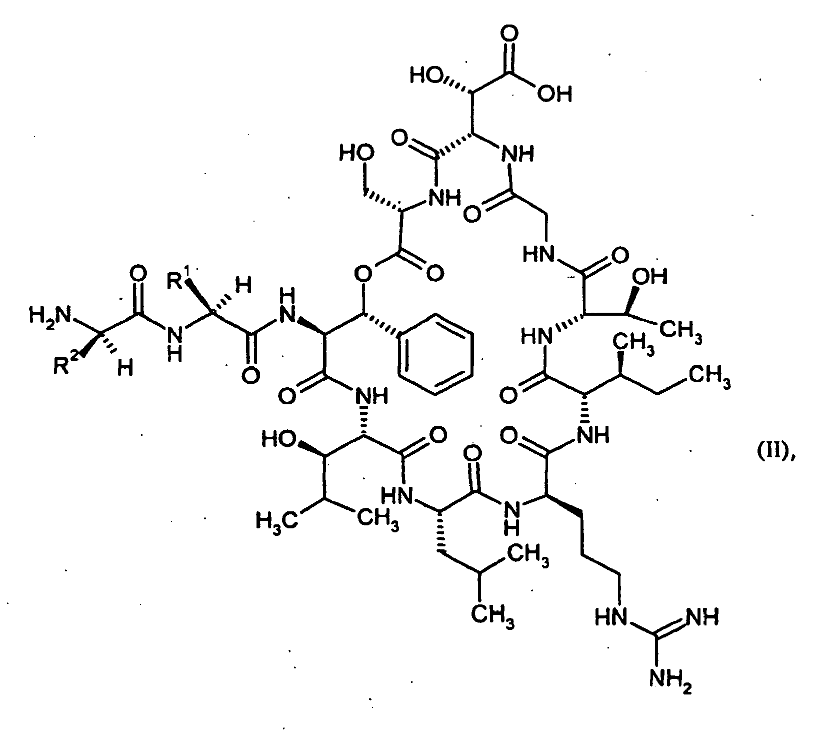 entsprechen,
in welcher
- R¹: 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
- R²: 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
und ihre Salze, ihre Solvate und die Solvate ihrer Salze.

Bevorzugt sind auch Verbindungen der Formeln (IIa) und (II), in welchen
- R¹: 2-Methylprop-1-yl bedeutet,
- R²: 2-Methylprop-1-yl bedeutet,
und ihre Salze, ihre Solvate und die Solvate ihrer Salze.

Bevorzugt sind auch Verbindungen der Formeln (IIa) und (II), in welchen
- R¹: 2,2-Dimethylprop-1-yl bedeutet,
- R²: 2,2-Dimethylprop-1-yl bedeutet,
und ihre Salze, ihre Solvate und die Solvate ihrer Salze.

Bevorzugt sind auch Verbindungen der Formeln (Ia), (I), (IIa) und (II), in welchen R¹ 2-Methylprop-1-yl bedeutet.

Bevorzugt sind auch Verbindungen der Formeln (Ia), (I), (IIa) und (II), in welchen R² 2-Methylprop-1-yl bedeutet.

Bevorzugt sind auch Verbindungen der Formeln (Ia), (I), (IIa) und (II), in welchen R¹ und R² 2,2-Dimethylprop-1-yl bedeuten.

Bevorzugt sind auch Verbindungen der Formeln (Ia), (I), (IIa) und (II), in welchen R¹ 1-Trimethylsilylmethyl und R² 3-Pyridylmethyl bedeutet.

Bevorzugt sind auch Verbindungen der Formeln (Ia) und (I), in welchen R⁴ Wasserstoff bedeutet.

Bevorzugt sind auch Verbindungen der Formeln (Ia) und (IIa), in welchen R⁵ Methyl bedeutet.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind auch Kombinationen von zwei oder mehreren der oben genannten Vorzugsbereiche.

Gegenstand der Erfindung ist weiterhin ein Verfahren zur Herstellung der Verbindungen der Formel (la), wobei Verbindungen der Formel 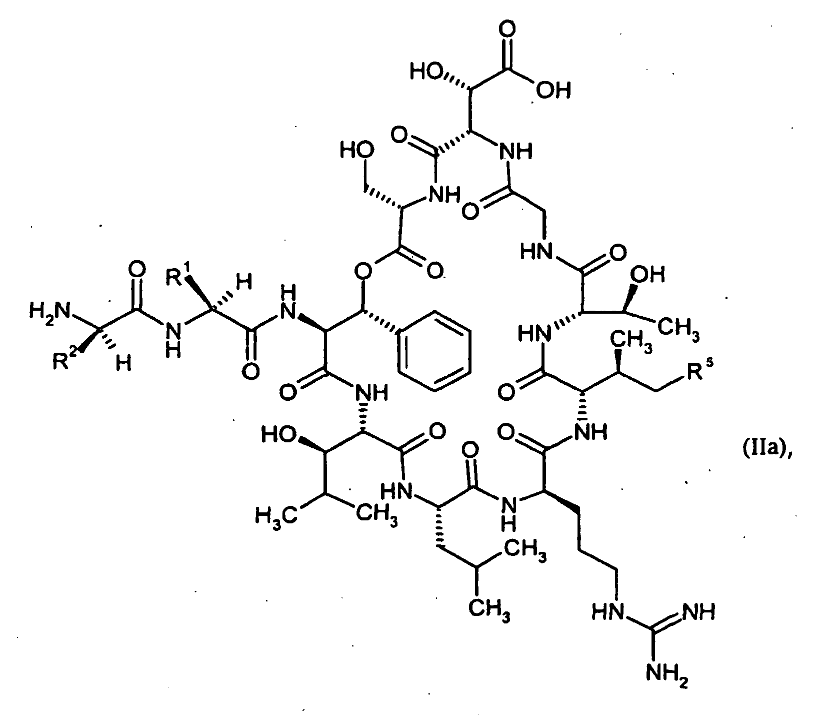 in welcher
R¹, R² und R⁵ die oben angegebene Bedeutung haben,
mit Verbindungen der Formel 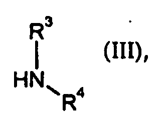 in welcher
R³ und R⁴ die oben angegebene Bedeutung haben,
umgesetzt werden.

Die freie Amino-Gruppe in dem Rest H₂N(CHR²)- wird vor der Umsetzung nach dem Fachmann bekannten Methoden zum Beispiel mit einer Boc-Schutzgruppe oder einer Benzyloxycarbonyl-Schutzgruppe geschützt, die nach der Umsetzung wieder abgespalten wird.

Die Umsetzung erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, *N*-Methylpiperidin, 4-Dimethylaminopyridin oder Diisopropylethylamin.

Als Dehydratisierungsreagenzien eignen sich hierbei beispielsweise Carbodiimide wie z.B. *N*,*N*'-Diethyl-, *N,N*'-Dipropyl-, *N,N'*-Diisopropyl-, *N,N'*-Dicyclohexylcarbodiimid, *N*-(3-Dimethylaminoisopropyl)-*N'*-ethylcarbodiimid-hydrochlorid (EDC), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid, oder *O*-(Benzotriazol-1-yl)-*N,N,N',N'*-tetramethyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N',N'-*tetramethyluroniumhexafluorophosphat (HATU); oder 1-Hydroxybenztriazol (HOBt), oder Benzotriazol-1-yloxytris(dimethylamino)phosphoniumhexafluorophosphat (BOP), oder Benzotriazol-1-yloxytris(pyrrolidino)phosphoniumhexafluorophosphat (PyBOP), oder N-Hydroxysuccinimid, oder Mischungen aus diesen, mit Basen.

Vorzugsweise wird die Kondensation mit HATU und N-Methylmorpholin durchgeführt.

Bevorzugt sind die Verfahren, in welchen R⁵ Methyl bedeutet.

Bevorzugt sind auch die Verfahren, in welchen
- R¹: 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
- R²: 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
- R³: C₁-C₄-Alkyl bedeutet,
wobei Alkyl substituiert sein kann mit 1 bis 2 Substituenten, wobei die Substituenten unabhängig voneinander ausgewählt werden aus der Gruppe, bestehend aus Hydroxy, Amino, Hydroxycarbonyl, Aminocarbonyl, {[Bis(dimethylamino)methylen]amino}ethoxy, Phenyl, Morpholin-4-yl, Pyrid-2-yl, Pyrid-3-yl, Pyrid-4-yl, Benzyloxycarbonyl und Benzyloxycarbonylamino,
- R⁴: Wasserstoff oder Methyl bedeutet,

Bevorzugt sind auch die Verfahren, in welchen
- R¹: 2-Methylprop-1-yl bedeutet,
- R²: 2-Methylprop-1-yl bedeutet,
- R³: C₁-C₃-Alkyl bedeutet,
wobei Alkyl substituiert sein kann mit einem Substituenten, wobei der Substituent ausgewählt wird aus der Gruppe, bestehend aus Hydroxy, Amino, Hydroxycarbonyl, Aminocarbonyl, {[Bis(dimethylamino)methylen]amino}ethoxy, Phenyl, Morpholin-4-yl, Pyrid-3-yl, Benzyloxycarbonyl und Benzyloxycarbonylamino,
- R⁴: Wasserstoff oder Methyl bedeutet,

Bevorzugt sind auch die Verfahren, in welchen
- R¹: 2,2-Dimethylprop-1-yl bedeutet,
- R²: 2,2-Dimethylprop-1-yl bedeutet,
- R³: C₁-C₃-Alkyl bedeutet,
wobei Alkyl substituiert sein kann mit einem Substituenten, wobei der Substituent ausgewählt wird aus der Gruppe, bestehend aus Hydroxy, Amino, Hydroxycarbonyl, Aminocarbonyl, {[Bis(dimethylamino)methylen]amino}ethoxy, Phenyl, Morpholin-4-yl, Pyrid-3-yl, Benzyloxycarbonyl und Benzyloxycarbonylamino,
- R⁴: Wasserstoff oder Methyl bedeutet,

Die Verbindungen der Formeln (Ia), (I), (IIa) und (II), die als Salze vorliegen, können zum Beispiel durch Umsetzung mit Salzsäure oder Methansulfonsäure in ein Salz mit einem anderen Gegenion überführt werden.

Die Verbindungen der Formeln (Ia), (I), (IIa) und (II), die als Salze vorliegen, können durch Umsetzung mit einer Base in die freie Base überführt werden.

Die Verbindungen der Formel (III) sind bekannt oder lassen sich nach bekannten Verfahren aus den entsprechenden Edukten synthetisieren.

Überraschend wurde gefunden, dass Verbindungen der Formel (IIa) hergestellt werden können durch selektive Hydrolyse von Verbindungen der Formel 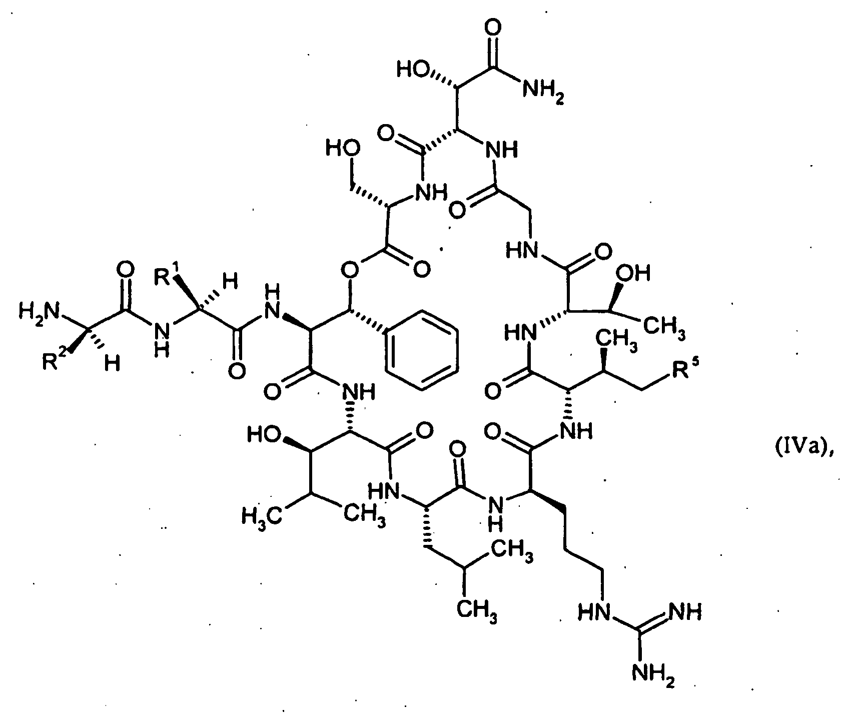 in welcher
R¹, R² und R⁵ die oben angegebene Bedeutung haben,
mit einer Säure in einem geeigneten Lösungsmittel.

Die Umsetzung erfolgt im Allgemeinen in einem Lösungsmittel, bevorzugt in einem Temperaturbereich von Raumtemperatur bis 50°C bei Normaldruck.

Lösungsmittel sind beispielsweise Gemische von Dioxan mit Wasser oder Tetrahydrofuran mit Wasser.

Säuren sind beispielsweise Mineralsäuren wie Salzsäure oder andere starke Säuren wie Methansulfonsäure, bevorzugt ist Salzsäure.

Gegenstand der Erfindung ist weiterhin ein Verfahren zur Herstellung der Verbindungen der Formel (IIa) durch Hydrolyse einer Verbindung der Formel (IVa) mit einer Säure in einem geeigneten Lösungsmittel.

Bevorzugt wird das Verfahren in einem Temperaturbereich von Raumtemperatur bis 50°C bei Normaldruck durchgeführt.

Besonders bevorzugt wird das Verfahren bei Raumtemperatur unter Normaldruck durchgeführt.

Bevorzugt ist das Lösungsmittel ausgewählt aus der Gruppe bestehend aus, Dioxan, Tetrahydrofuran, Wasser und Mischungen davon.

Besonders bevorzugt ist das Lösungsmittel ausgewählt aus der Gruppe bestehend aus einem Gemisch von Dioxan mit Wasser und einem Gemisch von Tetrahydrofuran mit Wasser.

Bevorzugt ist die Säure ausgewählt aus der Gruppe bestehend aus den Mineralsäuren und anderen starken Säure, insbesondere ist die Säure eine Mineralsäure.

Bevorzugt ist die Säure ausgewählt aus der Gruppe bestehend aus Salzsäure und Methansulfonsäure.

Besonders bevorzugt ist die Säure Salzsäure.

Bevorzugt sind die Verfahren, in denen R⁵ Methyl bedeutet.

Bevorzugt sind auch die Verfahren, in denen R¹ und R² 2-Methylprop-1-yl bedeuten.

Bevorzugt sind auch die Verfahren, in denen R¹ und R² 2,2-Dimethylprop-1-yl bedeuten.

Die Verbindungen der Formel (IVa) sind bekannt oder können hergestellt werden, indem Verbindungen der Formel 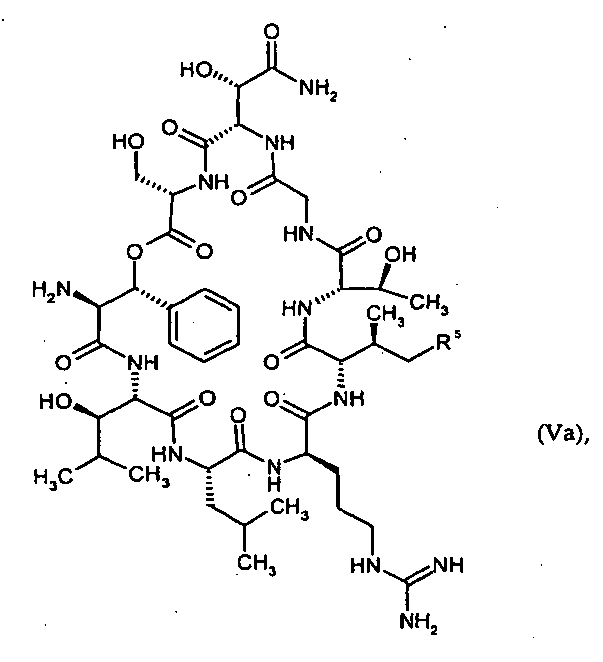 in welcher R⁵ die oben angegebene Bedeutung hat,
mit Verbindungen der Formel 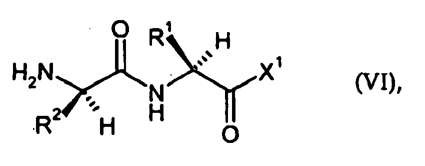 in welcher
R¹ und R² die oben angegebene Bedeutung haben, und
X¹ Halogen, bevorzugt Brom, Chlor oder Fluor, oder Hydroxy bedeutet,
umgesetzt werden.

Falls X¹ für Halogen steht, erfolgt die Umsetzung im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid. Als inerte Lösungsmittel sind Tetrahydrofuran oder Methylenchlorid bevorzugt.

Basen sind beispielsweise Triethylamin, Diisopropylethylamin oder N-Methylmorpholin, bevorzugt ist Diisopropylethylamin.

Falls X¹ für Hydroxy steht, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'*-Diethyl-, *N,N,'*-Dipropyl-, *N,N'*-Diisopropyl-, *N,N'*-Dicyclohexylcarbodiimid, *N*-(3-Dimethylaminoisopropyl)-*N'*-ethylcarbodiimid-hydrochlorid (EDC), *N-*Cyclohexylcarbodiimid-*N'*-propyloxymethyl-Polystyrol (PS-Carbodiimid) oder. Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N;N'-*tetramethyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N',N'-*tetramethyluroniumhexafluorophosphat (HATU), oder 1-Hydroxybenztriazol (HOBt), oder Benzotriazol-1-yloxytris(dimethylamino)phosphoniumhexafluorophosphat (BOP), oder N-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formel (VI) tragen gegebenenfalls Schutzgruppen, so dass sich in diesen Fällen der Umsetzung der Verbindung der Formel (Va) mit Verbindungen der Formel (VI) eine Abspaltung der Schutzgruppen mit zum Beispiel Trifluoressigsäure nach dem Fachmann bekannten Methoden anschließt.

Die Verbindungen der Formel (Va) lassen sich durch doppelten Edmann-Abbau aus Lysobactin (Beispiel 1A) oder Katanosin A synthetisieren.

Die Verbindungen der Formel (VI) sind bekannt oder lassen sich nach bekannten Verfahren aus den entsprechenden Edukten synthetisieren.

Die Herstellung der erfindungsgemäßen Verbindungen kann durch folgendes Syntheseschema verdeutlicht werden.

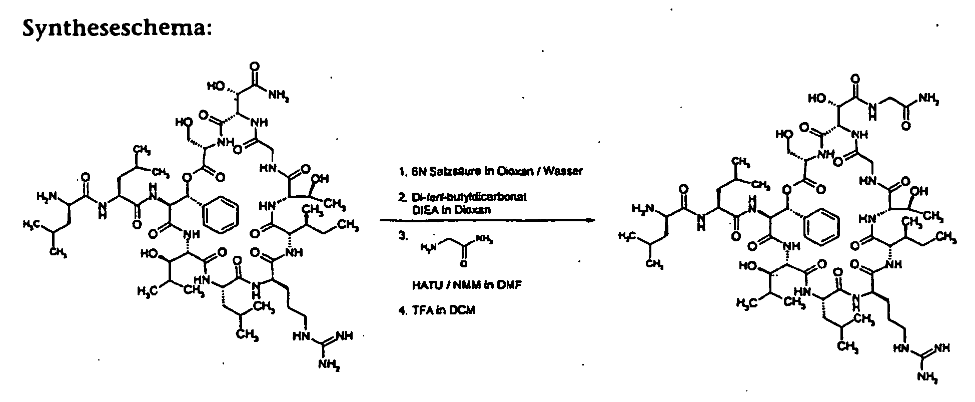

Die erfindungsgemäßen Verbindungen der Formeln (Ia) und (I) zeigen ein nicht vorhersehbares, wertvolles pharmakologisches Wirkspektrum. Sie zeigen eine antibakterielle Wirkung.

Sie eignen sich daher zur Verwendung als Arzneimittel zur Behandlung und/oder Prophylaxe von Krankheiten bei Menschen und Tieren.

Die erfindungsgemäßen Verbindungen der Formeln (Ia) und (I) zeichnen sich durch eine höhere freie Fraktion (fᵤ) in Ratten- und Humanplasma gegenüber Lysobactin aus.

Die erfindungsgemäßen Verbindungen der Formeln (Ia) und (I) zeichnen sich durch eine geringere Nephrotoxizität gegenüber Lysobactin aus.

Die erfindungsgemäßen Verbindungen der Formeln (Ia) und (I) zeichnen sich durch eine bessere Löslichkeit gegenüber Lysobactin aus.

Die beschriebenen Nonadepsipeptide wirken als Inhibitoren der bakteriellen Zellwandbiosynthese.

Besonders wirksam sind die erfindungsgemäßen Zubereitungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Grundsätzlich können die erfindungsgemäßen Zubereitungen gegen alle Bakterien und bakterienähnlichen Mikroorganismen verwendet werden, die im Besitz einer bakteriellen Zellwand (Murein sacculus) bzw. den dazugehörigen Enzymsystemen sind, beispielsweise gegen die folgenden Erreger oder gegen Mischungen der folgenden Erreger:

Gram-negative Kokken (*Neisseria gonorrhoeae*) sowie Gram-negative Stäbchen wie Enterobakteriaceen, z.B. *Escherichia coli, Hämophilus influenzae*, Pseudomonas, Klebsiella, Citrobacter (*C. freundii, C. divernis*), Salmonella und Shigella; ferner Enterobacter (*E. aerogenes, E. agglomerans*), Hafnia, Serratia (*S. marcescens*), Providencia, Yersinia, sowie die Gattung Acinetobacter, Branhamella und Chlamydia. Darüber hinaus umfasst das antibakterielle Spektrum strikt anaerobe Bakterien wie z.B. *Bacteroides fragilis*, Vertreter der Gattung Peptococcus, Peptostreptococcus sowie die Gattung Clostridium; ferner Mykobakterien, z.B. *M*. *tuberculosus*. Besonders ausgeprägte Wirkung zeigen die erfindungsgemäßen Verbindungen gegen Gram-positive Kokken, z.B. Staphylokokken (*S. aureus, S. epidermidis, S. haemolyticus, S. camosus*), Enterokokken (*E. faecalis, E. faecium*) und Streptokokken (*S. agalactiae, S. pneumoniae, S. pyogenes*).

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Zubereitungen verhindert, gebessert oder geheilt werden können, seien beispielsweise genannt:

Infektionskrankheiten beim Menschen wie z.B. unkomplizierte und komplizierte Harnwegsinfekte, unkomplizierte Haut- und Oberflächeninfektionen, komplizierte Haut- und Weichteilinfektionen, im Hospital und ambulant erworbene Lungenentzündung, nosokomiale Pneumonien, akute Exazerbationen und sekundäre bakterielle Infektionen der chronischen Bronchitis, akute Otitis media, akute Sinusitis, streptokokkale Pharyngitis, bakterielle Meningitis, unkomplizierte gonokokkale und nicht-gonokokkale Urethritis/Cervizitis, akute Prostatitis, Endocarditis, unkomplizierte und komplizierte intra-abdominale Infektionen, gynäkologische Infektionen, pelvische Entzündungskrankheit, bakterielle Vaginose, akute und chronische Osteomyelitis, akute bakterielle Arthritis, empirische Therapie in febrilen neutropenischen Patienten, des Weiteren Bakterämien, MRSA Infektionen, akute infektiöse Diarrhoe, *Helicobacter pylori* Infektionen, postoperative Infektionen, odontogene Infektionen, ophthalmologische Infektionen, postoperative Infektionen (inkl. periproktaler Abszess, Wundinfektionen, Galleninfektionen, Mastitis und akute Appendizitis), zystische Fibrose und Bronchiectasis.

Außer beim Menschen können bakterielle Infektionen auch bei anderen Spezies behandelt werden. Beispielhaft seien genannt:
Schwein: Diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): *E. coli*-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsia, Yersinia, erweitert.

Die erfindungsgemäßen Verbindungen der Formeln (IIa) und (II) bilden wichtige Zwischenstufen in der Synthese der Verbindungen der Formeln (Ia) und (I).

Weiterer Gegenstand der vorliegenden Erfindung ist der Einsatz der erfindungsgemäßen Verbindungen der Formeln (Ia) und (I) zur Behandlung und/oder Prophylaxe von Erkrankungen, insbesondere von bakteriellen Infektionskrankheiten.

Weiterer Gegenstand der vorliegenden Erfindung ist die Verwendung der erfindungsgemäßen Verbindungen der Formeln (Ia) und (I) zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen der Formeln (Ia) und (I) zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen der Formeln (Ia) und (I) zur Herstellung von Arzneimitteln verwendet, die zur Prophylaxe und/oder Behandlung von bakteriellen Erkrankungen geeignet sind.

Weiterhin wird ein Verfahren zur Behandlung und/oder Prophylaxe von Erkrankungen, insbesondere der zuvor genannten Erkrankungen, unter Verwendung einer antibakteriell wirksamen Menge der erfindungsgemäßen Verbindungen der Formeln (Ia) und (I), beschrieben.

Weiterer Gegenstand der vorliegenden Erfindung sind Arzneimittel, enthaltend mindestens eine erfindungsgemäße Verbindung der Formeln (Ia) und (I) und mindestens einen oder mehrere weitere Wirkstoffe, insbesondere zur Behandlung und/oder Prophylaxe der zuvor genannten Erkrankungen. Bevorzugte Kombinationswirkstoffe sind antibakteriell wirkende Verbindungen, die ein anderes Wirkspektrum, insbesondere ergänzendes Wirkspektrum, haben und/oder synergistisch zu den erfindungsgemäßen Verbindungen sind.

Die erfindungsgemäßen Verbindungen der Formeln (Ia) und (I) können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen der Formeln (Ia) und (I) können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglykole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung der Formeln (Ia) und (I), üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 100 mg/kg, vorzugsweise etwa 0.1 bis 10 mg/kg Körpergewicht zur Erzielung wirksamer Ergebnisse zu verabreichen, und bei oraler Applikation beträgt die Dosierung etwa 0.01 bis 50 mg/kg, vorzugsweise 0.5 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen

- Allg.: Allgemein(e)
- Area: (Peak)fläche
- ber.: berechnet
- BHI: Brain Heart Infusion
- Boc: *tert*-Butyloxycarbonyl
- br.: breites Signal (bei NMR-Spektren)
- Bsp.: Beispiel
- d: Dublett (bei NMR-Spektren)
- DC: Dünnschichtchromatographie
- DCI: direkte chemische Ionisation (bei MS)
- DCM: Dichlormethan
- DIEA: *N,N*-Diisopropylethylamin
- DMSO: Dimethylsulfoxid
- DMF: *N,N*-Dimethylformamid
- d. Th.: der Theorie
- EDC: 1-Ethyl-3-(3-dimethylaminopropyl)carbodiimide (auch EDCI)
- EDCxHCl: 1-Ethyl-3-(3-dimethylaminopropyl)carbodiimide-hydrochlorid
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- gef.: gefunden
- ges.: gesättigt
- h: Stunde
- HATU: *O*-(7-Azabenzotriazol-1-yl)-*N,N,N',N'*-tetramethyluroniumhexafluorphosphat
- HOBt: 1-Hydroxybenzotriazol
- HPLC: Hochdruck-, Hochleistungsflüssigchromatographie
- HR: High Resolution (Hochauflösung)
- i. V.: im Vakuum
- konz.: konzentriert
- LC-MS: Flüssigchromatographie-gekoppelte Massenspektroskopie
- LDA: Lithium-Diisopropylamid
- m: middle (mittel) (bei UV- und IR-Spektren)
- m: Multiplett (bei NMR-Spektren)
- MALDI: Matrix-Assisted Laser Desorption/Ionization
- MHK: Minimale Hemmkonzentration
- min: Minute/Minuten
- MRSA: Methicillin-resistenter *Staphylococcus aureus*
- MS: Massenspektroskopie
- NCCLS: National Committee for Clinical Laboratory Standards
- neg.: negativ
- NMM: N-Methylmorpholin
- NMR: Kernresonanzspektroskopie (nuclear magnetic resonance)
- p.a.: pro analysi
- Pd-C: Palladium auf Kohle
- pos.: positiv
- proz.: prozentig
- quant.: quantitativ
- RP-HPLC: Reverse Phase HPLC
- RT: Raumtemperatur
- Rₜ: Retentionszeit (bei HPLC)
- s: strong (stark) (bei UV- und IR-Spektren)
- s: Singulett (bei NMR-Spektren)
- TBTU: *O*-(Benzotriazol-1-yl)-*N,N,N,'N'*-tetramethyluroniumtetrafluoroborat
- TCTU: *O*-(1H-6-Chlorbenzotriazol-1-yl)-1,1,3,3-tetramethyluroniumtetrafluoroborat
- TFA: Trifluoressigsäure
- TFE: 2,2,2-Trifluorethanol
- THF: Tetrahydrofuran
- TOF: Flugzeit (time of flight)
- UV: Ultraviolett
- Vis: sichtbar (visible)
- VRSA: Vancomycin-resistenter *Staphylococcus aureus*
- w: weak (schwach) (bei UV- und IR-Spektren)
- Z, Cbz: Benzyloxycarbonyl

### Literatur

Zur Nomenklatur der Peptide und Cyclodepsipeptide vergleiche:
1. A Guide to IUPAC Nomenclature of Organic Compounds (Recommendations 1993), 1993, Blackwell Scientific publications.
2. Nomenclature and symbolism for amino acids and peptides. Recommendations 1983. IUPAC-IUB Joint Commission on Biochemical Nomenclature, UK. Biochemical Journal 1984, 219, 345-373. Sowie zitierte Literatur.
3. Zur Nomenklatur von Nonadepsipeptid-Derivaten, die in den Aminosäure-Seitenketten derivatisiert sind, wird das IUPAC-Prefix-System zur Adressierung der jeweiligen Derivatisierungsstelle verwendet (IUPAC, Nomenclature and Symbolism for Amino Acids and Peptides, Names and Symbols for Derivatives of Named Peptides, Section 3AA-22, Recommendations 1983-1992). So bezeichnet beispielsweise *N*^{ω.6}-Acetyl-lysobactin ein an der Aminosäure 6 (vom N-Terminus des Depsipeptids gerechnet, d.h. hier D-Arg) speziell am terminalen Stickstoffatom acetyliertes Lysobactin. Analog bezeichnet *O^{3.11}*-Methyllysobactin ein an der Aminosäure 11 (Ser) am Seitenkettensauerstoffatom (*O³*) methyliertes Derivat.

### Allgemeine Methoden LC-MS, HR-MS, HPLC und Gelchromatographie

**Methode 1 (HPLC):** Gerätetyp HPLC: HP 1100 Series; UV DAD Säule: Zorbax Eclipse XBD-C8 (Agilent), 150 mm x 4.6 mm, 5 µm; Eluent A: 5 ml HClO₄/l Wasser, Eluent B: Acetonitril; Gradient: 0-1 min 10%B, 1-4 min 10-90%B, 4-5 min 90%B; Fluss: 2.0 ml/min; Ofen: 30°C; UV-Detektion: 210 und 254 nm.
**Methode 2 (HPLC):** Säule: Kromasil RP-18, 60 mm x 2 mm, 3.5. µm; Eluent A: 5 ml HClO₄/l Wasser, Eluent B: Acetonitril; Gradient: 0 min 2%B, 0.5 min 2%B, 4.5 min 90%B, 9 min 90%B; Fluss: 0.75 ml/min; Ofen: 30°C; UV-Detektion: 210 nm.
**Methode 3 (LC-MS)**: Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.
**Methode 4 (HPLC):** Säule: Kromasil RP-18, 250 mm x 4 mm, 5 µm; Eluent A: 5 ml HClO₄/l Wasser, Eluent B: Acetonitril; Gradient: 0 min 5%B, 10 min 95%B; Fluss: 1 ml/min; Ofen: 40°C; UV-Detektion: 210 nm.
**Methode 5 (HPLC):** Säule: Kromasil RP-18, 250 mm x 4 mm, 5 µm; Eluent A: 2 ml HClO₄/l Wasser; Eluent B: Acetonitril; Isokratisch: 45%B, 55%A; Fluss: 1 ml/min; Ofen: 40°C; UV-Detektion: 210 nm.
**Methode 6 (präparative HPLC):** Gerät: Gilson Abimed HPLC; binäres Pumpensystem; Säule: Nucleodur C₁₈ Gravity, Fa. Macherey-Nagel, 5 µm; 250 mm × 21 mm; Eluent A: Wasser/0.05-0.1% Trifluoressigsäure, Eluent B: Acetonitril/ 0.05-0.1% Trifluoressigsäure; Gradient: 0-8 min 5%B, 8-40 min 5-60%B, 40-60 min 60%B, 60-75 min 60-100%B, 75-80 min 100%B, anschließend Regeneration der Chromatographiesäule; Fluss: 7-15 ml/min; UV-Detektion: 210 nm.
**Methode 7 (präparative HPLC):** Gerät: Gilson Abimed HPLC; binäres Pumpensystem; Säule: Kromasil-100A C₁₈, 5 µm; 250 mm x 30 mm; Eluent A: Wasser/0.05-0.5% TFA, Eluent B: Acetonitril; Gradient: 0-5 min 5%B, 5.01-10 min 10%B, 10.01-20 min 40%B, 20.01-27 min 50%B, 27.01-40 min 60%B, 40.01-45 min 90%B, 45.01-60 min 100%B; Fluss: 15-60 ml/min; UV Detektor 210 nm.
**Methode 8 (präparative HPLC):** Gerät: Gilson Abimed HPLC; binäres Pumpensystem; Säule: Kromasil-100A C₁₈, 5 µm; 250 mm x 30 mm; Eluent A: Wasser/0.05-0.5% Trifluoressigsäure, Eluent B: Acetonitril; 0-10 min 10%B, Rampe, 10.01-55 min 100% B; Fluss: 30 ml/min; UV Detektor 210 nm.
**Methode 9 (Gelchromatographie Sephadex LH-20):** Gelchromatographie wird ohne Druck an Sephadex LH-20 (Firma Pharmacia) durchgeführt. Es wird nach UV-Aktivität (UV Detektor für 210 nm, Firma Knauer) fraktioniert (Fraktionssammler ISCO Foxy 200). Säulendimensionen: 60 x 21 cm (2500-5000 µmol Maßstab); 50 x 10 cm (500-2500 µmol Maßstab); 30 x 5 cm (250-500 µmol Maßstab); 25 x 4 cm (50-250 µmol Maßstab); 40 x 2 cm (5-50 µmol Maßstab).
**Methode 10 (LC-MS)**: Gerätetyp MS: Micromass ZQ; Gerätetyp HPLC: Waters Alliance 2795/HP 1100; Säule: Phenomenex Synergi 2 µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.
**Methode 11 (TOF-HR-MS):** *TOF-HR-MS-ESI*+ Spektren werden mit einem Micromass LCT Gerät (Kapillarspannung: 3.2 KV, Cone-Spannung: 42 V, Quellentemperatur: 120°C, Desolvations-Temperatur: 280°C) aufgenommen. Hierzu wird eine Spritzenpumpe (Fa. Harvard Apparatus) für die Probenzuführung verwendet. Als Standard dient Leucin-Enkephalin (Tyr-Gly-Gly-Phe-Leu).
**Methode 12 (HPLC):** Säule: Gilson Abimed HPLC; Varian binäres Pumpensystem; Phenomenex Luna C18 5µ 250 mm x 20 mm; Fluss: 25 ml/min; Ofen: RT; UV-Detektion: 210 nm; Eluent A: Wasser/0.2% TFA, Eluent B: Acetonitril; Isokratisch 50%B.
**Methode 13 (HPLC):** Säule: Gilson Abimed HPLC; Varian binäres Pumpensystem; Kromasil 100 C18 5µ 250 mm x 20 mm; Fluss: 25 ml/min; Ofen: RT; UV-Detektion: 210 nm; Eluent A: Wasser/0.2% TFA, Eluent B: Acetonitril; Isokratisch 65%B.
**Methode 14 (analytische HPLC):** Gerätetyp HPLC: HP 1100 Series; UV DAD; Säule: Phenomenex Synergi 2 µ Hydro-RP Mercury 20 mm x 4 mm; Eluent A: 1 l Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.
**Methode 15 (analytische HPLC):** Gerätetyp HPLC: HP 1050 Series; UV DAD; Säule: Zorbax 300 mSB-C18 3.5 µ, 4.6 mm × 150 mm; Eluent A: 11 Wasser + 0.1% Trifluoressigsäure, Eluent B: 400 ml Acetonitril/600 ml Wasser + 0.1% Trifluoressigsäure; Gradient: 0.0 min 100%A, 1.3 min 10%B, 18.0 min 80%B, 20.0 min 80%B, 21.0 min 100%B, 25.0 min 100%B, 26.0 min 0%B, 30.0 min 0%B. Fluss: 1 ml/min; Ofen: 40°C; UV-Detektion: 210 nm.
**Methode 16 (analytische HPLC):** Gerätetyp HPLC: HP 1050 Series; UV DAD; Säule: Zorbax 300 mSB-C18 3.5 µ, 4.6 mm × 150 mm; Eluent A: 1 l Wasser + 0.1% Trifluoressigsäure, Eluent B: 400 ml Acetonitril/600 ml Wasser + 0.1% Trifluoressigsäure; Gradient: 0.0 min 100%A, 2.0 min 10%B, 50.0 min 80%B, 52.0 min 100%B, 55.0 min 100%A, 60.0 min 100%A. Fluss: 1 ml/min; Ofen: 40°C; UV-Detektion: 210 nm.

### Allgemeine Arbeitsvorschriften

### Allgemeine Arbeitsvorschrift 1 (Abspaltung von Boc-Schutzgruppen mit TFA)

Die Boc-geschützte Verbindung (2-15 µmol) wird in Dichlormethan suspendiert (1 ml) und anschließend unter Argon-Schutzgasatmosphäre mit Trifluoressigsäure (3 ml) in Dichlormethan (10 ml) versetzt und so lange bei RT gerührt bis das HPLC-Chromatogramm vollständigen Umsatz zeigt (z. B. Methode 14). Dann wird das Lösungsmittel im Vakuum abdestilliert, wobei die Badtemperatur 30°C nicht übersteigen soll. Das Rohprodukt wird in Toluol suspendiert, erneut am Rotationsverdampfer eingeengt und im Hochvakuum getrocknet. Diese Prozedur wird mehrfach wiederholt (2-5 ×).

### Allgemeine Arbeitsvorschrift 2 (Hydrogenolytische Esterspaltung/Carbamidspaltung)

Der peptidische Benzylester (1-15 µmol) wird in Dioxan (2 ml) und 0.1%ige wässriger Trifluoressigsäure (3 ml) gelöst und unter Argonschutzgasatmosphäre mit 10%iger Palladium-Kohle (10 mol%) versetzt. Bei RT und Normaldruck wird so lange hydriert bis die analytische HPLC (z. B. Methode 14) vollständigen Umsatz (ca. 30 min) zeigt. Das Reaktionsgemisch wird filtriert (z.B. über Spritzenfilter, Kieselgur, Celite®) und über die präparative RP-HPLC feingereinigt.

### Allgemeine Arbeitsvorschrift 3 (Amidkupplung)

Zu einer Lösung des Carbonsäurecyclopeptids (1.0 Äquivalente) und des Amins (5 - 15 Äquivalente) in trockenem DMF (5-30 µmol/ml) wird bei 0°C unter Argon-Schutzgasatmosphäre zunächst HATU (5-15 Äquivalente) und anschließend NMM (5-20 Äquivalente) zugegeben. Das Reaktionsgemisch wird langsam auf RT erwärmt und wird bei dieser Temperatur nachgerührt, bis sich ein vollständiger Umsatz zeigt. Das Reaktionsgemisch wird im Hochvakuum eingedampft und chromatographisch aufgereinigt.

### Ausgangsverbindungen

### Beispiel 1A

### D-Leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bis-trifluoracetat (Lysobactin)

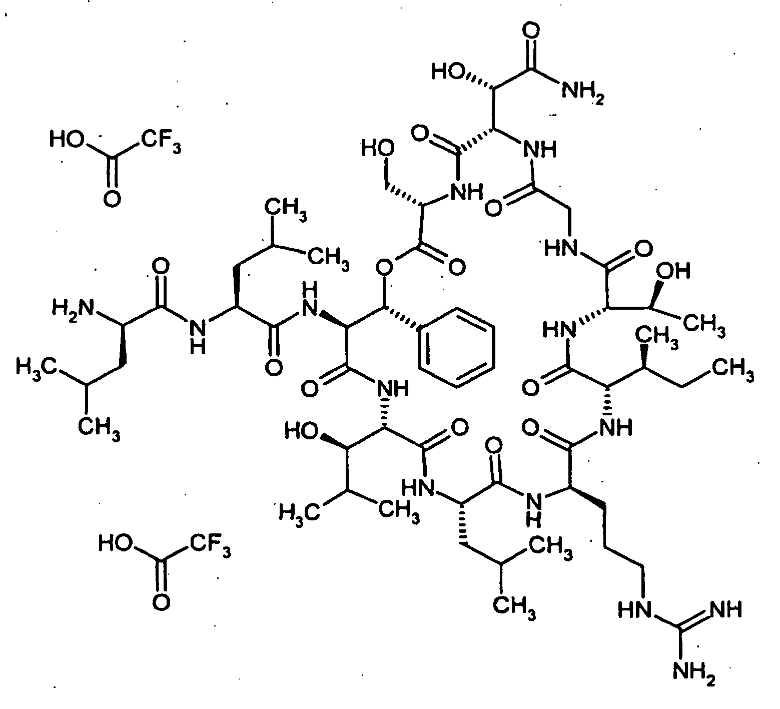

### Fermentation:

### Kultur Medium:

YM: Hefe-Malz Agar: D-Glucose (4 g/l), Hefe Extrakt (4 g/l), Malz Extrakt (10 g/l), 1 Liter Lewatit Wasser. Vor dem Sterilisieren (20 Minuten bei 121°C) wird der pH auf 7.2 eingestellt.
HPM: Mannit (5.4 g/l), Hefe Extrakt (5 g/l), Fleischpepton (3 g/l).
Arbeitskonserve: Der lyophilisierte Stamm (ATCC 53042) wird in 50 ml YM Medium angezogen.
Kolbenfermentation: 150 ml YM Medium oder 100 ml HPM Medium in einem 1 l Erlenmeyerkolben werden mit 2 ml der Arbeitskonserve beimpft und für 30-48 Stunden bei 28°C auf einem Schüttler bei 240 Upm wachsen gelassen.
30 l Fermentation: 300 ml der Kolbenfermentation (HPM Medium) werden zum Animpfen einer sterilen 30 l Nährmedienlösung verwandt (1 ml Antifoam SAG 5693/1). Diese Kultur wird für 21 Stunden bei 28°C, 300 Upm und einer Belüftung mit steriler Luft von 0.3 vvm wachsen gelassen. Der pH wird mit 1 M Salzsäure auf pH = 7.2 konstant gehalten. Insgesamt werden während der Kultivierungszeit 880 ml 1 M Salzsäure zugeführt.
Hauptkultur (200 l): 15 x 150 ml YM Medium in 1 l Erlenmeyerkolben werden mit 2 ml der Arbeitskonserve beimpft und bei 28°C für 48. Stunden und 240 Upm auf dem Schüttler wachsen gelassen. 2250 ml dieser Kultur werden zum Beimpfen einer sterilen 200 l Nährmedienlösung (YM) verwandt (1 ml Antifoam SAG 5693/l) und für 18.5 Stunden bei 28°C, 150 Upm und einer Belüftung mit steriler Luft von 0.3 vvm wachsen gelassen.

Zur Kontrolle des Fermentationsverlaufs werden stündlich Proben (50. ml) entnommen. 2 ml dieser Kulturbrühe werden mit 1 ml Methanol (0.5% Trifluoressigsäure) versetzt und über einen 0.45 µm Filter filtriert. 30 µl dieser Suspension werden mittels HPLC analysiert (Methode 1 und Methode 2).

Nach 18.5 Stunden wird die Kulturbrühe der Hauptkultur bei 17000 Upm in Überstand und Sediment getrennt.

### Isolierung:

Der Überstand (183 l) wird mit konzentrierter Trifluoressigsäure bzw. Natronlauge auf pH 6.5-7 eingestellt und auf eine Lewapolsäule (OC 1064, 60 l Inhalt) aufgetragen. Anschließend wird mit reinem Wasser, Wasser/Methanol 1:1 und anschließend mit reinem Methanol (mit 0.1% Trifluoressigsäure) eluiert. Diese organische Phase wird im Vakuum bis zu einem verbleibenden wässrigen Rest von 11.5 l eingeengt.
Die verbleibende wässrige Phase wird an Kieselgel C₁₈ gebunden und aufgetrennt (MPLC, Biotage Flash 75, 75 x 30 cm, KP-C18-WP, 15-20 µm, Fluss: 30 ml; Eluent: Acetonitril/ Wasser mit 0.1% Trifluoressigsäure; Gradient: 10%, 15% and 40% Acetonitril). Die 40% Acetonitril Phase, die die Hauptmenge von Beispiel 1A enthält, wird im Vakuum eingeengt und anschließend lyophilisiert (ca. 13 g). Dieses Feststoffgemisch wird in 1.2 g Portionen zunächst an einer präparativen HPLC (Methode 3), anschließend durch Gelfiltration an Sephadex LH-20 (5 x 70 cm, Acetonitril/Wasser 1:1, jeweils mit 0.05% Trifluoressigsäure) und einer weiteren präparativen HPLC (Methode 4) getrennt.

Dieser Prozess liefert 2250 mg Beispiel 1A.

Das Sediment wird in 4 l Aceton/Wasser 4:1 aufgenommen, mit 2 kg Celite versetzt, mit Trifluoressigsäure auf pH = 6 eingestellt, ausgerührt und zentrifugiert. Das Lösungsmittel wird im Vakuum eingeengt und der Rückstand gefriergetrocknet. Das erhaltene Lyophilisat (89.9 g) wird in Methanol aufgenommen, abfiltriert, eingeengt und an Kieselgel (Methode 5) getrennt. Beispiel 1A wird danach per Gelfiltration (Sephadex LH-20, 5 x 68 cm, Wasser/Acetonitril 9:1 (mit 0.05% Trifluoressigsäure), Fluss: 2.7 ml/min, Fraktionsgröße 13.5 ml) zur Reinsubstanz aufgereinigt.

Dieser Prozess liefert 447 mg Beispiel 1A.

HPLC (Methode 1): Rₜ = 6.19 min

MS (ESIpos): m/z = 1277 (M+H)⁺

¹H NMR (500.13 MHz, *d*₆-DMSO): δ = 0.75 (d, 3H), 0.78 (d, 6H), 0.80 (t, 3H), 0.82 (d, 3H), 0.90 (d, 3H), 0.91 (d, 3H), 0.92 (d, 3H), 0.95 (d, 3H), 0.96 (d, 3H), 1.05 (m, 1H), 1.19 (d, 3H), 1.25 (m, 2H), 1.50 (m, 4H), 1.51 (m, 2H), 1.55 (m, 1H), 1.61 (m, 1H), 1.65 (m, 1H), 1.84 (m, 1H), 1.85 (m, 1H), 1.86 (m, 1H) , 1.89 (m, 1H), 1.95 (m, 1H), 2.75 (m, 2H), 3.40 (m, 1H), 3.52 (m, 2H), 3.53 (dd, 1H), 3.64 (m, 2H), 3.66 (m, 1H), 3.68 (dd, 1H), 3.73 (m, 2H), 4.00 (dd, 1H), 4.02 (br., 1H), 4.13 (br., 1H), 4.32 (dd, 1H), 4.39 (t, 1H), 4.55 (m, 1H), 4.75 (dd, 1H), 5.19 (t, 1H), 5.29 (d, 1H), 5.30 (br., 1H), 5.58 (m, 2H), 6.68 (m, 3H), 6.89 (d, 1H), 6.93 (m, 3H), 6.94 (br., 1H), 6.98 (d, 1H), 7.12 (br., 1H), 7.20 (br., 2H), 7.23 (m, 2H), 7.42 (m, 2H), 7.54 (d, 1H), 7.58 (d, 1H), 8.32 (br., 1H), 9.18 (br., 1H), 9.20 (m, 2H), 9.50 (br., 1H).

¹³C-NMR (125.77 MHz, *d*₆-DMSO): δ = 10.3, 15.3, 19.0, 19.2, 19.6, 20.0, 20.9, 22.0, 22.4, 23.0, 23.2, 24.3, 24.4, 25.0, 25.4, 26.0, 27.8, 30.9, 35.4, 39.5, 40.8, 40.9, 41.6, 44.1, 51.5, 52.7, 55.9, 56.2, 56.4, 57.9, 58.8, 60.2, 61.1, 62.6, 70.1, 71.6, 71.7, 75.5, 128.1, 128.6, 136.7, 156.8, 168.2, 170.1, 170.4, 171.2, 171.5, 171.9, 172.2, 172.4, 173.7.

Die Zuordnung der Signale erfolgte nach der in der Literatur beschriebenen Zuordnung (T. Kato, H. Hinoo, Y. Terui, J. Antibiot., 1988, 61, 719-725).

### Beispiel 2A

### D-Leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-aspartyl]-L-serin-C^{1.11}-O^{3.3}-lacton-bis-trifluoracetat, [10-{(3S)-3-Hyoroxy-L-aspartat}] lysobactin-bis-trifluoracetat

### (Lysobactinsäure-bis-trifluoracetat)

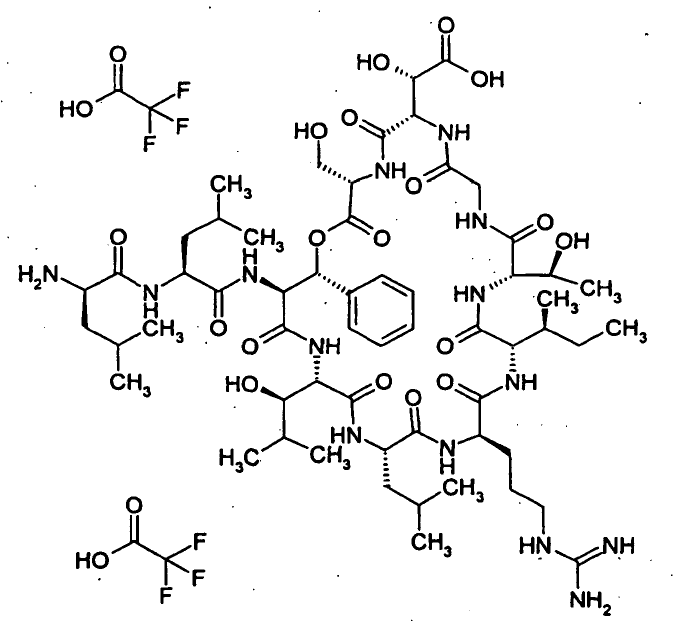

Eine Suspension von Lysobactin-bis-trifluoracetat (Beispiel 1A, 30.0 mg, 19.94 µmol) in Dioxan/10% Wasser (1.5 ml) wird mit 6 N Salzsäure (6 ml) versetzt und in 2 Tagen unter Rühren bei RT zum vollständigen Umsatz gebracht. Die Reaktion wird ständiger HPLC-Kontrolle unterworfen. Zur Aufarbeitung wird das Lösungsmittel am Rotationsverdampfer bei 30°C Badtemperatur entfernt und der Rückstand mittels präparativer RP-HPLC (Methode 6) bei RT gereinigt. Man erhält 21 mg (76% d. Th.) vom Produkt.

HPLC (Methode 16) Rₜ = 37.46 min.

LC-MS (Methode 10): Rₜ = 1.60 min; MS (ESlpos): *m*/*z* (%) = 1277 (5) [M+H]⁺, 639 (100) [M + 2H]²⁺; MS (ESIneg): *m*/*z* (%) = 1275 (60) [M - H]⁻, 637 (100) [M - 2H]²⁻.

HR-TOF-MS (Methode 11): C₅₈H₉₇N₁₄O₁₈ [M + H]⁺ gef. 1277.7104, ber. 1277.7100.

### Beispiel 3A

### N^{2.1}-(Benzyloxycarbonyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-aspartyl]-L-serin-C^{1.11}-O^{3.3}-lacton-trifluoracetat

### (N^{2.1}-(Benzyloxycarbonyl)-lysobactinsäure-trifluoracetat)

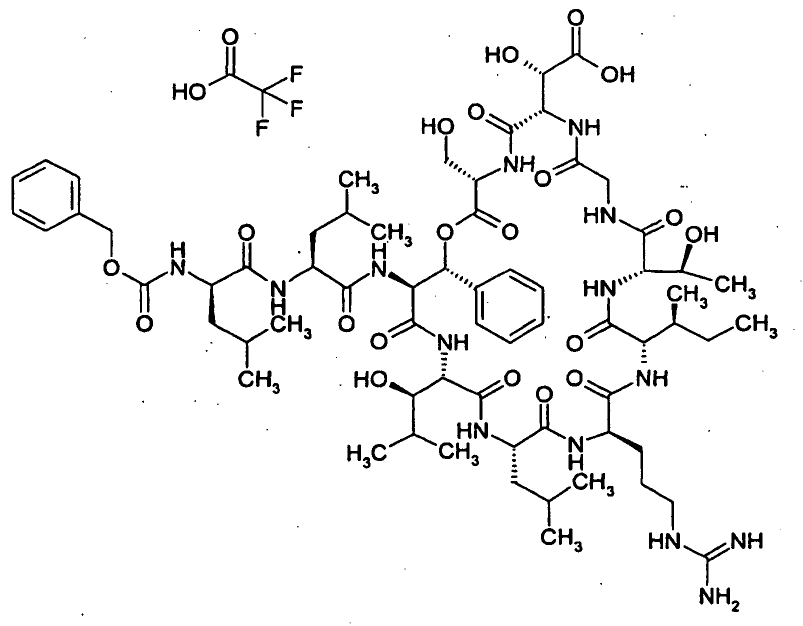

Lysobactinsäure-bis-trifluoracetat (Beispiel 2A, 200.0 mg, 0.13 µmol) wird in einer Mischung aus THF (30 ml) und DMF (5 ml) gelöst, dann werden *N*-(Benzoyloxy-carbonyloxy)succinimid (99.3 mg, 0.40 mmol) und NMM (39 µl, 36.3 mg, 0.36 mmol) bei 0°C zugegeben. Man erwärmt die Reaktion auf RT. Man rührt über Nacht und beobachtet dabei vollständigen Umsatz. Das Lösungsmittel wird am Rotationsverdampfer bei 30°C Badtemperatur entfernt und mittels präparativer RP-HPLC (Methode 8) gereinigt. Nach Gefriertrocknung erhält man 99.0 mg (49% d. Th.) der Titelverbindung.

HPLC (Methode 15) Rₜ = 18.75 min

LC-MS (Methode 10): Rₜ = 2.27 min; MS (ESIpos): *m*/*z* (%) = 1411 (37) [M + H]⁺, 706 (100) [M + 2H]²⁺; MS (ESIneg): *m*/*z* (%) = 1410 (100) [M - H]⁻, 704 (40) [M-2H]²⁻.

HR-TOF-MS (Methode 11): C₆₆H₁₀₃N₁₄O₂₀ [M + H]⁺ gef. 1411.7493, ber. 1411.7468.

### Beispiel 4A

### N^{2.1}-(tert-Butoxycarbonyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-aspartyl]-L-serin-C^{1.11}-O^{3.3}-lacton-trifluoracetat

### (N^{2.1}-(tert-Butoxycarbonyl)-lysobactinsäure-trifluoracetat)

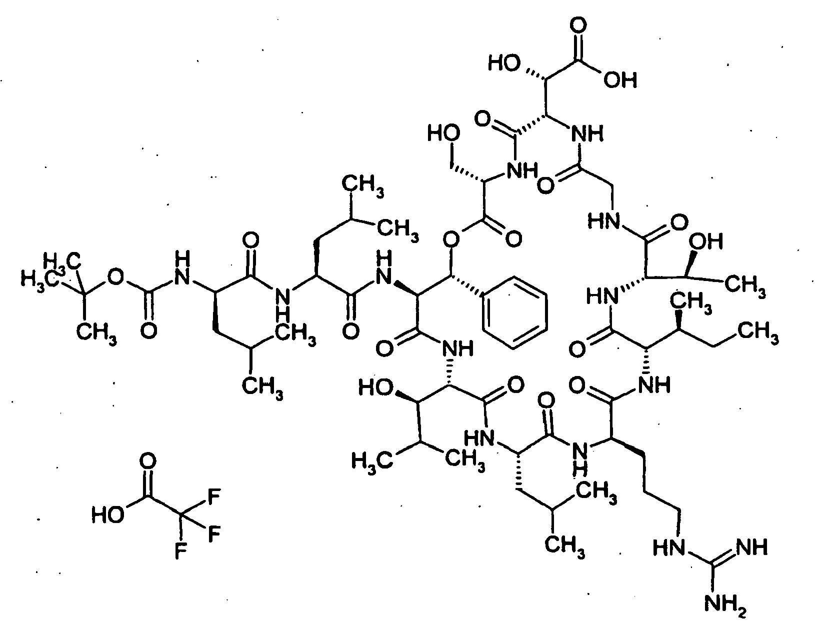

Unter Argon-Schutzgasatmosphäre wird Lysobactinsäure-bis-trifluoracetat (Beispiel 2A, 200.0 mg, 0.13 mmol) in einem Gemisch aus Dioxan (22.4 ml), Puffer pH 6 (11.2 ml, Fa. Riedel de Haën, mit Fungizid) und Phosphat-Puffer pH 7 (11.2 ml, Fa. Dr. Lang, LCX021) gelöst. Anschließend werden nacheinander bei 0°C Di-*tert-*butyldicarbonat (34.8 mg, 0.16 mmol, 1.2 Äquivalente) und *N*,*N*-Diisopropylethylamin (28 µl, 20.6 mg, 0.16 mmol, 1.2 Äquivalente) zugegeben und man rührt über Nacht bei RT nach. Nach weiterer Zugabe von Di-*tert*-butyldicarbonat (29.0 mg, 0.13 mmol, 1 Äquivalent) und *N*,*N*-Diisopropylethylamin (14 µl, 10.3 mg, 0.08 mmol, 0.6 Äquivalente) bei RT und dreistündigem Rühren bei RT wird ein vollständiger Umsatz erreicht. Zur Aufarbeitung wird die Reaktionslösung direkt auf die präparative RP-HPLC (Methode 7) gegeben. Man erhält 147.9 mg (75% d. Th.) von der Titelverbindung.

HPLC (Methode 15) Rₜ = 23.10 min

LC-MS (Methode 10): Rₜ = 2.28 min; MS (ESIpos): *m*/*z* (%) = 1378 (34) [M + H]⁺, 639 (100); MS (ESIneg): *m*/*z* (%) = 1376 (73) [M - H]⁻, 688 (100) [M - 2H]²⁻.

### Beispiel 5A

### N^{2.1}-[Benzyloxycarbonyl)]-N^{4.10}-(2-morpholin-4-ylethyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bis-trifluoracetat

### (N^{2.1}-[Benzyloxycarbonyl]-N^{4.10}-(2-moipholin-4-ylethyl)-lysobactin-bis-trifluoracetat)

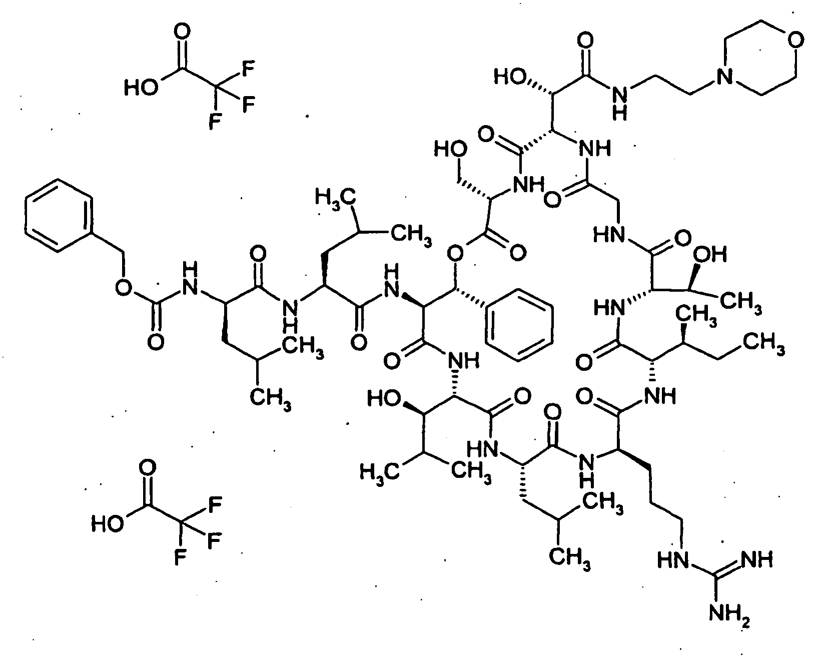

Nach der Arbeitsvorschrift 3 werden das Cyclopeptid (Beispiel 3A, 4.0 mg, 2.62 µmol), *N*-(2-Aminoethyl)morpholin (3 µl, 3.4 mg, 26.2 µmol, 10 Äquivalente) und HATU (11.7 mg, 31.46 µmol, 12 Äquivalente) in DMF (500 µl) über Nacht zum Amid bei RT umgesetzt. Nach präparativer HPLC (Methode 6) werden 3.0 mg (65% d. Th.) der Titelverbindung erhalten.

HPLC (Methode 15): Rₜ = 22.06 min.

LC-MS (Methode 10): Rₜ = 1.88 min; MS (ESIpos.): *m*/*z* (%) = 763 (100) [M + 2H]²⁺; ESIneg: *m*/*z* (%) = 1522 (50) [M - H]⁻, 761 (100) [M - 2H]²⁻.

HR-TOF-MS (Methode 11): C₇₂H₁₁₅N₁₆O₂₀ ber. 1523.8469, gef. 1523.8517 [M + H]⁺.

### Beispiel 6A

### N^{2.1}-[Benzyloxycarbonyl]-N^{4.10}-(2-hydroxyethyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-trifluoracetat

### (N^{2.1}-[Benzyloxycarbonyl]-N^{4.10}-(2-hydroxyethyl)-lysobactin-trifluoracetat)

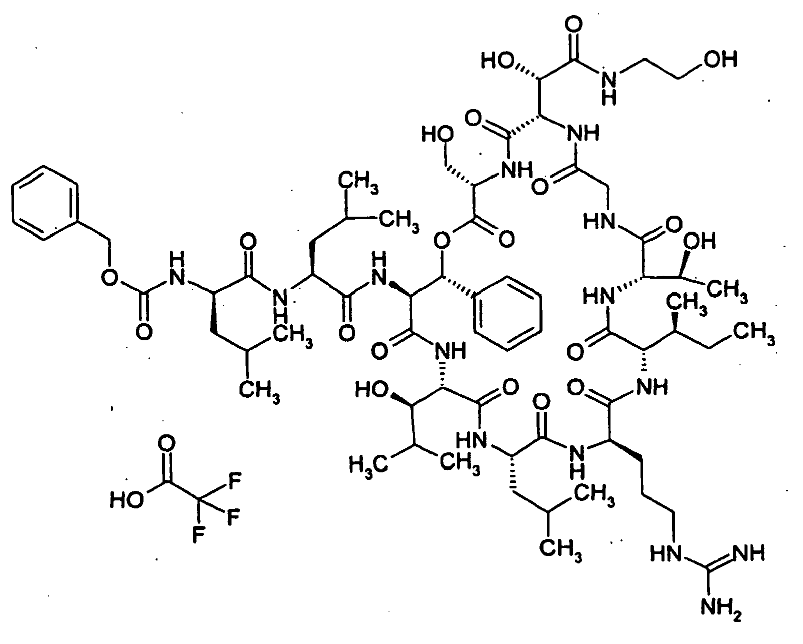

Nach der Arbeitsvorschrift 3 werden das Cyclopeptid (Beispiel 3A, 20.0 mg, 13.11 µmol), Ethanolamin (11 pl, 11.2 mg, 183.53 µmol, 14 Äquivalente), HATU (39.9 mg, 104.88 µmol, 8 Äquivalente) und NMM (12 µl, 10.8 mg, 104.88 µmol, 8 Äquivalente) in DMF (500 µl) über Nacht zum Amid bei RT umgesetzt. Nach präparativer HPLC (Methode 6) erhält man 15.7 mg (76% d. Th.) der Titelverbindung.

HPLC (Methode 15): Rₜ = 21.82 min.

LC-MS (Methode 10): Rₜ = 2.07 min; MS (ESIpos.): *m*/*z* (%) = 1455 (35) [M + H]⁺; 728 (100) [M + 2H]²⁺, ESIneg: *m*/*z* (%) = 1523 (60) [M - H]⁻, 726 (12) [M - 2H]²⁻, 672 (100).

HR-TOF-MS (Methode 11): C₆₈H₁₀₈N₁₅O₂₀ ber. 1454.7890, gef. 1454.7860 [M + H]⁺.

### Beispiel 7A

### N^{2.1}-[Benzyloxycarbonyl]-N^{4.10}-methyl-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-trifluoracetat

### (N^{2.1}-[Benzyloxycarbonyl]-N^{4.10}-methyl-lysobactin-trifluoracetat)

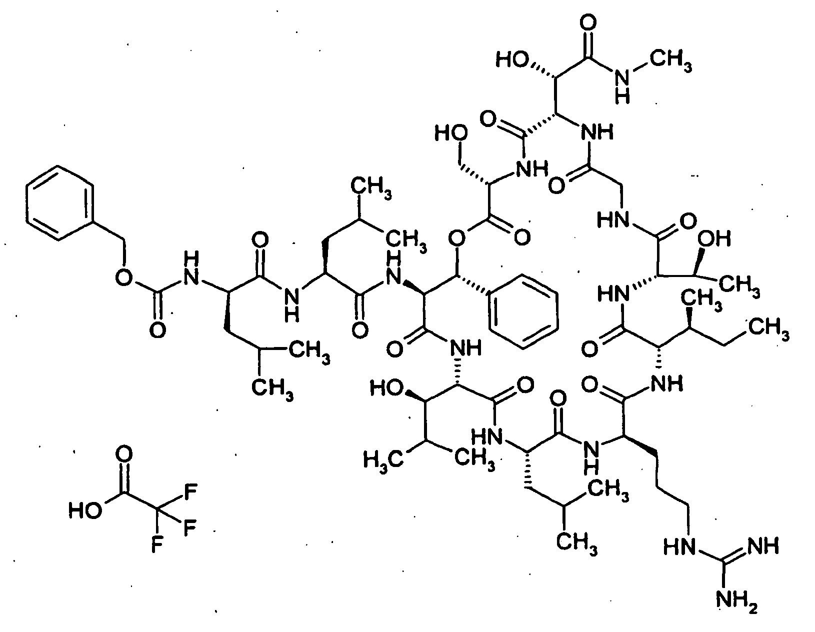

Nach der Arbeitsvorschrift 3 werden das Cyclopeptid (Beispiel 3A, 20.0 mg, 13.11 µmol) und das Methylamin-Hydrochlorid (4.4 mg, 65.55 µmol, 5 Äquivalente) unter Zuhilfenahme von HATU (15.0 mg, 39.33 µmol, 3 Äquivalente) und NMM (20 µl, 18.56 mg, 183.54 µmol, 14 Äquivalente) in DMF (500 µl) innerhalb von 3 Tagen bei 4°C zum Amid umgesetzt. Nach präparativer HPLC (Methode 6) werden 17.0 mg (84% d. Th.) der Titelverbindung erhalten.

HPLC (Methode 15): Rₜ = 21.26 min.

LC-MS (Methode 10): Rₜ = 2.07 min; MS (ESIpos.): *m*/*z* (%) = 1425 (35) [M + H]⁺, 713 (100) [M + 2H]²⁺; ESIneg: *m*/*z* (%) = 1524 (100) [M - H]⁻, 711 (85) [M - 2H]²⁻.

HR-TOF-MS (Methode 11): C₆₇H₁₀₅N₁₅O₁₉ ber. 1424.7824, gef. 1424.7830 [M + H]⁺.

### Beispiel 8A

### N^{2.1}-[Benzyloxycarbonyl]-N^{4.10}-(3-morpholin-4-yl-propyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bis-trifluoracetat

### (N^{2.1}-[Benzyloxycarbonyl]-N^{4.10}-(3-morpholin-4-yl-propyl)-lysobactin-bis-trifluoracetat)

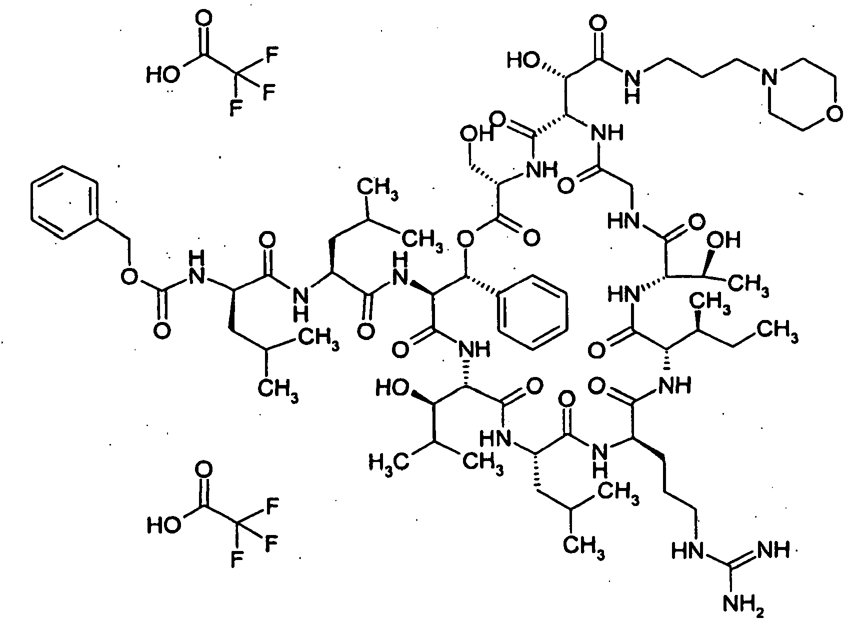

Nach der Arbeitsvorschrift 3 werden das Cyclopeptid (Beispiel 3A, 13.0 mg, 8.52 µmol), 3-(Morpholin-4-yl)propylamin (20 µl, 17.2 mg, 119.28 µmol, 14 Äquivalente), HATU (25.9 mg, 68.16 µmol, 8 Äquivalente) und NMM (7 µl, 6.9 mg, 68.16 µmol, 8 Äquivalente) in DMF (500 µl) innerhalb von 3 Tagen bei 4°C zum Amid umgesetzt. Nach präparativer HPLC (Methode 6) werden 12.0 mg (80% d. Th.) der Titelverbindung erhalten.

HPLC (Methode 15): Rₜ = 22.15 min.

LC-MS (Methode 10): Rₜ = 2.07 min; MS (ESIpos.): *m*/*z* (%) = 770 (100) [M + 2H]²⁺; ESIneg: *m*/*z* (%) = 1537 (100) [M - H]⁻, 768 (75) [M - 2H]²⁻.

HR-TOF-MS (Methode 11): C₇₃H₁₁₇N₁₆O₂₀ ber. 1537.8625, gef. 1537.8623 [M + H]⁺.

### Beispiel 9A

### N^{2.1}-[Benzyloxycarbonyl]-N^{4.10}-(pyridin-3-yl-methyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bis-trifluoracetat

### (N^{2.1}-[Benzyloxycarbonyl]-N^{4.10}-(pyridin-3-yl-methyl)-lysobactin-bis-trifluoracetat)

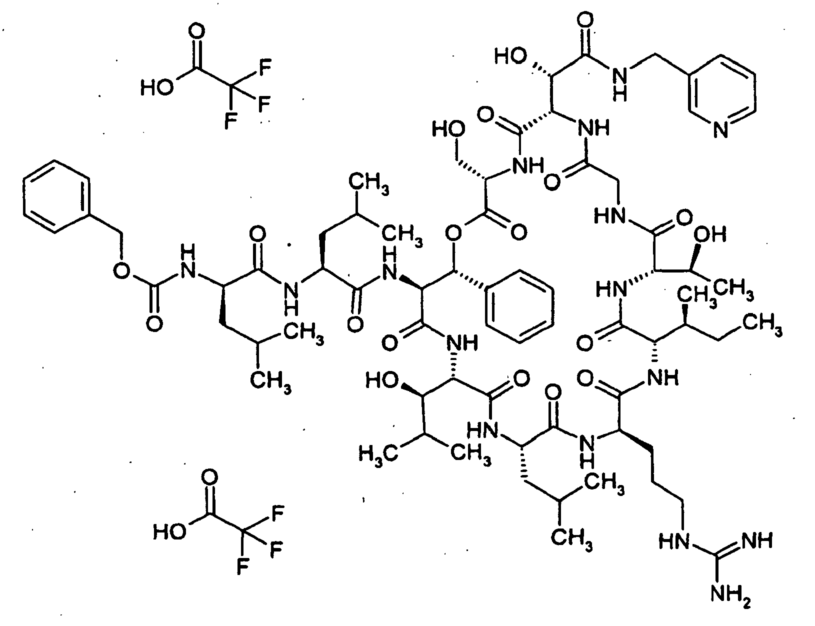

Nach der Arbeitsvorschrift 3 werden das Cyclopeptid (Beispiel 3A, 20.0 mg, 13.11 µmol), 3-Picolylamin (19.5 mg, 183.5 µmol, 14 Äquivalente), HATU (39.9 mg, 104.88 µmol, 8 Äquivalente) und NMM (12 µl, 10.6 mg, 104.88 µmol, 8 Äquivalente) in DMF (500 µl) bei RT über Nacht zum Amid umgesetzt. Nach präparativer HPLC (Methode 6) werden 17.0 mg (84% d. Th.) der Titelverbindung erhalten.

HPLC (Methode 14): Rₜ = 2.11 min.

LC-MS (Methode 10): Rₜ = 1.95 min; MS (ESIpos.): *m*/*z* (%) = 1503 (5) [M + H]⁺, 751 (100) [M + 2H]²⁺; ESIneg: *m*/*z* (%) = 1500 (84) [M - H]⁻, 750 (30) [M - 2H]²⁻, 695 (100).

HR-TOF-MS (Methode 11): C₇₂H₁₀₇N₁₅O₂₀ ber. 1501.7812, gef. 1501.7819 [M + H]⁺.

### Beispiel 10A

### N^{2.1}-[Benzyloxycarbonyl]-N^{4.10}-benzyl-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonylglycyl-[(3S)-3-hydroxy-L-asparaginyl]-L-serin-C^{1.11}-O^{3.3}-lacton-trifluoracetat

### (N^{2.1}-[Benzyloxycarbonyl]-N^{4.10}-benzyl-lysobactin-trifluoracetat)

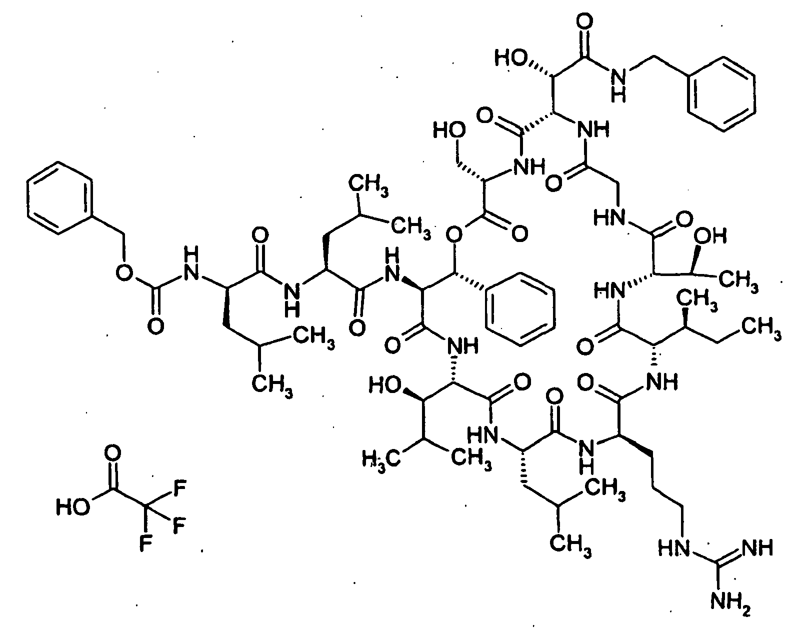

Nach der Arbeitsvorschrift 3 werden das Cyclopeptid (Beispiel 3A, 20.0 mg, 13.11 µmol), Benzylamin (20µl, 19.7 mg, 183.54 µmol, 14 Äquivalente), HATU (39.9 mg, 104.88 µmol, 8 Äquivalente) und NMM (12 µl, 10.6 mg, 104.88 µmol, 8 Äquivalente) in DMF (500 µl) bei RT über Nacht zum Amid umgesetzt. Nach präparativer HPLC (Methode 6) werden 17.6 mg (83% d. Th.) der Titelverbindung erhalten.

HPLC (Methode 15): Rₜ = 23.21 min.

LC-MS (Methode 10): Rₜ = 2.14 min; MS (ESIpos.): *m*/*z* (%) = 1502 (32) [M + H]⁺, 751 (100) [M + 2H]²⁺; ESIneg: *m*/*z* (%) = 1500 (75) [M - H]⁻, 749 (20) [M - 2H]²⁻, 695 (100).

HR-TOF-MS (Methode 11): C₇₃H₁₁₀N₁₅O₁₉ ber. 1500.8097, gef. 1500.8131 [M + H]⁺.

### Beispiel 11A

### N^{2.1}-(tert.-Butoxycarbonyl)-N^{4.10},N^{4.10}-dimethyl-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonylglycyl-[(3S)-3-hydroxy-L-asparaginyl]-L-serin-C^{1.11}-O^{3.3}-lacton-trilluoracetat

### (N^{2.1}-(tert.-Butoxycarbonyl)-N^{4.10},N^{4.10}-dimethyl-lysobactin-trifluoracetat)

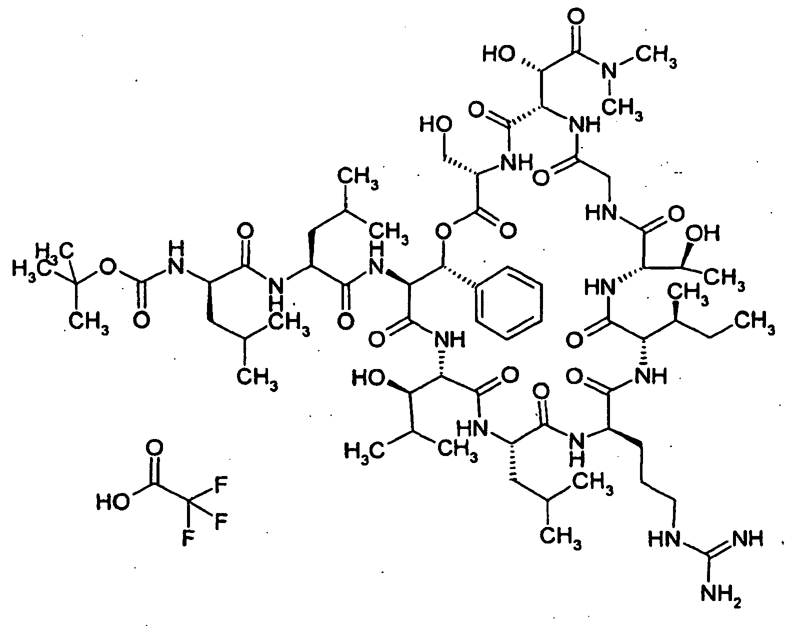

Nach der Arbeitsvorschrift 3 werden das Cyclopeptid (Beispiel 4A, 20.0 mg, 13.41 µmol) und eine 2M Lösung von Dimethylamin (67 µl, 6.0 mg, 134.10 µmol, 10 Äquivalente) in THF unter Zuhilfenahme von HATU (25.5 mg, 67.05 µmol, 5 Äquivalente) und NMM (12 µl, 10.6 mg, 107.28 µmol, 8 Äquivalente) in DMF (500 µl) bei 0°C über Nacht zum Amid umgesetzt. Nach einer Trennung über präparative HPLC (Methode 6) werden 8.5 mg (42% d. Th.) der Titelverbindung erhalten.

HPLC (Methode 15): Rₜ = 23.89 min.

LC-MS (Methode 10): Rₜ = 2.26 min; MS (ESIpos.): *m*/*z* (%) = 1405 (9) [M + H]⁺, 753 (100) [M + 2H]²⁺; ESIneg: *m*/*z* (%) = 1403 (100) [M - H]⁻.

HR-TOF-MS (Methode 11): C₆₅H₁₁₀N₁₅O₁₉ ber. 1404.8097, gef. 1404.8094 [M + H]⁺.

### Beispiel 12A

### N^{2.1}-(tert-Butoxycarbonyl)-N^{4.10}-[2-(benzyloxy)-2-oxoethyl]-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-β-asparaginyl]-L-serin-C^{1.11}-O^{3.3}-lacton-trifluoracetat

### (N^{2.1}-(tert-Butoxycarbonyl)-N^{4.10}-[2-(benzyloxy)-2-oxoethyl]-lysobactin-trifluoracetat)

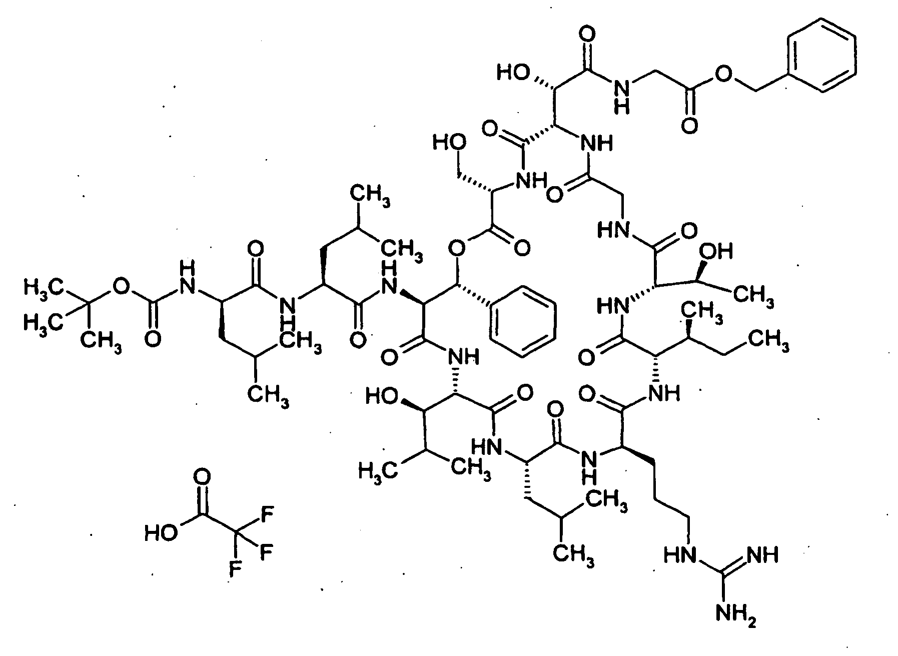

Nach der Arbeitsvorschrift 3 werden das Cyclopeptid (Beispiel 4A, 20.0 mg, 13.41 µmol), Glycinbenzylester-hydrochlorid (21.6 mg, 107.28 µmol, 8 Äquivalente), HATU (25.5 mg, 67.05 µmol, 5 Äquivalente) und NMM (29 µl, 27.1 mg, 268.2 µmol, 20 Äquivalente) in DMF (500 µl) bei RT über Nacht zum Amid umgesetzt. Nach chromatographischer Reinigung (Methode 6) werden 21.5 mg (98% d. Th.) der Titelverbindung erhalten.

HPLC (Methode 15): Rₜ = 23.60 min.

LC-MS (Methode 10): Rₜ = 2.32 min; MS (ESIpos.): *m*/*z* (%) = 1525 (40) [M + H]⁺, 763 (38) [M + 2H]²⁺, 713 (100); ESIneg: *m*/*z* = 1523 (60) [M - H]⁻, 761 (100) [M - 2H]²⁻.

HR-TOF-MS (Methode 11): C₇₂H₁₁₄N₁₅O₂₁ ber. 1524.8309, gef. 1524.8311 [M + H]⁺.

### Beispiel 13A

### N^{2.1}-(tert-Butoxycarbonyl)-N^{4.10}-(2-{[benzyloxycarbonyl]amino}ethyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-trifluoracetat

### (N^{2.1}-(tert-Butoxycarbonyl)-N^{4.10}-(2-{[benzyloxycarbonyl]amino}ethyl)-lysobactintrifluoracetat)

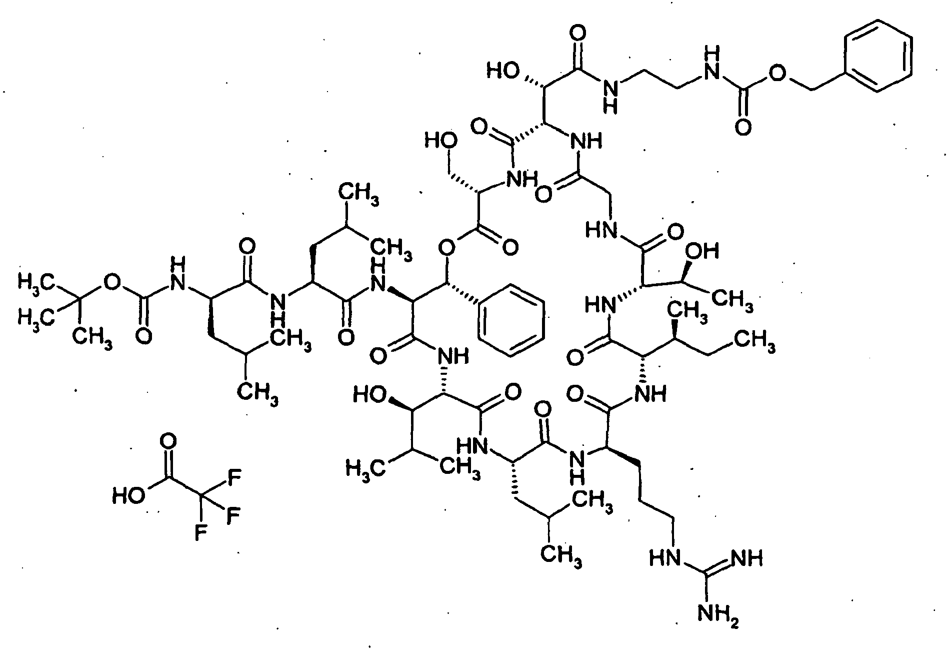

Nach der Arbeitsvorschrift 3 werden das Cyclopeptid (Beispiel 4A, 40.0 mg, 26.82 µmol), Benzyl(2-aminoethyl)carbamat-hydrochlorid (49.5 mg, 214.53 µmol, 8 Äquivalente) unter Zuhilfenahme von HATU (50.982 mg, 134.08 µmol, 5 Äquivalente) und NMM (59 µl, 54.2 mg, 536.31 µmol, 20 Äquivalente) in DMF (500 µl) bei RT über Nacht zum Amid umgesetzt. Nach einer chromatographischen Reinigung (Methode 6) werden 40.2 mg (90% d. Th.) der Titelverbindung erhalten.

HPLC (Methode 15): Rₜ = 23.64 min.

LC-MS (Methode 10): Rₜ = 2.30 min; MS (ESIpos.): *m*/*z* (%) = 1554 (100) [M + H]⁺, 777 (53) [M + 2H]²⁺; ESIneg: *m*/*z* (%) = 1552 (19) [M - H]⁻, 775 (100) [M - 2H]²⁻.

HR-TOF-MS (Methode 11): C₇₃H₁₁₇N₁₆O₂₁ ber. 1553.8574, gef. 1553.8595 [M + H]⁺.

### Beispiel 14A

### N^{4,10}-(2-Amino-2-oxoethyl)-N^{2.1}-(tert-butoxycarbonyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-trifluoracetat

### (N^{4,10}-(2-Amino-2-oxoethyl)-N^{2.1}-(tert-butoxycarbonyl)-lysobactin-trifluoracetat)

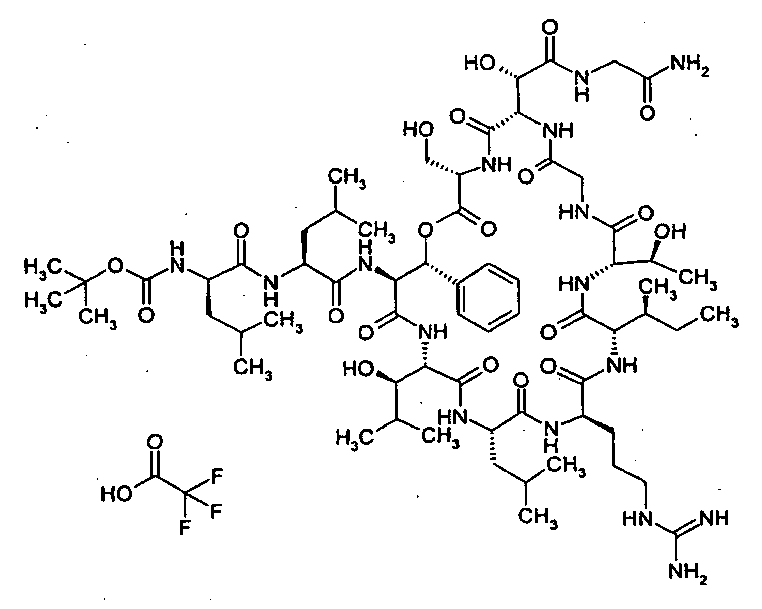

Nach der Arbeitsvorschrift 3 werden das Cyclopeptid (Beispiel 4A, 20.0 mg, 13.41 µmol) und Glycinamin-hydrochlorid (11.9 mg, 107.28 µmol, 8 Äquivalente) unter Zuhilfenahme von HATU (25.5 mg, 67.05 µmol, 5 Äquivalente) und NMM (29 µl, 10.6 mg, 268.20 µmol, 20 Äquivalente) in DMF (500 µl) bei RT über Nacht zum Amid umgesetzt. Nach einer chromatographischen Reinigung (Methode 6) werden 12.8 mg (60% d. Th.) der Titelverbindung erhalten.

HPLC (Methode 15): Rₜ = 21.97 min.

LC-MS (Methode 10): Rₜ = 2.19 min; MS (ESIpos.): *m*/*z* (%) = 1435 (23) [M + H]⁺, 718 (18) [M + 2H]²⁺, 667 (100); ESIneg: *m*/*z* (%) = 1433 (13) [M - H]⁻, 716 (100) [M - 2H]²⁻.

HR-TOF-MS (Methode 11): C₆₅H₁₀₉N₁₆O₂₀ ber. 1433.7999, gef. 1433.8000 [M + H]⁺.

### Beispiel 15A

### N^{2.1}-(tert-Butoxycarbonyl)-N^{4.10}-[2-(2-{[bis(dimethylamino)methylen]amino}ethoxy)-ethyl]-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bis-trifluoracetat

### (N^{2.1}-(tert-Butoxycarbonyl)-N^{4.10}-[2-(2-{[bis(dimethylamino)methylen]aminio}ethoxy)-ethyl]-lysobactin-bis-trifluoracetat)

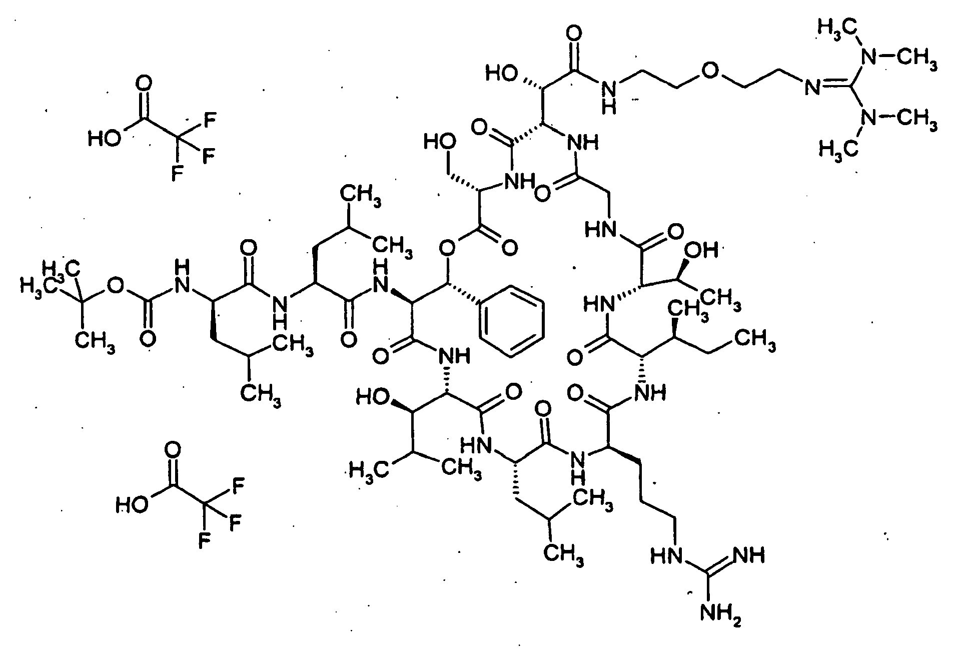

Nach der Arbeitsvorschrift 3 werden das Cyclopeptid (Beispiel 4A, 20.0 mg, 13.41 µmol) und 2,2'-Oxydiethylamin-hydrochlorid (1.4 mg, 8.05 µmol, 0.6 Äquivalente) unter Zuhilfenahme von HATU (15.3 mg, 40.23 µmol, 3 Äquivalente) und NMM (12 µl, 10.9 mg, 107.28 µmol, 8 Äquivalente) in DMF (500 µl) bei RT über Nacht zum Amid umgesetzt. Nach einer chromatographischen Reinigung (Methode 6) werden 5.3 mg (22% d. Th.) der Titelverbindung erhalten.

HPLC (Methode 15): Rₜ = 21.81 min.

LC-MS (Methode 10): Rₜ = 2.02 min; MS (ESIpos.): *m*/*z* (%) = 782 (100) [M + 2H]²⁺; ESIneg: *m*/*z* (%) = 1560 (83) [M - H]⁻, 780 (28) [M - 2H]²⁻, 1606 (100).

HR-TOF-MS (Methode 11): C₇₂H₁₂₅N₁₈O₂₀ ber. 1561.9313, gef. 1561.9352 [M + H]⁺.

### Beispiel 16A

### N^{4.10}-(2-Aminoethyl)-N^{2.1}-(tert-butoxycarbonyl)-D-leucyl-L-leucyl-[(3R)-3-hydroxyL-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bis-trifluoracetat

### (N^{4.10}-(2-Aminoethyl)-N^{2.1}-(tert-butoxycarbonyl)-lysobactin-bis-trifluoracetat)

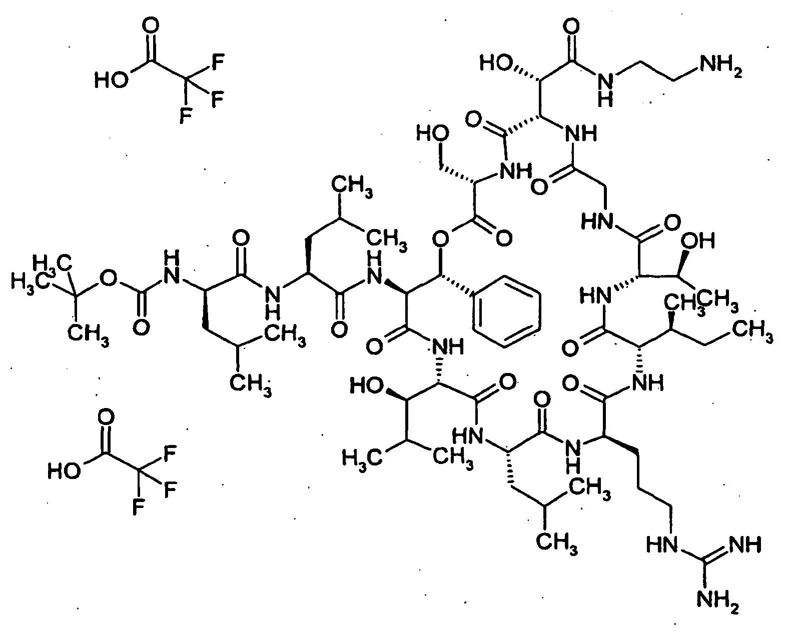

Gemäß der Arbeitsvorschrift 2 wird von der Verbindung aus Beispiel 13A (19.0 mg, 11.39 µmol) die Cbz-Schutzgruppe hydrogenolytisch abgespalten. Man erhält nach der Feinreinigung mittels präparativer HPLC (Methode 6) 14.8 mg (78.9% d.Th.) der Titelverbindung.

HPLC (Methode 15): Rₜ = 21.13 min.

LC-MS (Methode 10): Rₜ = 1.91 min; MS (ESIpos.): *m*/*z* (%) = 1420 (5) [M + H]⁺, 710 (100) [M + 2H]²⁺; ESIneg: *m*/*z* (%) = 1418 (55) [M - H]⁻, 709 (100) [M - 2H]²⁻.

HR-TOF-MS (Methode 11): C₆₅H₁₁₁N₁₆O₁₉ ber. 1419.8206, gef. 1419.8221 [M + H]⁺.

### Beispiel 17A

### [3-tert-Butyl-D-alanyl]-[3-tert-butyl-L-alanyl]-[(3R)-3-hydroxy-L-phenylalanyl)]-[(3R)-3-hydroxy-L-leucyl]-L-leucyl-D-arginyl-L-isoleucyl-L-allothreonyl-glycyl-[(3S)-3-hydroxyL-aspartyl]-L-serin-C^{1.11}-O^{3.3}-lacton-bis-trifluoracetat

### (C^{4.1},C^{4.2}-Dimethyl-lysobactinsäure-bis-trifluoracetat)

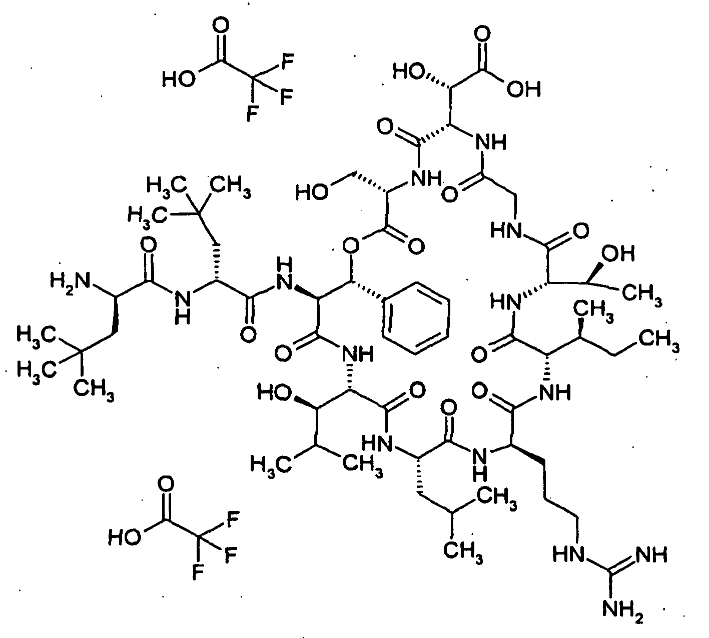

Eine Suspension von [3-*tert*-Butyl-D-alanyl]-[3-*tert*-butyl-L-alanyl]-[(3*R*)-3-hydroxy-L-phenylalanyl)]-[(3*R*)-3-hydroxy-L-leucyl]-L-leucyl-D-arginyl-L-isoleucyl-L-allothreonyl-glycyl-[(3*S*)-3-hydroxy-L-asparaginyl]-L-serin-*C^{1.11}*-*O^{3.3}*-lacton-bis-trifluciracetat (150.0 mg, 98 µmol) in Dioxan/50% Wasser (3 ml) wird mit 6 N Salzsäure (9 ml) versetzt und in 5 Tagen unter Rühren zum vollständigen Umsatz gebracht. Die Reaktion wird ständiger HPLC-Kontrolle unterworfen. Zur Aufarbeitung wird das Lösungsmittel am Rotationsverdampfer bei 30°C Badtemperatur entfernt und der Rückstand mittels präparativer RP-HPLC (Methode 6) gereinigt. Man erhält 92.8 mg (67% d.Th.) der Titelverbindung.

HPLC (Methode 3) Rₜ = 1.85 min

LC-MS (Methode 10): Rₜ = 1.74 min, MS (ESIpos.): m/z (%) = 653.7 (100) [M+2H]²⁺, 1306.0 (10) [M+H]⁺; MS (ESIneg): m/z (%) = 651.7 (100) [M-H]⁻, 1339.9 (90) [M-H]⁻.

HR-TOF-MS (Methode 11): C₆₀H₁₀₁N₁₄O₁₈ [M+H]⁺ ber.: 1305.7413, gef.: 1305.7433.

### Ausführungsbeispiele

### Beispiel 1

### N^{4.10}-(3-Morpholin-4-ylpropyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-tris-trifluoracetat

### (N^{4.10}-(3-Morpholin-4-ylpropyl)-lysobactin-tris-trifluoracetat)

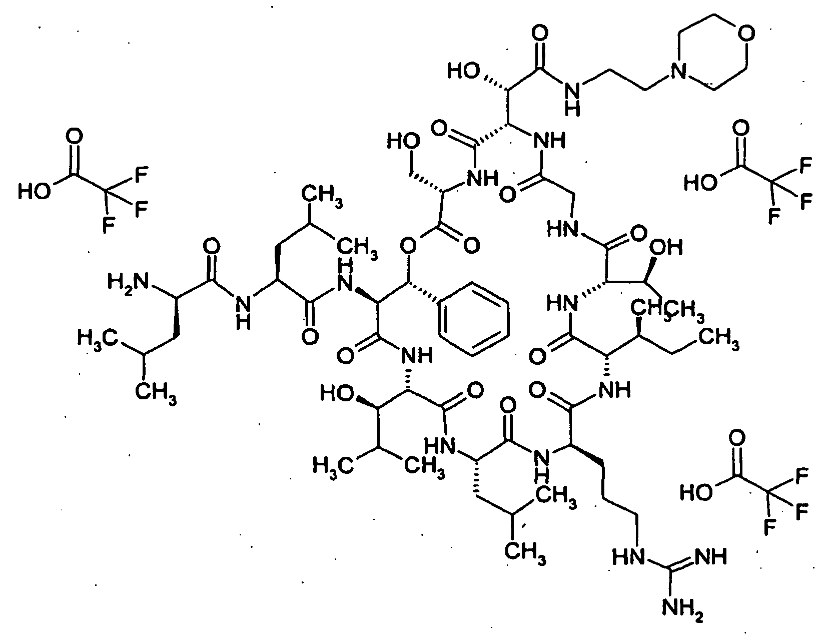

Gemäß der Arbeitsvorschrift 2 wird von der Verbindung aus Beispiel 5A (2.5 mg, 1.43 µmol) die Benzyloxycarbonyl-Schutzgruppe ydrogenolytisch abgespalten. Nach der Feinreinigung (Methode 6) und Gefriertrocknung wird die Titelverbindung (2.0 mg, 81% d. Th.) als amorpher Feststoff erhalten.

HPLC (Methode 14): Rₜ = 1.57 min.

LC-MS (Methode 10): Rₜ = 1.91 min; MS (ESIpos.): *m*/*z* (%) = 1390 (3) [M + H]⁺, 695 (94) [M + 2H]²⁺, 464 (100); ESIneg: *m*/*z* (%) = 1388 (88) [M - H]⁻, 693 (100) [M - 2H]²⁻.

HR-TOF-MS (Methode 11): C₆₄H₁₀₉N₁₆O₁₈ ber. 1389.8101, gef. 1389.8116 [M + H]⁺.

### Beispiel 2

### N^{4.10}-(2-Hydroxyethyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bis-trifluoracetat

### (N^{4.10}-(2-Hydroxyethyl)-lysobactin-bis-trifluoracetat)

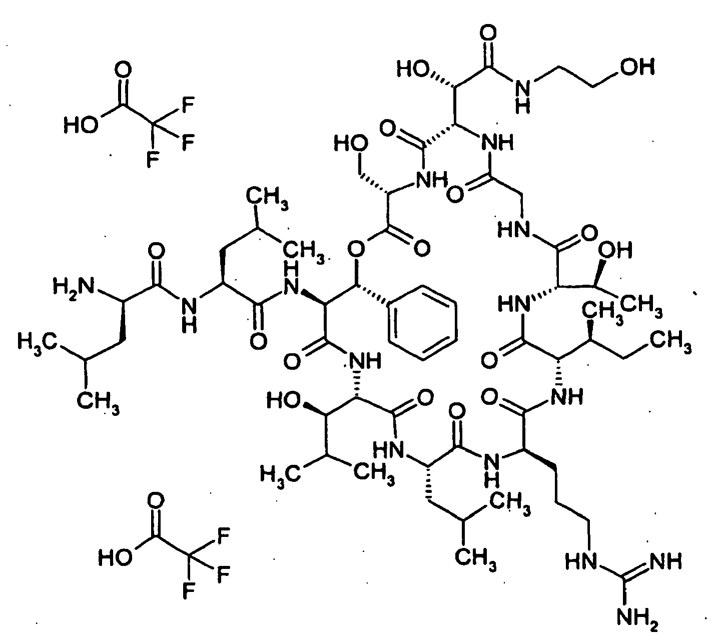

Gemäß der Arbeitsvorschrift 2 wird von der Verbindung aus Beispiel 6A (14.5 mg, 9.24 µmol) die Benzyloxycarbonyl-Schutzgruppe hydrogenolytisch abgespalten. Nach der Feinreinigung (Methode 6) und einer Gefriertrocknung wird die Titelverbindung (9.4 mg, 66% d. Th.) als amorpher Feststoff erhalten.

HPLC (Methode 15): Rₜ = 15.70 min.

LC-MS (Methode 10): Rₜ = 1.50 min; MS (ESIpos.): *m*/*z* (%) = 1321 (8) [M + H]⁺, 661 (100) [M + 2H]²⁺; ESIneg: *m*/*z* (%) = 1318 (72) [M - H]⁻, 659 (100) [M - 2H]²⁻.

HR-TOF-MS (Methode 11): C₆₀H₁₀₂N₁₅O₁₈ ber. 1320.7522, gef. 1320.7504 [M + H]⁺.

### Beispiel 3

### N^{4.10}-Methyl-D-leucyl-L-leucyl-[(3R)-3-hydroxy-L-phenylalanyl]-[(3R)-3-hydroxy-L-leucyl]-L-leucyl-D-arginyl-L-isoleueyl-L-allothreonyl-glycyl-[(3S)-3-hydroxy-L-asparaginyl]-L-serin-C^{1.11}-O^{3.3}-lacton-bis-trifluoracetat

### (N^{4.10}-Methyl-lysobactin-bis-trifluoracetat)

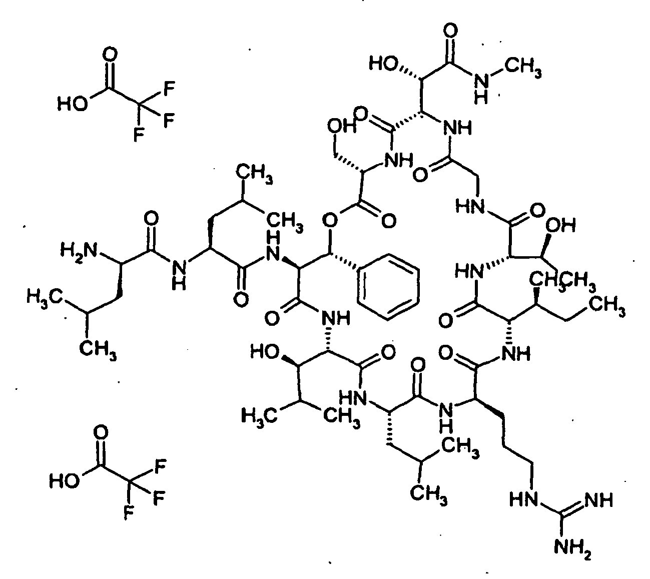

Gemäß der Arbeitsvorschrift 2 wird von der Verbindung aus Beispiel 7A (16.0 mg, 10.40 µmol) die Benzyloxycarbonyl-Schutzgruppe hydrogenolytisch abgespalten. Nach der Feinreinigung (Methode 6) und einer Gefriertrocknung wird die Titelverbindung (10.9 mg, 69% d. Th.) als amorpher Feststoff erhalten.

HPLC (Methode 15): Rₜ = 15.79 min.

LC-MS (Methode 10): Rₜ = 1.50 min; MS (ESIpos.): *mlz* (%) = 1291 (12) [M + H]⁺, 646 (100) [M + 2H]²⁺; ESIneg: *m*/*z* (%) = 1289 (100) [M - H]⁻, 644 (37) [M - 2H]²⁻.

HR-TOF-MS (Methode 11): C₅₉H₁₀₀N₁₅O₁₇ ber. 1290.7417, gef. 1290.7404 [M + H]⁺.

### Beispiel 4

### N^{4.10}-(3-Morpholin-4-yl-propyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-senn-C^{1.11}-O^{3.3}-lacton-tris-trifluoracetat

### (N^{4.10}-(3-Morpholin-4-yl-propyl)-lysobactin-tris-trifluoracetat)

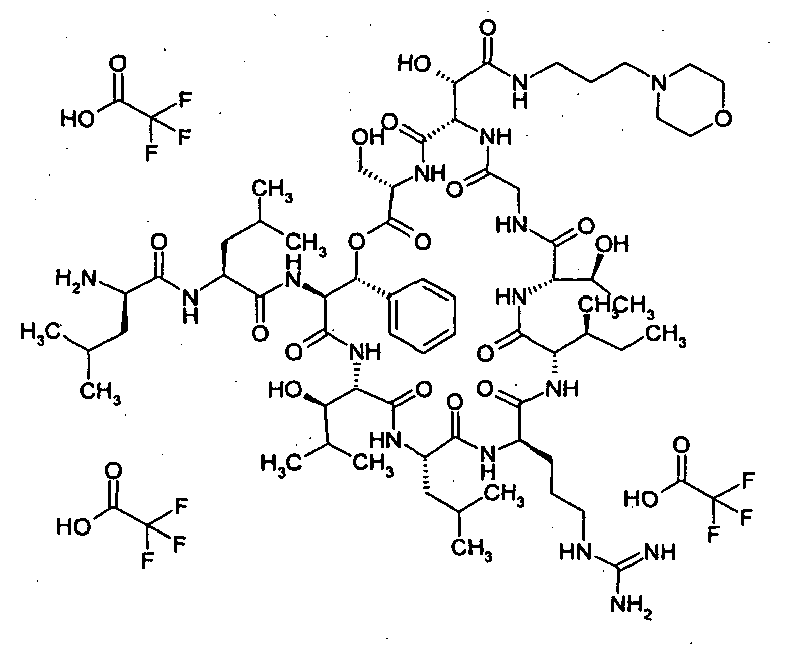

Gemäß der Arbeitsvorschrift 2 wird von der Verbindung aus Beispiel 8A (11.0 mg, 6.23 µmol) die Benzyloxycarbonyl-Schutzgruppe hydrogenolytisch abgespalten. Nach der Feinreinigung (Methode 6) und einer Gefriertrocknung wird die Titelverbindung (9.2 mg, 85% d. Th.) als amorpher Feststoff erhalten.

HPLC (Methode 15): Rₜ = 15.61 min.

LC-MS (Methode 10): Rₜ = 1.38 min; MS (ESIpos.): *m*/*z* (%) = 702 (58) [M + 2H]²⁺, 469 (100); ESIneg: *m*/*z* (%) = 1402 (22) [M - H]⁻, 701 (100) [M - 2H]²⁻.

HR-TOF-MS (Methode 11): C₆₅H₁₁₁N₁₆O₁₈ ber. 1403.8257, gef. 1403.8289 [M + H]⁺.

### Beispiel 5

### N^{4.10}-(Pyridin-3-yl-methyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-tris-trifluoracetat

### (N^{4.10}-(Pyridin-3-yl-methyl)-lysobactin-tris-trifluoracetat)

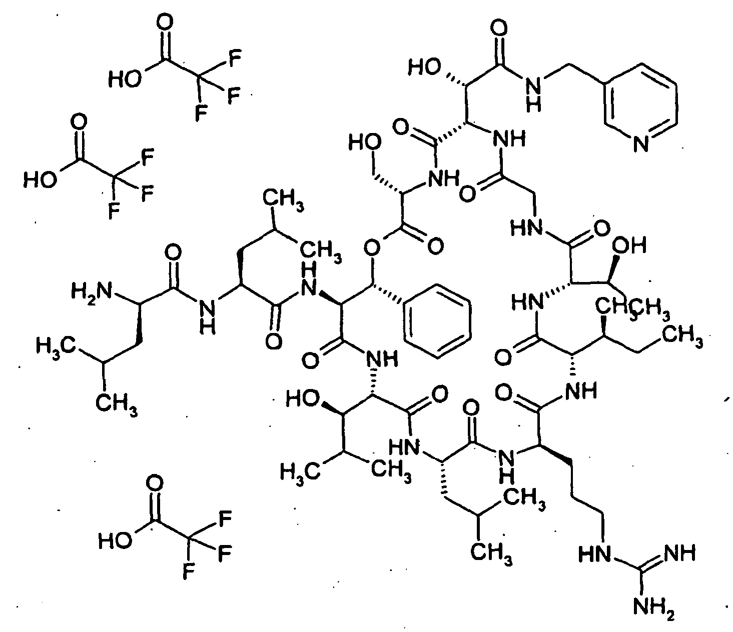

Gemäß der Arbeitsvorschrift 2 wird von der Verbindung aus Beispiel 9A (8.5 mg, 4.91 µmol) die Benzyloxycarbonyl-Schutzgruppe hydrogenolytisch abgespalten. Nach der Feinreinigung (Methode 6) und einer Gefriertrocknung wird die Titelverbindung (7.S mg, 89% d. Th.) als amorpher Feststoff erhalten.

HPLC (Methode 15): Rₜ = 15.56 min.

LC-MS (Methode 10): Rₜ = 1.45 min; MS (ESIpos.): *m*/*z* (%) = 1368 (5) [M + H]⁺, 684 (72) [M + 2H]²⁺, 456 (100); ESIneg: *m*/*z* (%) = 1366 (72) [M - H]⁻, 682 (100) [M - 2H]²⁻.

HR-TOF-MS (Methode 11): C₆₄H₁₀₃N₁₆O₁₇ ber. 1367.7682, gef. 1367.7684 [M + H]⁺.

### Beispiel 6

### N^{4.10}-Benzyl-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bis-trifluoracetat

### (N^{4.10}-Benzyl-lysobactin-bis-trifluoracetat)

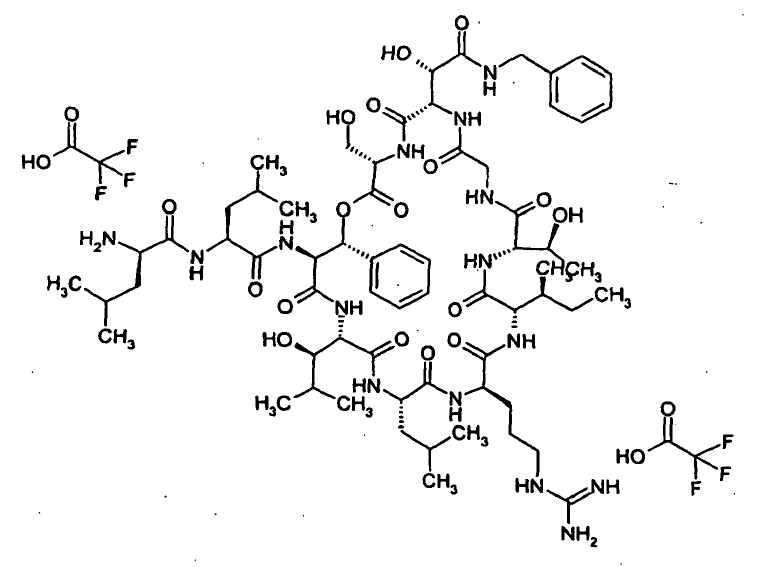

Gemäß der Arbeitsvorschrift 2 wird von der Verbindung aus Beispiel 10A (16.0 mg, 9.91 µpmol) die Benzyloxycarbonyl-Schutzgruppe hydrogenolytisch abgespalten. Nach der Feinreinigung (Methode 6) und einer Gefriertrocknung wird die Titelverbindung (12.7 mg, 80% d. Th.) als amorpher Feststoff erhalten.

HPLC (Methode 15): Rₜ = 17.09 min.

LC-MS (Methode 10): Rₜ = 1.60 min; MS (ESIpos.): *m*/*z* (%) = 1366 (15) [M + H]⁺, 684 (100) [M + 2H]²⁺; ESIneg: *m*/*z* (%) = 1364 (100) [M - H]⁻, 682 (100) [M - 2H]²⁻.

HR-TOF-MS (Methode 11): C₆₅S₁₀₄N₁₅O₁₇ ber. 1366.7730, gef. 1366.7698 [M + H]⁺.

### Beispiel 7

### N^{4.10},N^{4.10}-Dimethyl-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bis-trifluoracetat

### (N^{4.10},N^{4.10}-Dimethyl-lysobactin-bis-trifluoracetat)

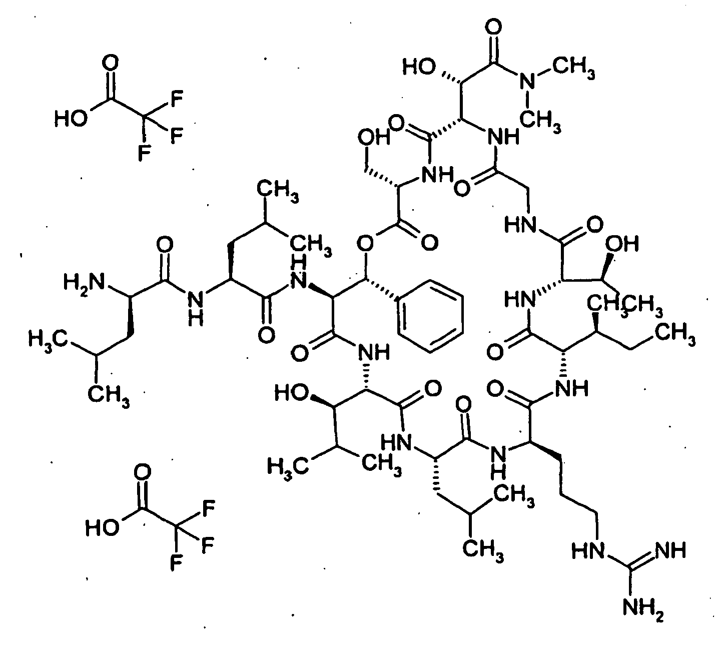

Gemäß der Arbeitsvorschrift 1 wird von der Verbindung aus Beispiel 11A (7.0 mg, 4.61 µmol) die Boc-Schutzgruppe abgespalten. Nach der Feinreinigung (Methode 6) und Gefriertrocknung wird die Titelverbindung (3.6 mg, 51% d. Th.) als amorpher Feststoff erhalten.

HPLC (Methode 15): Rₜ = 16.13 min.

LC-MS (Methode 10): Rₜ = 1.50 min; MS (ESIpos.): *m*/*z* (%) = 1305 (15) [M + H]⁺, 653 (100) [M + 2H]²⁺; ESIneg: *m*/*z* (%) = 1303 (100) [M - H]⁻, 651 (100) [M - 2H]²⁻.

R-TOF-MS (Methode 11): C₆₀H₁₀₂N₁₅O₁₇ ber. 1304.7573, gef. 1304.7615 [M + H]⁺.

### Beispiel 8

### N^{4.10}-(2-(Benzyloxy)-2-oxoethyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-β-asparaginyl]-L-serin-C^{1.11}-O^{3.3}-lacton-bis-trifluoracetat

### (N^{4.10}-(2-(Benzyloxy)-2-oxoethyl)-lysobactin-bis-trifluoracetat)

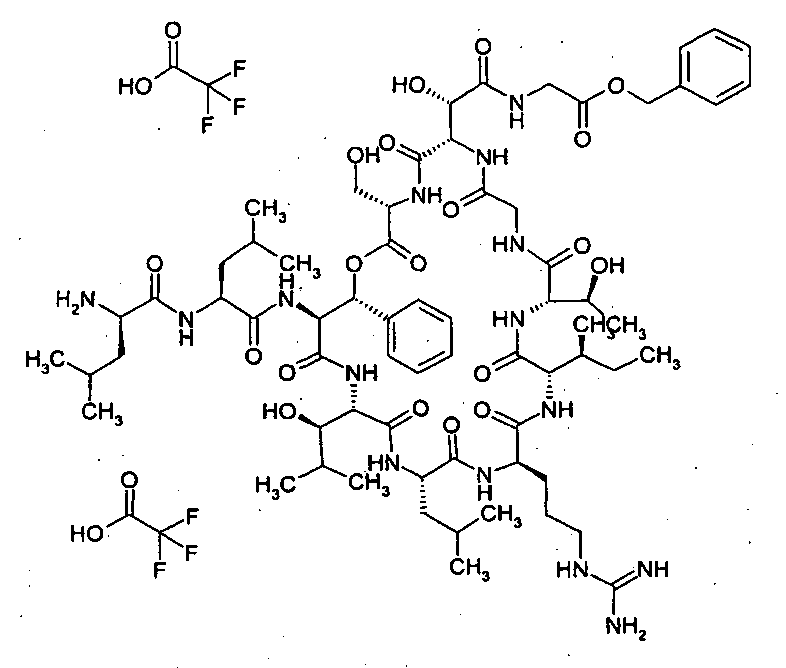

Gemäß der Arbeitsvorschrift 1 wird von der Verbindung aus Beispiel 12A (19.0 mg, 11.59 µmol) die Boc-Schutzgruppe abgespalten. Nach der Feinreinigung (Methode 6) und Gefriertrocknung wird die Titelverbindung (16.0 mg, 84% d. Th.) als amorpher Feststoff erhalten.

HPLC (Methode 15): Rₜ = 17.81 min.

LC-MS (Methode 10): Rₜ = 1.64 min; MS (ESIpos.): *m*/*z* (%) = 1425 (3) [M + H]⁺, 613 (100) [M + 2H]²⁺; ESIneg: *m*/*z* (%) = 1423 (18) [M - H]⁻, 711 (100) [M - 2H]²⁻.

HR-TOF-MS (Methode 11): C₆₇H₁₀₆N₁₅O₁₉ ber. 1424.7784, gef. 1424.7782 [M + H]⁺.

### Beispiel 9

### N^{4.10}-(2-([(Benzyloxy)carbonyl]amino)ethyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonylglycyl-[(3S)-3-hydroxy-L-asparaginyl]-L-serin-C^{1.11}-O^{3.3}-lacton-bis-trifluoracetat

### (N^{4.10}-(2-{[(Benzyloxy)carbonyl]amino}ethyl)-lysobactin-bis-trifluoracetat)

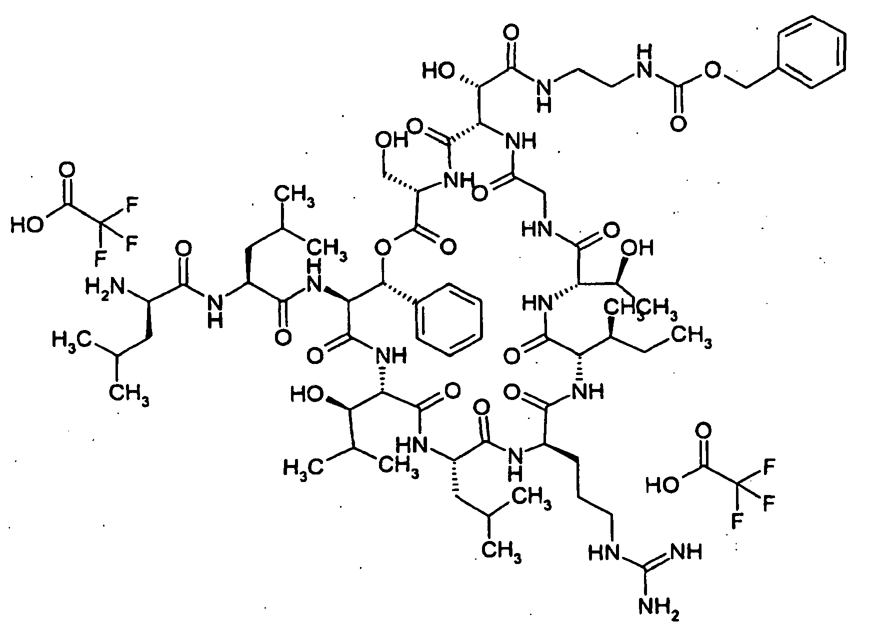

Gemäß der Arbeitsvorschrift 1 wird von der Verbindung aus Beispiel 13A (10.0 mg, 6.00 µmol) die Boc-Schutzgruppe abgespalten. Nach der Feinreinigung (Methode 6) und Gefriertrocknung wird die Titelverbindung (10.0 mg, 99% d. Th.) als amorpher Feststoff erhalten.

HPLC (Methode 15): Rₜ = 18.07 min.

LC-MS (Methode 10): Rₜ = 1.64 min; MS (ESIpos.): *m*/*z* (%) = 1455 (5) [M + H]⁺, 728 (100) [M + 2H]²⁺; ESIneg: *m*/*z* (%) = 1453 (15) [M - H]-, 726 (100) [M - 2H]²⁻.

HR-TOF-MS (Methode 11): C₆₈H₁₀₉N₁₆O₁₉ ber. 1453.8050, gef. 1453.8058 [M + H]⁺.

### Beispiel 10

### N^{4.10}-(2-Amino-2-oxoethyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.3}-lacton-bis-trifluoracetat

### (N^{4.10}-(2-Amino-2-oxoethyl)-lysobactin-bis-trifluoracetat)

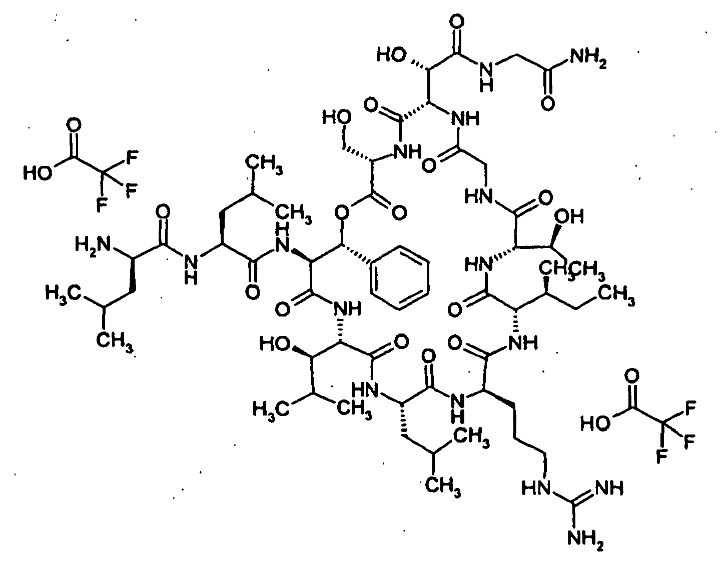

Gemäß der Arbeitsvorschrift 1 wird von der Verbindung aus Beispiel 14A (11.0 mg, 7.11 µmol) die Boc-Schutzgruppe abgespalten. Nach der Feinreinigung (Methode 6) und Gefriertrocknung wird die Titelverbindung (7.2 mg, 65% d. Th.) als amorpher Feststoff erhalten.

HPLC (Methode 15): Rₜ = 15.60 min.

LC-MS (Methode 10): Rₜ = 1.48 min; MS (ESIpos.): *m*/*z* (%) = 1334 (6) [M + H]⁺, 667 (100) [M + 2H]²⁺; ESIneg: *m*/*z* (%) = 1332 (20) [M - H]⁻, 665 (100) [M - 2H]²⁻.

HR-TOF-MS (Methode 11): C₆₀H₁₀₁N₁₆O₁₈ ber. 1333.7475, gef. 1333.7477 [M + H]⁺.

### Beispiel 11

### N^{4.10}-[2-(2-{[Bis(dimethylamino)methylen]amino}ethoxy)ethyl]-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonylglycyl-[(3S)-3-hydroxy-L-asparaginyl]-L-serin-C^{1.11}-O^{3.3}-lactontris-trifluoracetat

### (N^{4.10}-[2-(2-{[Bis(dirnethylamino)methylen]amino}ethoxy)ethyl]-lysobactin-tris-trifluoracetat)

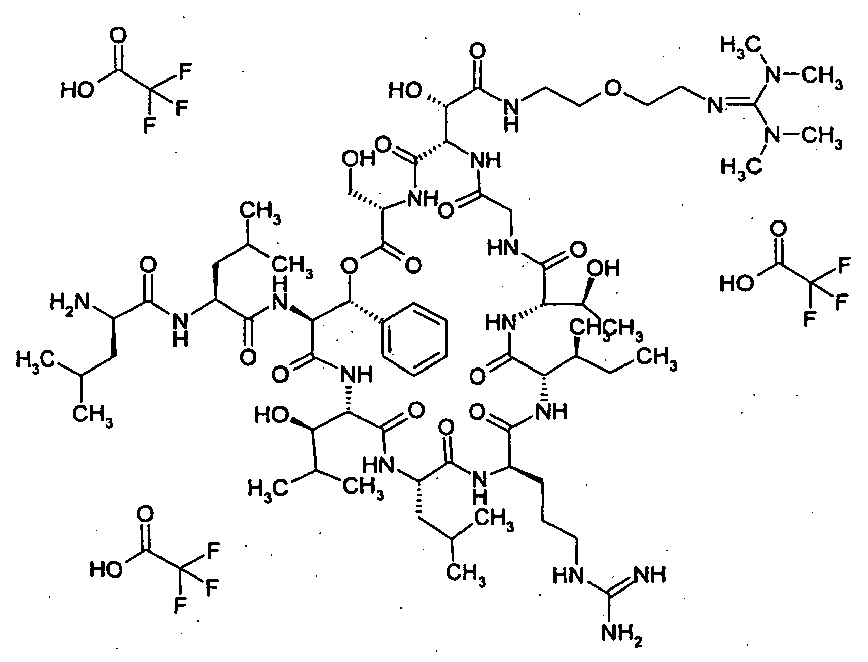

Gemäß der Arbeitsvorschrift 1 wird von der Verbindung aus Beispiel 15A (4.0 mg, 2.22 µmol) die Boc-Schutzgruppe abgespalten. Nach der Feinreinigung (Methode 6) und Gefriertrocknung wird die Titelverbindung (2.7 mg, 67% d. Th.) als amorpher Feststoff erhalten.

HPLC (Methode 15): Rₜ = 16.00 min.

LC-MS (Methode 10): Rₜ = 1.40 min; MS (ESIpos.): *m*/*z* (%) = 732 (48) [M + 2H]²⁺, 488 (100); ESIneg: *m*/*z* (%) = 1462 (20) [M - H]-, 1506 (100).

HR-TOF-MS (Methode 11): C₆₇H₁₁₇N₁₈O₁₈ ber. 1461.8788, gef. 1461.8805 [M + H]⁺.

### Beispiel 12

### N^{4.10}-(2-Aminoethyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-O^{3.}³-lacton-tris-trifluoracetat

### (N^{4.10}-(2-Aminoethyl)-lysobactin-tris-trifluoracetat)

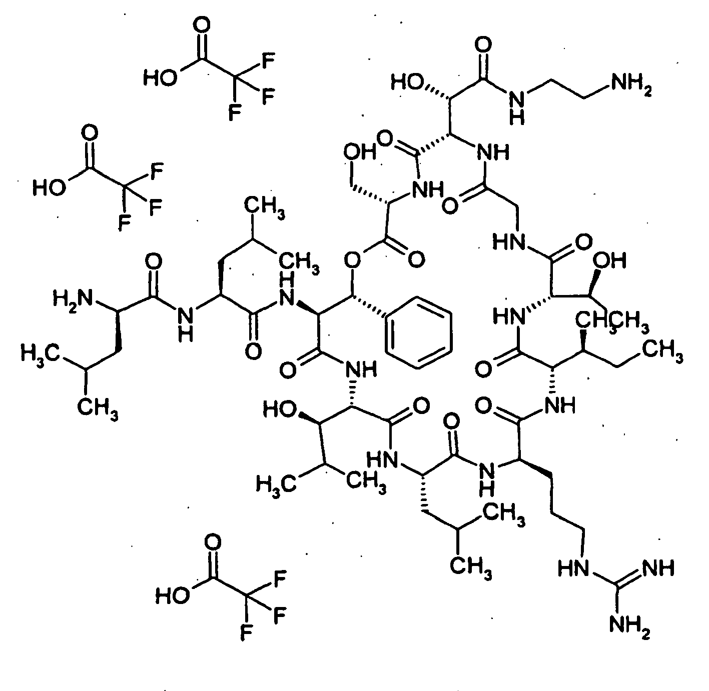

Gemäß der Arbeitsvorschrift 2 wird von der Verbindung aus Beispiel 9 (8.0 mg, 4.76 µmol) die Benzyloxycarbonyl-Schutzgruppe hydrogenolytisch abgespalten. Nach der Feinreinigung (Methode 6) und Gefriertrocknung wird die Titelverbindung (5.3 mg, 67% d. Th.) als amorpher Feststoff erhalten.

HPLC (Methode 15): Rₜ = 15.43 min.

LC-MS (Methode 10): Rₜ = 1.48 min; MS (ESIpos.): *m*/*z* (%) = 1320 (3) [M + H]⁺, 660 (60) [M + 2H]²⁺, 440 (100); ESIneg: *m*/*z* (%) = 1318 (78) [M - H]-, 658 (100) [M - 2H]²⁻.

HR-TOF-MS (Methode 11): C₆₀H₁₀₃N₁₆O₁₇ ber. 1319.7682, gef. 1319.7725 [M + H]⁺.

### Beispiel 13

### N^{4.10}-(Carboxymethyl)-D-leucyl-L-leucyl-[(3R)-3-hydroxy-L-phenylalanyl]-[(3R)-3-hydroxy-L-leucyl]-L-leucyl-D-arginyl-L-isoleucyl-L-allothreonyl-glycyl-[(3S)-3-hydroxy-L-β-asparaginyl]-L-serin-C^{1.11}-O^{3.3}-lacton-bis-trifluoracetat

### (N^{1.10}-(Carboxymethyl)-lysobactin-bis-trifluoracetat)

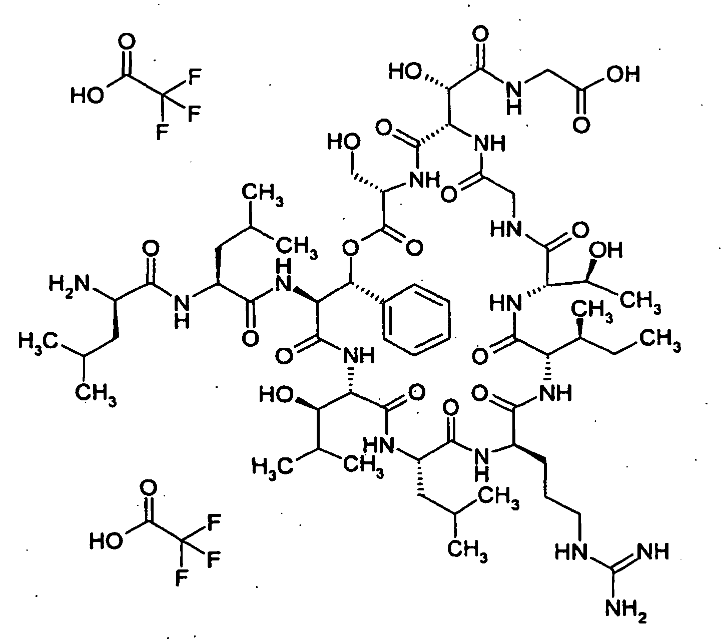

Gemäß der Arbeitsvorschrift 2 wird von der Verbindung aus Beispiel 8 (4.0 mg, 2.42 µmol) die Benzyloxycarbonyl-Schutzgruppe hydrogenolytisch abgespalten. Nach der Feinreinigung (Methode 6) und Gefriertrocknung wird die Titelverbindung (1.7 mg, 45% d. Th.) als amorpher Feststoff erhalten.

HPLC (Methode 15): Rₜ = 15.88 min.

LC-MS (Methode 10): Rₜ = 1.66 min; MS (ESIpos.): *m*/*z* (%) = 1335 (4) [M + H]⁺, 668 (100) [M + 2H]²⁺; ESIneg: *m*/*z* = 1333 (32) [M - H]-, 666 (100) [M - 2H]²⁻.

HR-TOF-MS (Methode 11): C₆₀H₁₀₀N₁₅O₁₉ ber. 1334.7315, gef. 1334.7316 [M + H]⁺.

### B. Bewertung der physiologischen Wirksamkeit

Die *in vitro*-Wirkung der erfindungsgemäßen Verbindungen kann in folgenden Assays gezeigt werden:

### Bestimmung der minimalen Hemmkonzentration (MHK):

Die MHK wird im Flüssigdilutionstest gemäß der NCCLS-Richtlinien bestimmt. Übernachtkulturen von *Staphylococcus aureus* 133, *Enterococcus faecalis* 27159, *E. faecium* 4147 und *Streptococcus pneumoniae* G9a werden mit den beschriebenen Testsubstanzen in einer 1:2 Verdünnungsreihe inkubiert. Die MHK-Bestimmung wird mit einer Zellzahl von 10⁵ Keimen pro ml in Isosensitest-Medium (Fa. Difco, Irvine/USA) durchgeführt, mit Ausnahme von *S. pneumoniae,* der in BHI-Bouillon (Fa. Difco, Irvine/USA) mit 10% Rinderserum bei einer Zellzahl von 10⁶ Keimen pro ml getestet wird. Die Kulturen werden bei 37°C für 18-24 Stunden inkubiert, *S. pneumoniae* in Gegenwart von 10% CO₂.

Die jeweils niedrigste Substanzkonzentration, bei der kein sichtbares Bakterienwachstum mehr auftritt, wird als MHK definiert. Die MHK-Werte werden in µg/ml angegeben.

Repräsentative in-vitro-Wirkdaten für die erfindungsgemäßen Verbindungen sind in Tabelle A wiedergegeben:

**Tabelle A**

| **Beispiel- Nr.** | **MHK *S. aureus* 133 [µg/ml]** | **MHK *S. pneumoniae G9a* [µg/ml]** | **MHK *E. faecalis ICB 27159* [µg/ml]** |
|---|---|---|---|
| **2** | 1 | 2 | 16 |
| **3** | 0.25 | 0.5 | 2 |
| **8** | 0.5 | 2 | 16 |
| **9** | 0.5 | 2 | 16 |
| **10** | 0.25 | 1 | 2 |
| **12** | 0.5 | 2 | 8 |

Die Eignung der erfindungsgemäßen Verbindungen zur Behandlung von bakteriellen Infektionen kann im folgenden Tiermodell gezeigt werden:

### Systemische Infektion mit Staphylococcus aureus 133:

Zellen von *S. aureus* 133 werden über Nacht in BHI-Bouillon (Fa. Oxoid, New York/USA) angezüchtet. Die Übernachtkultur wird 1:100 in frische BHI-Bouillon verdünnt und für 3 Stunden inkubiert. Die dann in der logarithmischen Wachstumsphase befindlichen Zellen werden abzentrifugiert und zweimal mit gepufferter, physiologischer Kochsalzlösung gewaschen. Danach wird photometrisch eine Zellsuspension in Kochsalzlösung mit einer Extinktion von 50 Einheiten eingestellt. Nach einem Verdünnungsschritt (1:15) wird diese Suspension 1:1 mit einer 10%igen Mucinlösung gemischt. Von dieser Infektionslösung werden 0.25 ml/20 g Maus intraperitoneal (entsprechend 1x10⁶ Keimen/Maus) appliziert. Die Therapie erfolgt intraperitoneal oder intravenös 30 Minuten nach der Infektion. Für den Infektionsversuch werden' weibliche CFW1-Mäuse verwendet. Das Überleben der Tiere wird über 6 Tage protokolliert.

Die Eigenschaften der erfindungsgemäßen Verbindungen im Hinblick auf die Nierenverträglichkeit können im folgenden Tiermodell gezeigt werden:

### Maus-Modell zur Bestimmung nephrotoxischer Effekte:

Nephrotoxische Nebenwirkungen der Nonadepsipeptide werden durch histopathologische Untersuchungen der Nieren in Mäusen und/oder Ratten nach mehrfacher Gabe einer bestimmten Dosierung analysiert. Dafür werden 5 bis 6 Tiere täglich entweder intravenös (i.v.) oder intraperitoneal (i.p.) mit Substanzen behandelt, die in wässriger Lösung oder unter Zusatz von Solutol gelöst werden. Nierentoxische Effekte werden durch lichtmikroskopische Auswertung Hämatoxilin und Eosin (H&E) gefärbter Paraffinschnitte der Nieren bestimmt. Eine 'Periodic-Acid Schiff' (PAS)-Reaktion wird wahlweise zur besseren Darstellung von Glycoproteinen durchgeführt. Nephrotoxische Effekte werden semiquantitativ für jedes Tier als Schweregrade der auftretenden tubulären Basophilie und Degeneration/Regeneration festgelegt (Schweregrade: 0 = kein Effekt; 1 = minimaler Effekt; 2 = leichter Effekt; 3 = moderater Effekt; 4 = schwere Läsionen). Der durchschnittliche Schweregrad der tubulären Degeneration/Regeneration sowie die Inzidenz (Anzahl der betroffenen Tiere) wird pro Tiergruppe oder Derivat berechnet. Darüber hinausgehende Nierenveränderungen wie tubuläre Dilatation sowie Nekrosen und Ansammlung nekrotischen Materials werden ebenfalls aufgeführt.

### Prinzip der Bestimmung der freien Fraktion via Transil:

Die hier beschriebene Methode zur Bestimmung der freien Fraktion (fᵤ) einer Prüfsubstanz gliedert sich in 2 Teile:
a) Bestimmung des Transil^{®}/Puffer-Verteilungsverhältnisses (MA_{buffer}) durch Inkubation der Prüfsubstanz in einer Transil^{®}-Puffer (pH 7.4)-Dispersion und anschließende Bestimmung der Konzentration in der Dispersion und im Puffer-Überstand.
b) Bestimmung des Transil^{®}/Plasma-Verteilungsverhältnisses (MAₚₗₐₛₘₐ) durch Inkubation der Prüfsubstanz in einer Transil^{®}-Plasma-Dispersion und anschließende Bestimmung der Konzentration in der Dispersion und im Plasma.

Der Quotient der beiden Verteilungsverhältnisse ergibt fᵤ.

Bei hochproteingebundenen Substanzen wird das Plasma in der Regel mit isotonischem Phosphatpuffer (pH 7.4) verdünnt und anschließend mit Transil^{®} suspendiert. Die Bestimmung von fᵤ' (freie Fraktion in verdünntem Plasma) in dieser verdünnten Proteinlösung erfolgt analog zur Bestimmung von fᵤ. Die freie Fraktion in unverdünntem Plasma wird aus tᵤ' und dem Verdünnungsfaktor berechnet.

Vergleich zu dieser Methode auch: Schuhmacher, Joachim; Kohlsdorfer, Christian; Buehner, Klaus; Brandenburger, Tim; Kruk, Renate, "High-throughput determination of the free fraction of drugs strongly bound to plasma proteins." Journal of Pharmaceutical Sciences 2004, 93, 816-830.

Repräsentative Daten aus der Bestimmung der freien Fraktion für die erfindungsgemäßen Verbindungen sind in Tabelle B wiedergegeben:

**Tabelle B**

| **Beispiel-Nr.** | **Freie Fraktion (Rattenplasma)** | **Freie Fraktion (Humanplasma)** |
|---|---|---|
| **2** | 13.7 | 9.9 |
| **3** | 12.9 | 5.1 |
| **4** | 6.8 | 2.4 |
| **6** | 7.0 | 2.6 |
| **1a (Lysobactin)** | 0.76 | 0.81 |

### Bestimmung der Membranaffinität einer Prüfsubstanz nach Verteilung zwischen Transil^{®} und Puffer (MA_{buffer}):

Alle Inkubationen werden in geeigneten Glasgefäßen, z.B. Glasvials, Schliffreagenzgläsern, durchgeführt. In der Regel beträgt das Gesamtvolumen 0.5-5 mL, das Transil^{®} Volumen 10-100 µl. Im Falle von hohen erwarteten Membranaffinitäten kann die Transil^{®} Dispersion mit Phosphatpuffer pH 7.4 z.B. Dulbeccos PBS bis zu 20fach verdünnt werden. Phosphatpuffer pH 7.4 wird in die Inkubationsgefäße vorgelegt und das Transil^{®} nach gründlichem Mischen zupipettiert. Die Prüfsubstanz wird in einer Konzentration von z.B. 200 ng/mL, n = 6, zupipettiert. Der Anteil an organischem Lösungsmittel sollte ≤2% sein. Die Ansätze werden 30 min bei Raumtemperatur z.B. auf einem Mini-Shaker im Winkel von ca. 45° bei ca. 400 UPM inkubiert. Zur Bestimmung des 100% Wertes wird mindestens ein Aliquot von z.B. 100 µl entnommen, der restliche Ansatz wird ca. 10 min bei ca. 1800 g zentrifugiert.

Von jeder Probe werden mindestens 2 Aliquote (z.B. 100 µl) des Überstandes für die Konzentrationsbestimmung entnommen.

### Bestimmung von MAₚₗₐₛₘₐ in unverdünntem oder verdünntem Plasma:

Das Gesamtinkubationsvolumen und das zugesetzte Transil^{®} Volumen hängen von der erwarteten freien Fraktion ab. In der Regel beträgt das Gesamtvolumen 0.5 -1 mL, das Transil^{®} Volumen 10-100 µl. Im Falle von sehr niedrigen freien Fraktionen wird das Plasma der zu untersuchenden Spezies mit isotonischer Pufferlösung, pH 7.4 z.B. 10 -400fach verdünnt und anschließend mit Transil^{®} versetzt. Das weitere Vorgehen erfolgt wie oben für die Bestimmung der MA_{buffer} Werte beschrieben.

### Prinzip der Bestimmung der freien Fraktion via Ultrafiltration:

Das Plasma der zu untersuchenden Spezies wird über eine semipermeable Membran filtriert. Die Substanzkonzentration im Filtrat wird gemessen und daraus die freie Fraktion fᵤ berechnet. Das Centrifree micropartition system von Millipore/Amicon wird verwendet. Die Ultrafiltrationsmembranen haben eine Ausschlussgröße von 30 000 Da. Die Substanz wird zu 1 mL Plasma in einer Konzentration von ca. 1 µg/mL zudotiert. Der Lösungsmittelanteil sollte < 2 % sein. Nach 30 minütiger Inkubation bei Raumtemperatur wird das Plasma in das Ultrafiltrationssystem pipettiert und 10 Minuten bei 1800 g zentrifugiert. Die Substanzkonzentration im Ultrafiltrat (C_{u;} ungebundene Substanzkonzentration) und im Plasma vor der Zentrifugation (C; Gesamtsubstanzkonzentration) wird gemessen. Die freie Fraktion errechnet sich nach der Formel: fᵤ (%) = Cᵤ/C *100.

**Die Löslichkeit** einer Verbindung wird nach dem Fachmann bekannten Methoden bestimmt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung;

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung;

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

100-200 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 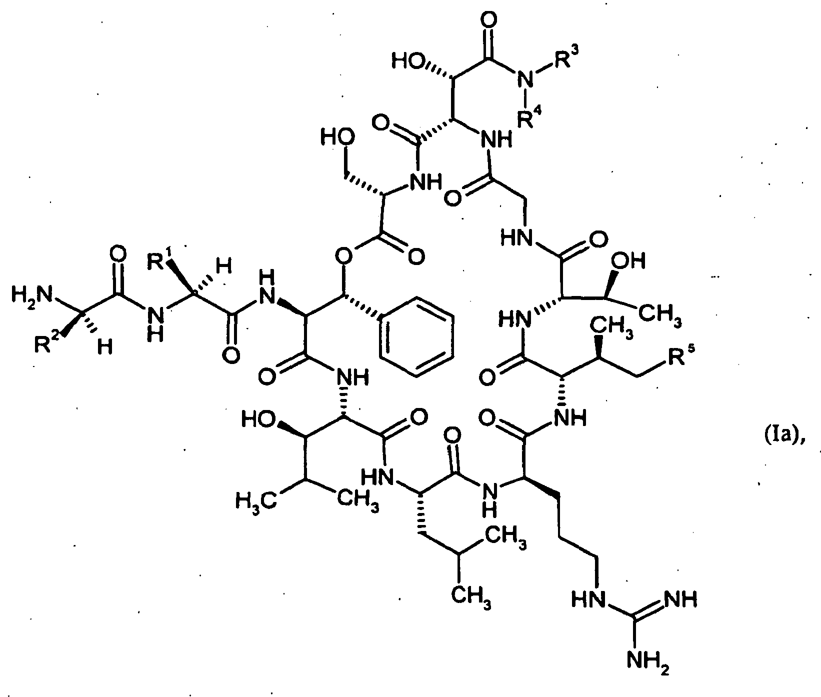 in welcher
R¹ 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
R² 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
R³ C₁-C₆-Alkyl bedeutet,
wobei Alkyl substituiert sein kann mit 1 bis 3 Substituenten, wobei die Substituenten unabhängig voneinander ausgewählt werden aus der Gruppe, bestehend aus Hydroxy, Amino, Hydroxycarbonyl, Aminocarbonyl, {[Bis(dimethylamino)methylen]amino}ethoxy, Phenyl, 5- oder 6-gliedriges Heterocyclyl, 5- oder 6-gliedriges Heteroaryl, Benzyloxycarbonyl und Benzyloxycarbonylamino,
R⁴ Wasserstoff, C₁-C₄-Alkyl, Cyclopropyl oder Cyclopropylmethyl bedeutet,
R⁵ Wasserstoff oder Methyl bedeutet,
oder eines ihrer Salze, ihrer Solvate oder der Solvate ihrer Salze.

2. Verbindung nach Anspruch 1, **dadurch gekennzeichnet, dass** sie der Formel 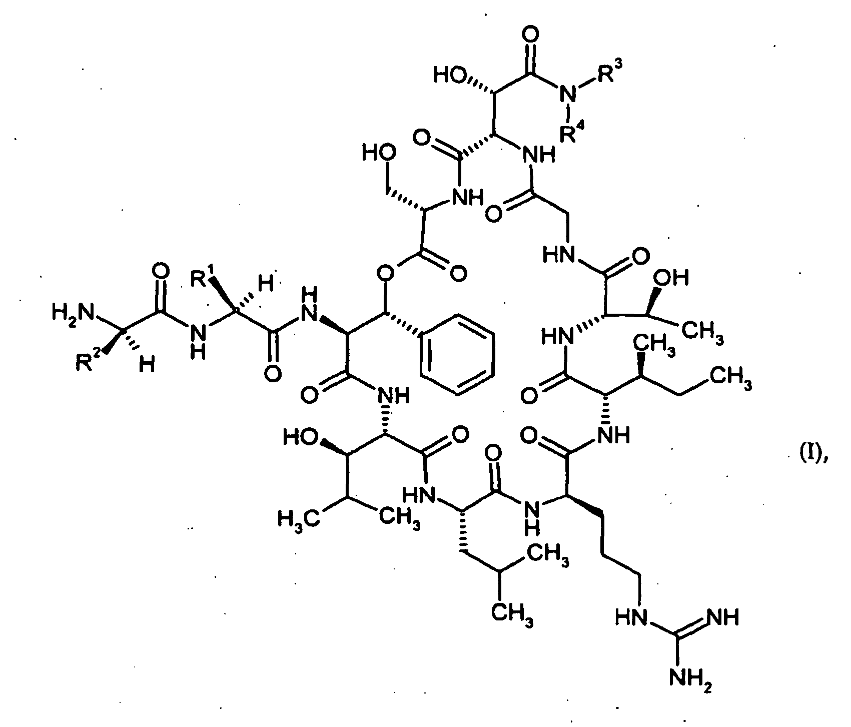 entspricht,
in welcher
R¹ 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
R² 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
R³ C₁-C₆-Alkyl bedeutet,
wobei Alkyl substituiert sein kann mit 1 bis 3 Substituenten, wobei die Substituenten unabhängig voneinander ausgewählt werden aus der Gruppe, bestehend aus Hydroxy, Amino, Hydroxycarbonyl, Aminocarbonyl, {[Bis(dimethylamino)methylen]amino}ethoxy, Phenyl, 5- oder 6-gliedriges Heterocyclyl, 5- oder 6-gliedriges Heteroaryl, Benzyloxycarbonyl und Benzyloxycarbonylamino,
R⁴ Wasserstoff, C₁-C₄-Alkyl, Cyclopropyl oder Cyclopropylmethyl bedeutet,
oder eines ihrer Salze, ihrer Solvate oder der Solvate ihrer Salze.

3. Verbindung nach Anspruch 1 oder 2, **dadurch gekennzeichnet, dass**
R¹ 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
R² 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
R³ C₁-C₄-Alkyl bedeutet,
wobei Alkyl substituiert sein kann mit 1 bis 2 Substituenten, wobei die Substituenten unabhängig voneinander ausgewählt werden aus der Gruppe, bestehend aus Hydroxy, Amino, Hydroxycarbonyl, Aminocarbonyl, {[Bis(dimethylamino)methylen]amino}ethoxy, Phenyl, Morpholin-4-yl, Pyrid-2-yl, Pyrid-3-yl, Pyrid-4-yl, Benzyloxycarbonyl und Benzyloxycarbonylamino,
R⁴ Wasserstoff oder Methyl bedeutet,
oder eines ihrer Salze, ihrer Solvate oder der Solvate ihrer Salze.

4. Verbindung nach einem der Ansprüche 1 bis 3, **dadurch gekennzeichnet, dass**
R¹ 2-Methylprop-1-yl bedeutet,
R² 2-Methylprop-1-yl bedeutet,
R³ C₁-C₃-Alkyl bedeutet,
wobei Alkyl substituiert sein kann mit einem Substituenten, wobei der Substituent ausgewählt wird aus der Gruppe, bestehend aus Hydroxy, Amino, Hydroxycarbonyl, Aminocarbonyl, {[Bis(dimethylamino)methylen]amino}ethoxy, Phenyl, Morpholin-4-yl, Pyrid-3-yl, Benzyloxycarbonyl und Benzyloxycarbonylamino,
R⁴ Wasserstoff oder Methyl bedeutet,
oder eines ihrer Salze, ihrer Solvate oder der Solvate ihrer Salze.

5. Verbindung nach einem der Ansprüche 1 bis 3, **dadurch gekennzeichnet, dass**
R¹ 2,2-Dimethylprop-1-yl bedeutet,
R² 2,2-Dimethylprop-1-yl bedeutet,
R³ C₁-C₃-Alkyl bedeutet,
wobei Alkyl substituiert sein kann mit einem Substituenten, wobei der Substituent ausgewählt wird aus der Gruppe, bestehend aus Hydroxy, Amino, Hydroxycarbonyl, Aminocarbonyl, {[Bis(dimethylamino)methylen]amino}ethoxy, Phenyl, Morpholin-4-yl, Pyrid-3-yl, Benzyloxycarbonyl und Benzyloxycarbonylamino,
R⁴ Wasserstoff oder Methyl bedeutet,
oder eines ihrer Salze, ihrer Solvate oder der Solvate ihrer Salze.

6. Verfahren zur Herstellung einer Verbindung der Formel (la) nach Anspruch 1, **dadurch gekennzeichnet, dass** eine Verbindung der Formel 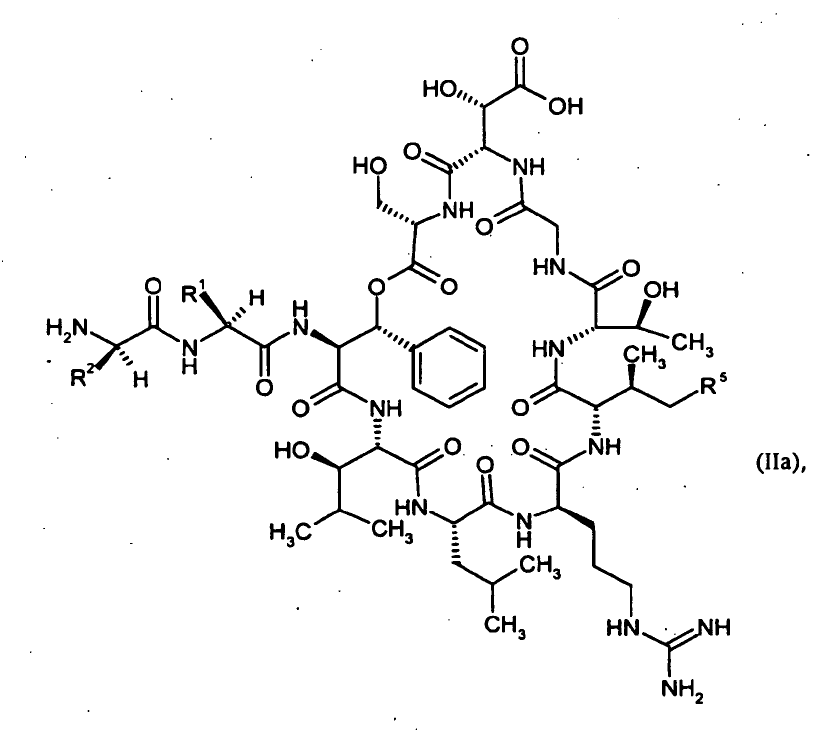 in welcher
R¹, R² und R⁵ die in Anspruch 1 angegebene Bedeutung haben,
mit einer Verbindung der Formel 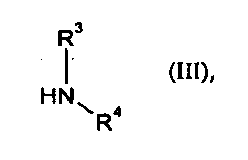 in welcher
R³ und R⁴ die in Anspruch 1 angegebene Bedeutung haben,
umgesetzt wird.

7. Verbindung der Formel 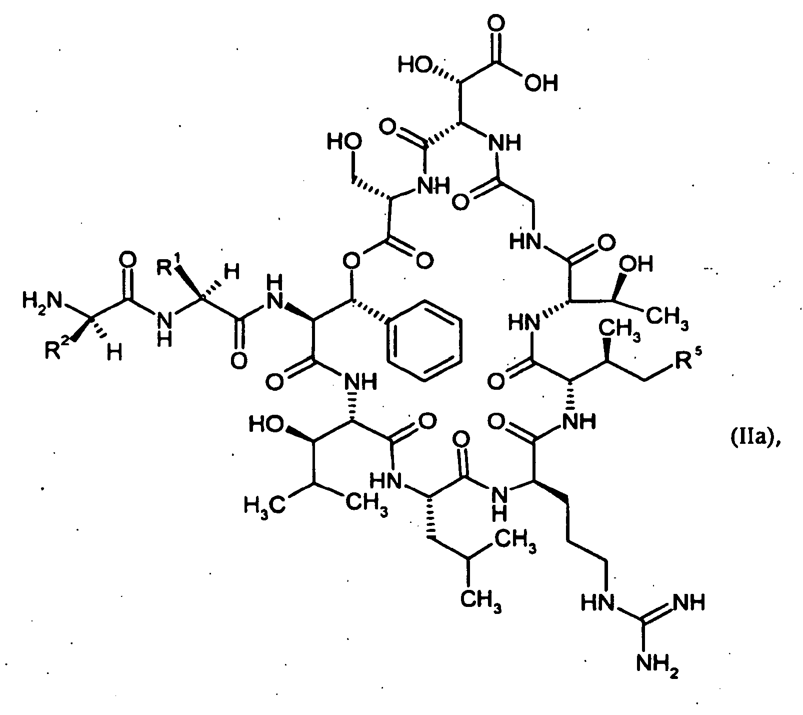 in welcher
R¹ 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
R² 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
R⁵ Wasserstoff oder Methyl bedeutet,
oder eines ihrer Salze, ihrer Solvate oder der Solvate ihrer Salze.

8. Verbindung nach Anspruch 7, **dadurch gekennzeichnet, dass** sie der Formel 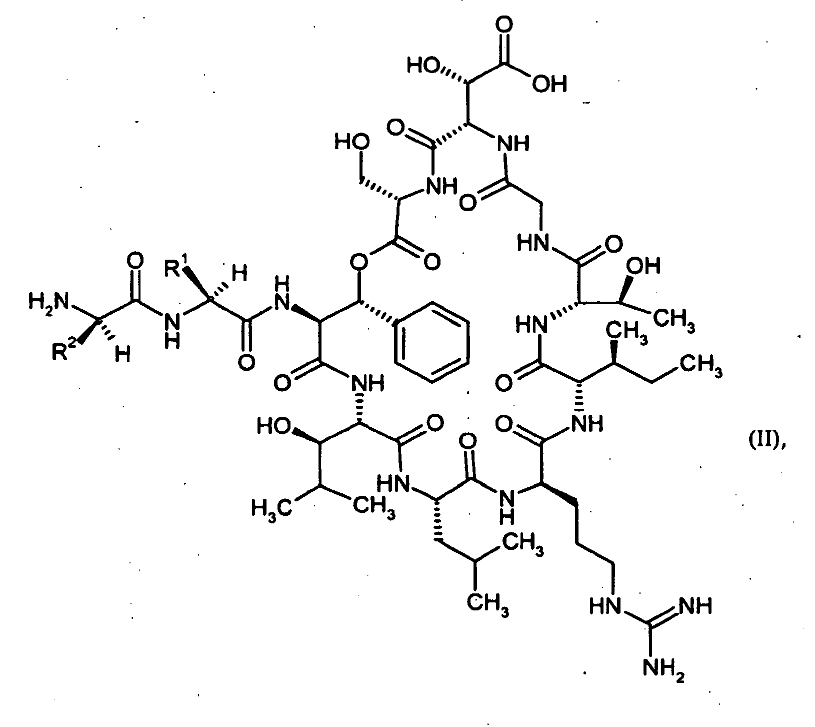 entspricht,
in welcher
R¹ 2-Methylprop-1-yl, 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
R² 2-Methylprop-1-yl, 2,2-Dimethylprop-1-yl, 2,2-Dimethylbut-1-yl, 2-Ethyl-2-methylbut-1-yl, 2,2-Diethylbut-1-yl, 2,2-Dimethylpent-1-yl oder 1-Trimethylsilylmethyl bedeutet,
oder eines ihrer Salze, ihrer Solvate oder der Solvate ihrer Salze.

9. Verbindung nach Anspruch 7 oder 8, **dadurch gekennzeichnet, dass**
R¹ 2-Methylprop-1-yl bedeutet,
R² 2-Methylprop-1-yl bedeutet,
oder eines ihrer Salze, ihrer Solvate oder der Solvate ihrer Salze.

10. Verbindung nach Anspruch 7 oder 8, **dadurch gekennzeichnet, dass**
R¹ 2,2-Dimethylprop-1-yl bedeutet,
R² 2,2-Dimethylprop-1-yl bedeutet,
oder eines ihrer Salze, ihrer Solvate oder der Solvate ihrer Salze.

11. Verfahren zur Herstellung einer Verbindung der Formel (IIa) nach Anspruch 7, **dadurch gekennzeichnet, dass** eine Verbindung der Formel 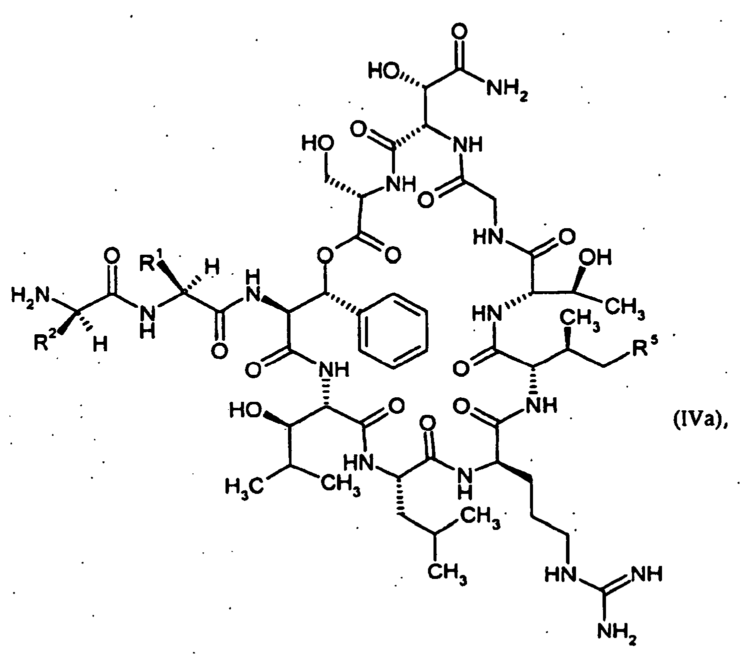 in welcher
R¹, R² und R⁵ die in Anspruch 7 angegebene Bedeutung haben,
mit einer Säure in einem geeigneten Lösungsmittel hydrolysiert wird.

12. Verbindung nach einem der Ansprüche 1 bis 5 zur Behandlung und/oder Prophylaxe von Krankheiten.

13. Verwendung einer Verbindung nach einem der Ansprüche 1 bis 5 zur Herstellung eines Arzneimittels zur Behandlung und/oder Prophylaxe von bakteriellen Infektionen.

14. Arzneimittel enthaltend eine Verbindung nach einem der Ansprüche 1 bis 5 in Kombination mit einem inerten, nichttoxischen, pharmazeutisch geeigneten Hilfsstoff.

15. Arzneimittel nach Anspruch 14 zur Behandlung und/oder Prophylaxe von bakteriellen Infektionen.

## Claims

1. Compound of formula 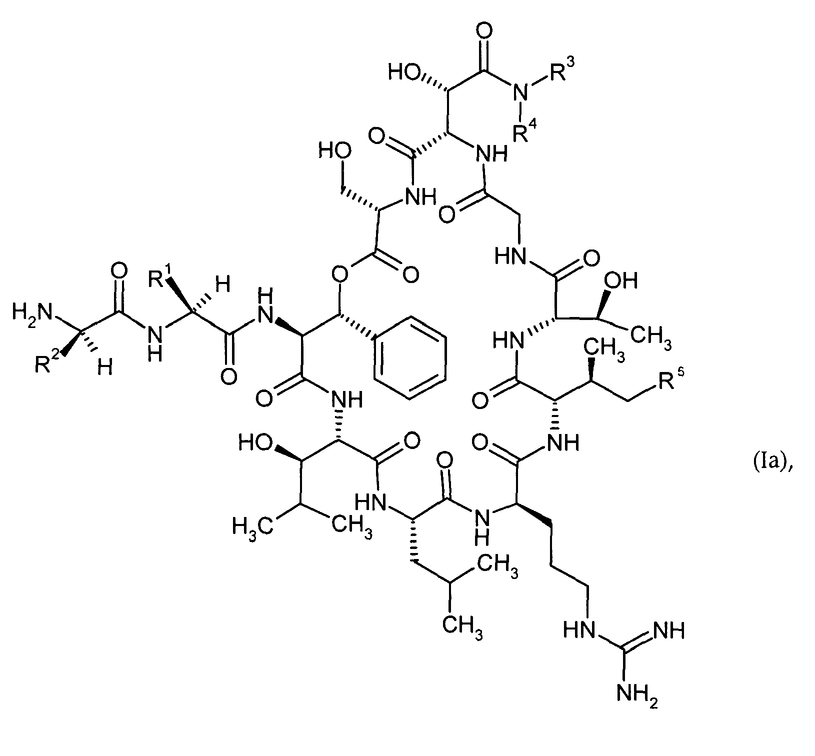 in which
R¹ represents 2-methylprop-1-yl, 2,2-dimethylprop-1-yl, 1-trimethylsilylmethyl or 3-pyridylmethyl,
whereby 3-pyridylmethyl may be substituted with a trifluoromethyl substituent,
R² represents 2-methylprop-1-yl, 2,2-dimethylprop-1-yl, 2,2-dimethylbut-1-yl, 2-ethyl-2-methylbut-1-yl, 2,2-diethylbut-1-yl, 2,2-dimethylpent-1-yl or 1-trimethylsilylmethyl,
R³ represents C₁-C₆-alkyl,
whereby alkyl may be substituted with 1 to 3 substituents, whereby the substituents are selected independently of one another from the group consisting of hydroxy, amino, hydroxycarbonyl, aminocarbonyl, {[bis-(dimethylamino)methylene]amino}ethoxy, phenyl, 5- or 6-membered heterocyclyl, 5- or 6-membered heteroaryl, benzyloxycarbonyl and benzyloxycarbonylamino,
R⁴ represents hydrogen, C₁-C₄-alkyl, cyclopropyl or cyclopropylmethyl,
R⁵ represents hydrogen or methyl,
or one of the salts thereof, the solvates thereof or the solvates of the salts thereof.

2. Compound according to claim 1, **characterized in that** it corresponds to formula 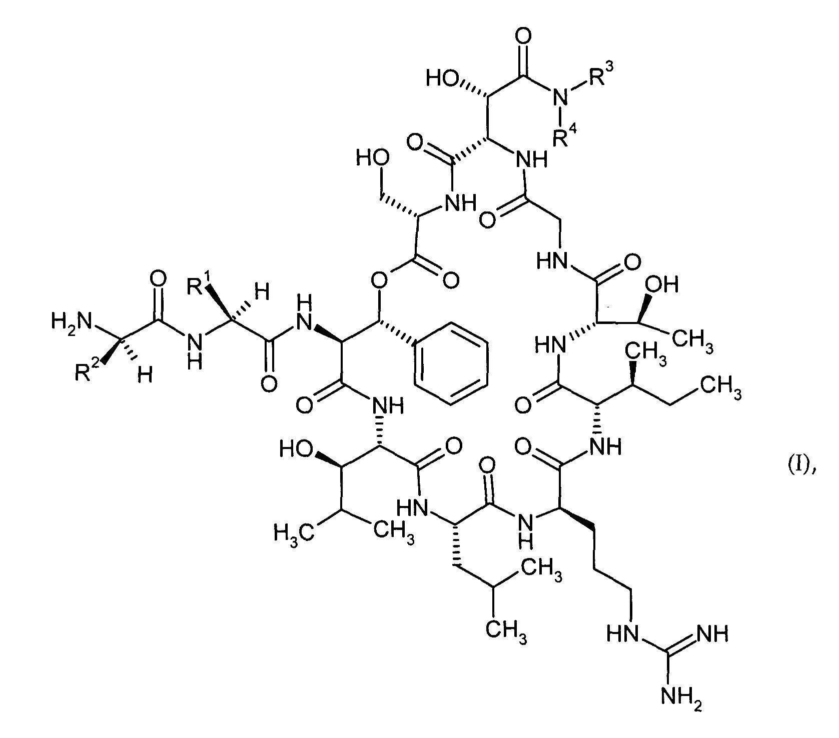 in which
R¹ represents 2-methylprop-1-yl, 2,2-dimethylprop-1-yl, 1-trimethylsilylmethyl or 3-pyridylmethyl,
whereby 3-pyridylmethyl may be substituted with a trifluoromethyl substituent,
R² represents 2-methylprop-1-yl, 2,2-dimethylprop-1-yl, 2,2-dimethylbut-1-yl, 2-ethyl-2-methylbut-1-yl, 2,2-diethylbut-1-yl, 2,2-dimethylpent-1-yl or 1-trimethylsilylmethyl,
R³ represents C₁-C₆-alkyl,
whereby alkyl may be substituted with 1 to 3 substituents, whereby the substituents are selected independently of one another from the group consisting of hydroxy, amino, hydroxycarbonyl, aminocarbonyl, {[bis-(dimethylamino)methylene]amino}ethoxy, phenyl, 5- or 6-membered heterocyclyl, 5- or 6-membered heteroaryl, benzyloxycarbonyl and benzyloxycarbonylamino,
R⁴ represents hydrogen, C₁-C₄-alkyl, cyclopropyl or cyclopropylmethyl,
or one of the salts thereof, the solvates thereof or the solvates of the salts thereof.

3. Compound according to claim 1 or 2, **characterized in that**
R¹ represents 2-methylprop-1-yl, 2,2-dimethylprop-1-yl, 1-trimethylsilylmethyl or 3-pyridylmethyl,
whereby 3-pyridylmethyl may be substituted with a trifluoromethyl substituent,
R² represents 2-methylprop-1-yl, 2,2-dimethylprop-1-yl, 2,2-dimethylbut-1-yl, 2-ethyl-2-methylbut-1-yl, 2,2-diethylbut-1-yl, 2,2-dimethylpent-1-yl or 1-trimethylsilylmethyl,
R³ represents C₁-C₄-alkyl,
whereby alkyl may be substituted with 1 to 2 substituents, whereby the substituents are selected independently of one another from the group consisting of hydroxy, amino, hydroxycarbonyl, aminocarbonyl, {[bis-(dimethylamino)methylene]amino}ethoxy, phenyl, morpholin-4-yl, pyrid-2-yl, pyrid-3-yl, pyrid-4-yl, benzyloxycarbonyl and benzyloxycarbonylamino,
R⁴ represents hydrogen or methyl,
or one of the salts thereof, the solvates thereof or the solvates of the salts thereof.

4. Compound according to any one of claims 1 to 3, **characterized in that**
R¹ represents 2-methylprop-1-yl,
R² represents 2-methylprop-1-yl,
R³ represents C₁-C₃-alkyl,
whereby alkyl may be substituted with a substituent, whereby the substituent is selected from the group consisting of hydroxy, amino, hydroxycarbonyl, aminocarbonyl, {[bis(dimethylamino)methylene]amino}ethoxy, phenyl, morpholin-4-yl, pyrid-3-yl, benzyloxycarbonyl and benzyloxycarbonylamino,
R⁴ represents hydrogen or methyl,
or one of the salts thereof, the solvates thereof or the solvates of the salts thereof.

5. Compound according to any one of claims 1 to 3, **characterized in that**
R¹ represents 2,2-dimethylprop-1-yl,
R² represents 2,2-dimethylprop-1-yl,
R³ represents C₁-C₃-alkyl,
whereby alkyl may be substituted with a substituent, whereby the substituent is selected from the group consisting of hydroxy, amino, hydroxycarbonyl, aminocarbonyl, {[bis(dimethylamino)methylene]amino}ethoxy, phenyl, morpholin-4-yl, pyrid-3-yl, benzyloxycarbonyl and benzyloxycarbonylamino,
R⁴ represents hydrogen or methyl,
or one of the salts thereof, the solvates thereof or the solvates of the salts thereof.

6. Method for preparing a compound of formula (Ia) according to claim 1, **characterized in that** a compound of formula 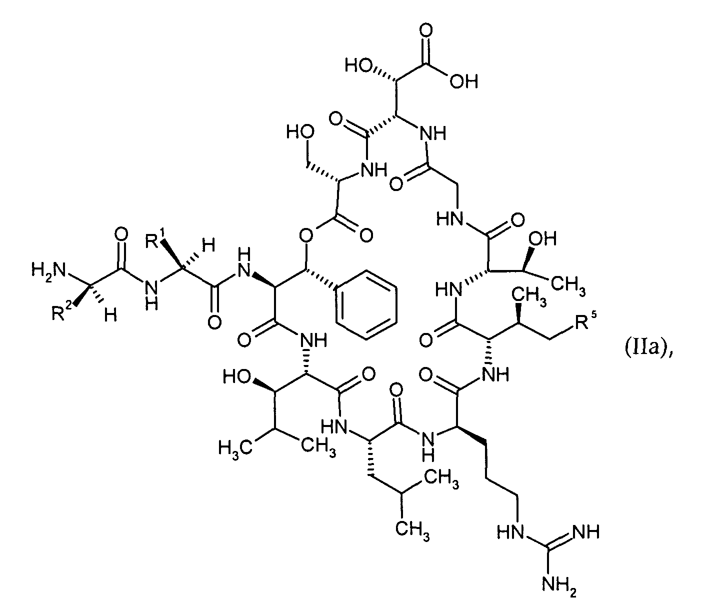 in which
R¹, R² and R⁵ have the meaning indicated in claim 1, is reacted with a compound of formula 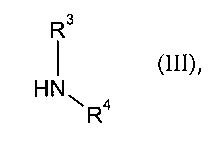 in which
R³ and R⁴ have the meaning indicated in claim 1.

7. Compound of formula 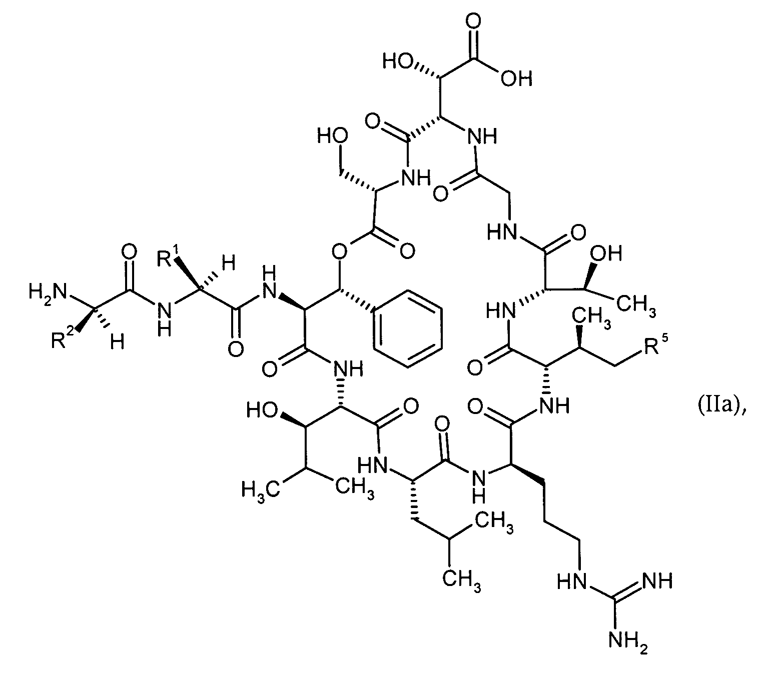 in which
R¹ represents 2-methylprop-1-yl, 2,2-dimethylprop-1-yl, 1-trimethylsilylmethyl or 3-pyridylmethyl,
whereby 3-pyridylmethyl may be substituted with a trifluoromethyl substituent,
R² represents 2-methylprop-1-yl, 2,2-dimethylprop-1-yl, 2,2-dimethylbut-1-yl, 2-ethyl-2-methylbut-1-yl, 2,2-diethylbut-1-yl, 2,2-dimethylpent-1-yl or 1-trimethylsilylmethyl,
R⁵ represents hydrogen or methyl,
or one of the salts thereof, the solvates thereof or the solvates of the salts thereof.

8. Compound according to claim 7, **characterized in that** it corresponds to formula 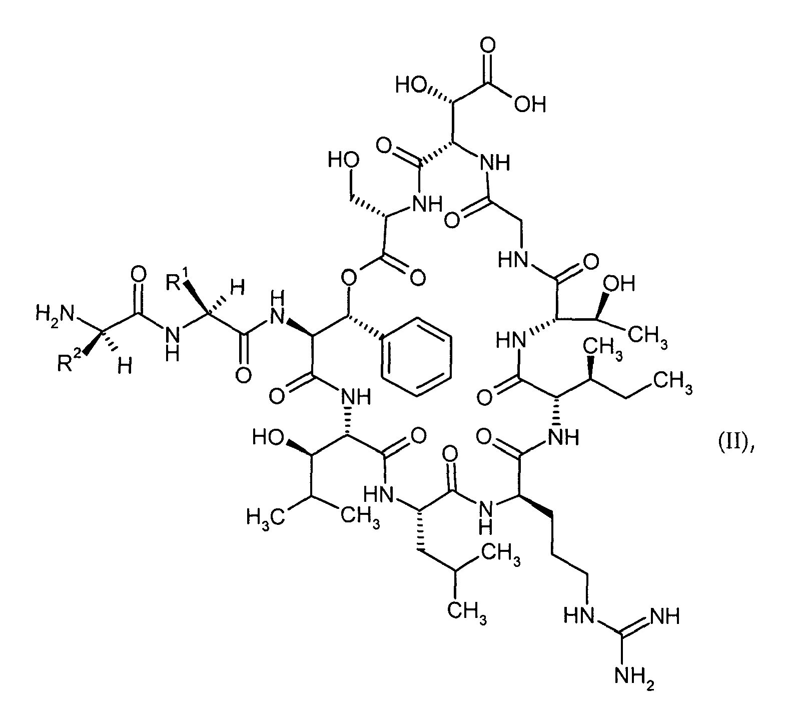 in which
R¹ represents 2-methylprop-1-yl, 2,2-dimethylprop-1-yl, 1-trimethylsilylmethyl or 3-pyridylmethyl,
whereby 3-pyridylmethyl may be substituted with a trifluoromethyl substituent,
R² represents 2-methylprop-1-yl, 2,2-dimethylprop-1-yl, 2,2-dimethylbut-1-yl, 2-ethyl-2-methylbut-1-yl, 2,2-diethylbut-1-yl, 2,2-dimethylpent-1-yl or 1-trimethylsilylmethyl,
or one of the salts thereof, the solvates thereof or the solvates of the salts thereof.

9. Compound according to claim 7 or 8, **characterized in that**
R¹ represents 2-methylprop-1-yl,
R² represents 2-methylprop-1-yl,
or one of the salts thereof, the solvates thereof or the solvates of the salts thereof.

10. Compound according to claim 7 or 8, **characterized in that**
R¹ represents 2,2-dimethylprop-1-yl,
R² represents 2,2-dimethylprop-1-yl,
or one of the salts thereof, the solvates thereof or the solvates of the salts thereof.

11. Method for preparing a compound of formula (IIa) according to claim 7, **characterized in that** a compound of formula 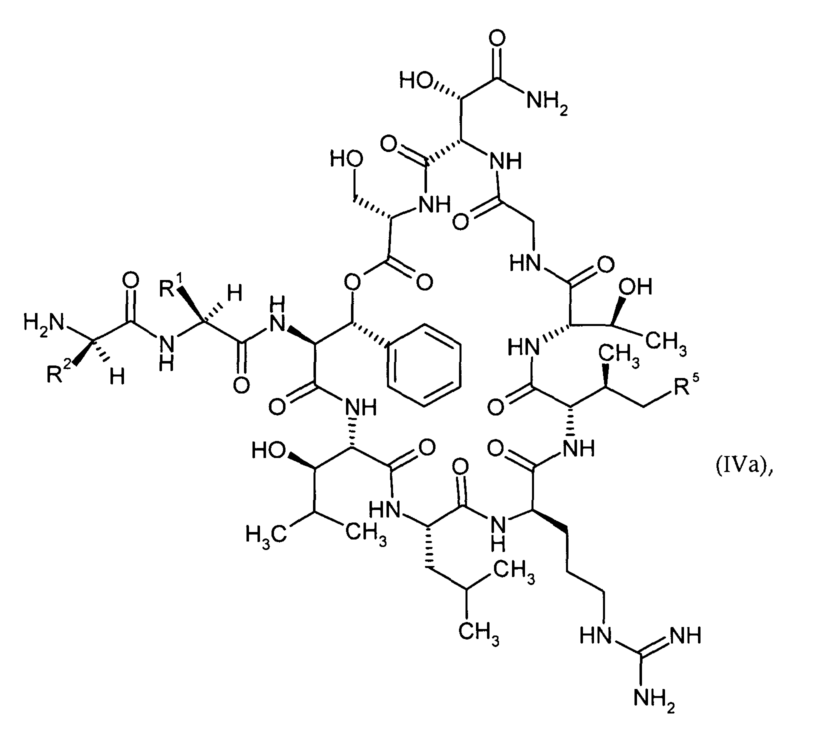 in which
R¹, R² and R⁵ have the meaning indicated in claim 7,
is hydrolyzed with an acid in a suitable solvent.

12. Compound according to any one of claims 1 to 5 for the treatment and/or prophylaxis of diseases.

13. Use of a compound according to any one of claims 1 to 5 for manufacturing a medicament for the treatment and/or prophylaxis of bacterial infections.

14. Medicament comprising a compound according to any one of claims 1 to 5 in combination with an inert, nontoxic, pharmaceutically acceptable excipient.

15. Medicament according to claim 14 for the treatment and/or prophylaxis of bacterial infections.

## Revendications

1. Composé de formule 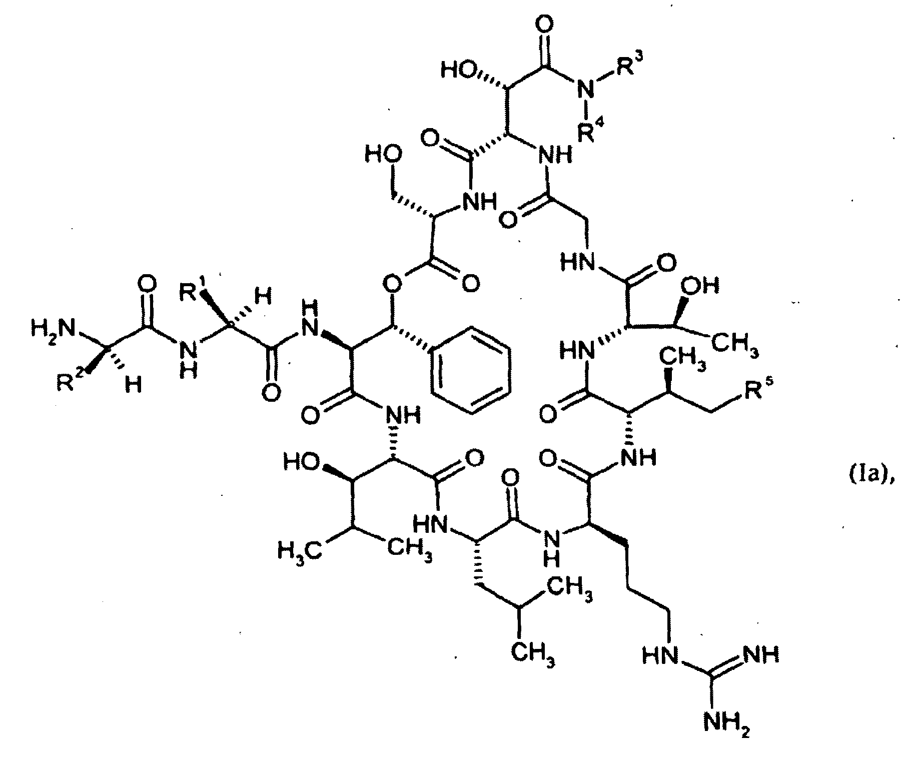 dans laquelle
R¹ est un groupe 2-méthylprop-1-yle, 2,2-diméthylprop-1-yle, 1-triméthylsilylméthyle ou 3-pyridylméthyle,
le groupe 3-pyridylméthyle pouvant être substitué par un substituant trifluorométhyle,
R² est un groupe 2-méthylprop-1-yle, 2,2-diméthylprop-1-yle, 2,2-diméthylbut-1-yle, 2-éthyl-2-méthylbut-1-yle, 2,2-diéthylbut-1-yle, 2,2-diméthylpent-1-yle ou 1-triméthylsilylméthyle,
R³ est un groupe alkyle en C₁ à C₆,
le groupe alkyle pouvant être substitué par 1 à 3 substituants, les substituants étant choisis indépendamment les uns des autres dans le groupe comprenant les groupes hydroxy, amino, hydroxycarbonyle, aminocarbonyle, {[bis(diméthylamino)méthylène]amino}éthoxy, phényle, hétérocyclyle comportant 5 ou 6 chaînons, hétéroaryle comportant 5 ou 6 chaînons, benzyloxycarbonyle et benzyloxycarbonylamino,
R⁴ est un atome d'hydrogène, un groupe alkyle en C₁ à C₄, cyclopropyle ou cyclopropylméthyle,
R⁵ est un atome d'hydrogène ou un groupe méthyle,
ou l'un de leurs sels, solvates ou solvate de leurs sels.

2. Composé selon la revendication 1, **caractérisé en ce qu'**il correspond à la formule 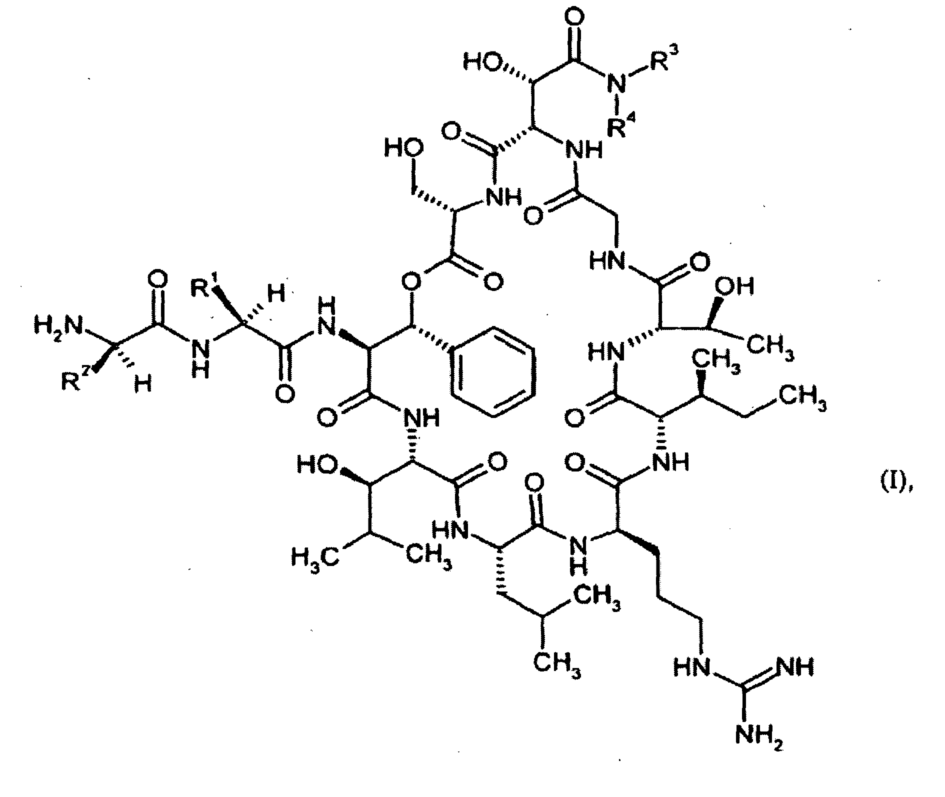 dans laquelle R¹ est un groupe 2-méthylprop-1-yle, 2,2-diméthylprop-1-yle, 1-triméthylsilylméthyle ou 3-pyridylméthyle,
le groupe 3-pyridylméthyle pouvant être substitué par un substituant trifluorométhyle,
R² est un groupe 2-méthylprop-1-yle, 2,2-diméthylprop-1-yle, 2,2-diméthylbut-1-yle, 2-éthyl-2-méthylbut-1-yle, 2,2-diéthylbut-1-yle, 2,2-diméthylpent-1-yle ou 1-triméthylsilylméthyle,
R³ est un groupe alkyle en C₁ à C₆,
le groupe alkyle pouvant être substitué par 1 à 3 substituants, les substituants étant choisis indépendamment les uns des autres dans le groupe comprenant les groupes hydroxy, amino, hydroxycarbonyle, aminocarbonyle, {[bis(diméthylamino)méthylène]amino}éthoxy, phényle, hétérocyclyle comportant 5 ou 6 chaînons, hétéroaryle comportant 5 ou 6 chaînons, benzyloxycarbonyle et benzyloxycarbonylamino,
R⁴ est un atome d'hydrogène, un groupe alkyle en C₁ à C₄, cyclopropyle ou cyclopropylméthyle,
ou l'un de leurs sels, solvates ou solvate de leurs sels.

3. Composé selon la revendication 1 ou 2, **caractérisé en ce que**
R¹ est un groupe 2-méthylprop-1-yle, 2,2-diméthylprop-1-yle, 1-triméthylsilylméthyle ou 3-pyridylméthyle,
le groupe 3-pyridylméthyle pouvant être substitué par un substituant trifluorométhyle,
R² est un groupe 2-méthylprop-1-yle, 2,2-diméthylprop-1-yle, 2,2-diméthylbut-1-yle, 2-éthyl-2-méthylbut-1-yle, 2,2-diéthylbut-1-yle, 2,2-diméthylpent-1-yle ou 1-triméthylsilylméthyle,
R³ est un groupe alkyle en C₁ à C₄,
le groupe alkyle pouvant être substitué par 1 à 2 substituants, les substituants étant choisis indépendamment les uns des autres dans le groupe comprenant les groupes hydroxy, amino, hydroxycarbonyle, aminocarbonyle, {[bis(diméthylamino)méthylène]amino}éthoxy, phényle, morpholin-4-yle, pyrid-2-yle, pyrid-3-yle, pyrid-4-yle, benzyloxycarbonyle et benzyloxycarbonylamino,
R⁴ est un atome d'hydrogène ou un groupe méthyle,
ou l'un de leurs sels, solvates ou solvate de leurs sels.

4. Composé selon l'une des revendications 1 à 3, **caractérisé en ce que**
R¹ est un groupe 2-méthylprop-1-yle,
R² est un groupe 2-méthylprop-1-yle,
R³ est un groupe alkyle en C₁ à C₃,
le groupe alkyle pouvant être substitué par un substituant, le substituant étant choisi dans le groupe comprenant les groupes hydroxy, amino, hydroxycarbonyle, aminocarbonyle, {[bis(diméthylamino)méthylène]amino}éthoxy, phényle, morpholin-4-yle, pyrid-3-yle, benzyloxycarbonyle et benzyloxycarbonylamino,
R⁴ est un atome d'hydrogène ou un groupe méthyle,
ou l'un de leurs sels, solvates ou solvates de leurs sels.

5. Composé selon l'une des revendications 1 à 3, **caractérisé en ce que**
R¹ est un groupe 2,2-diméthylprop-1-yle,
R² est un groupe 2,2-diméthylprop-1-yle,
R³ est un groupe alkyle en C₁ à C₃,
le groupe alkyle pouvant être substitué par un substituant, le substituant étant choisi dans le groupe comprenant les groupes hydroxy, amino, hydroxycarbonyle, aminocarbonyle, {[bis(diméthylamino)-méthylène]amino}éthoxy, phényle, morpholin-4-yle, pyrid-3-yle, benzyloxycarbonyle et benzyloxycarbonylamino,
R⁴ est un atome d'hydrogène ou un groupe méthyle,
ou l'un de leurs sels, solvates ou solvate de leurs sels.

6. Procédé de production d'un composé de formule (la) selon la revendication 1, **caractérisé en ce qu'**un composé de formule 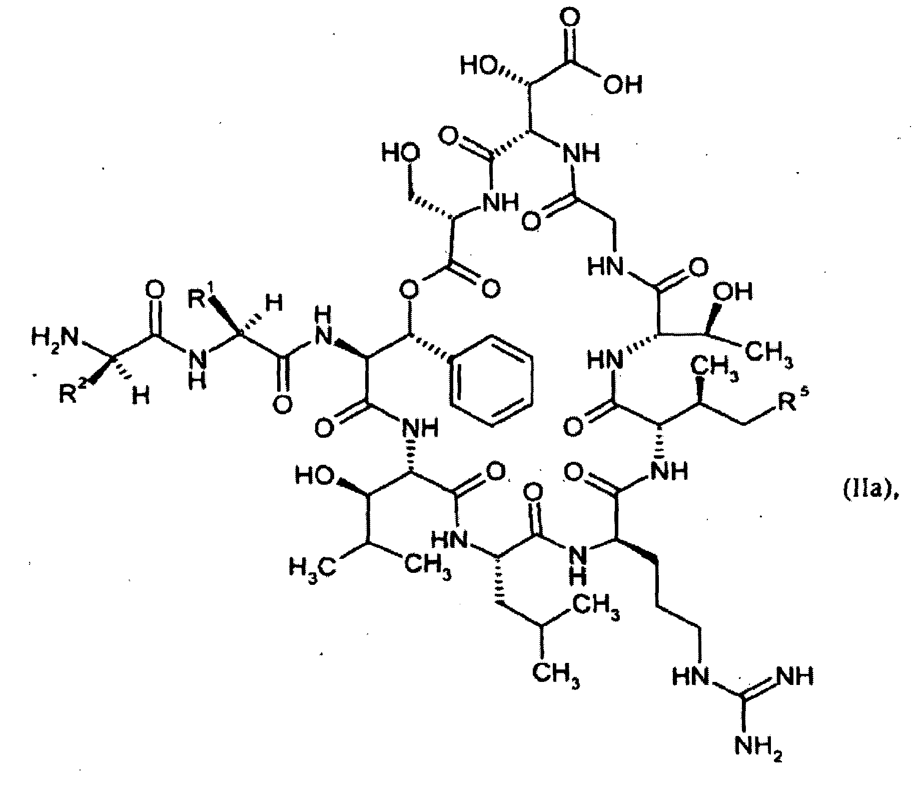 dans laquelle
R¹, R² et R⁵ sont tels que définis dans la revendication 1, est converti avec un composé de formule 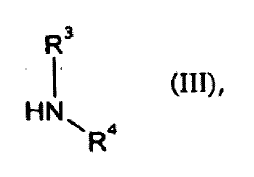 dans laquelle
R³ et R⁴ sont tels que définis dans la revendication 1.

7. Composé de formule 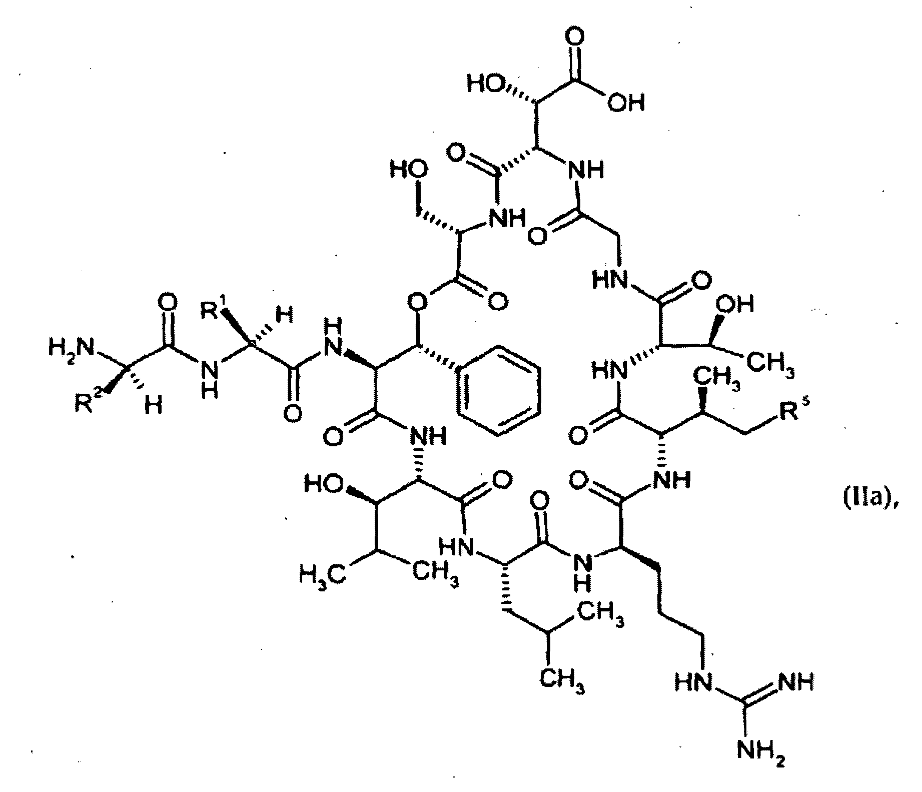 dans laquelle
R¹ est un groupe 2-méthylprop-1-yle, 2,2-diméthylprop-1-yle, 1-triméthylsilylméthyle ou 3-pyridylméthyle,
le groupe 3-pyridylméthyle pouvant être substitué par un substituant trifluorométhyle,
R² est un groupe 2-méthylprop-1-yle, 2,2-diméthylprop-1-yle, 2,2-diméthylbut-1-yle, 2-éthyl-2-méthylbut-1-yle, 2,2-diéthylbut-1-yle, 2,2-diméthylpent-1-yle ou 1-triméthylsilylméthyle,
R⁵ est un atome d'hydrogène ou un groupe méthyle,
ou l'un de leurs sels, solvates ou solvate de leurs sels.

8. Composé selon la revendication 7, **caractérisé en ce qu'**il correspond à la formule 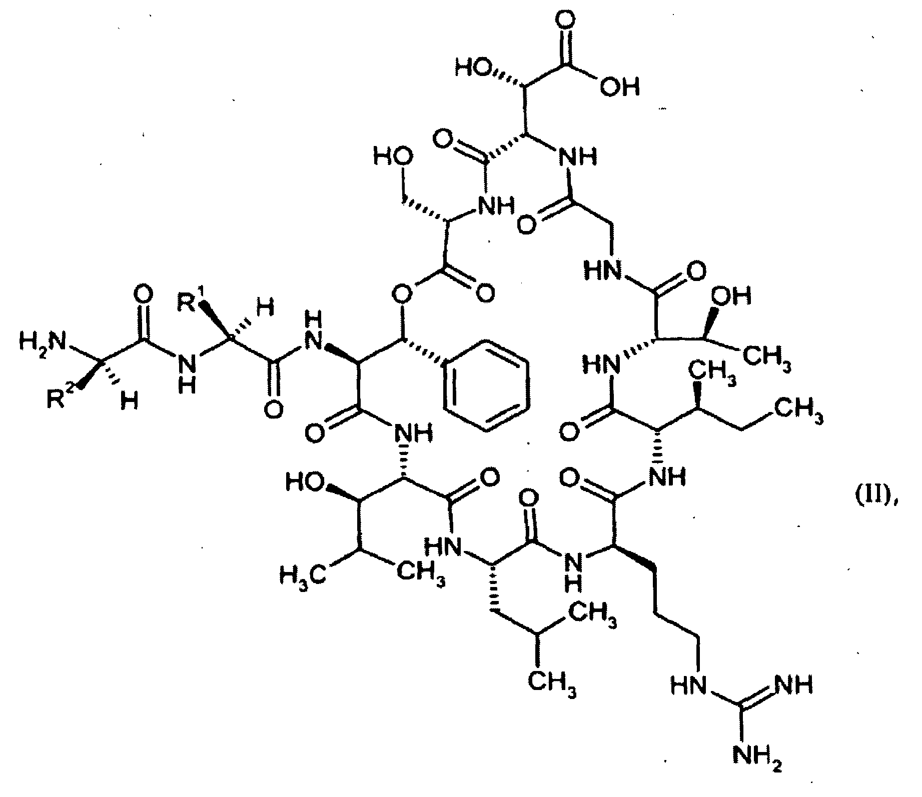 dans laquelle
R¹ est un groupe 2-méthylprop-1-yle, 2,2-diméthylprop-1-yle, 1-triméthylsilylméthyle ou 3-pyridylméthyle,
le groupe 3-pyridylméthyle pouvant être substitué par un substituant trifluorométhyle,
R² est un groupe 2-méthylprop-1-yle, 2,2-diméthylprop-1-yle, 2,2-diméthylbut-1-yle, 2-éthyl-2-méthylbut-1-yle, 2,2-diéthylbut-1-yle, 2,2-diméthylpent-1-yle ou 1-triméthylsilylméthyle,
ou l'un de leurs sels, solvates ou solvate de leurs sels.

9. Composé selon la revendication 7 ou 8, **caractérisé en ce que**
R¹ est un groupe 2-méthylprop-1-yle,
R² est un groupe 2-méthylprop-1-yle,
ou l'un de leurs sels, solvates ou solvates de leurs sels.

10. Composé selon la revendication 7 ou 8, **caractérisé en ce que**
R¹ est un groupe 2,2-diméthylprop-1-yle,
R² est un groupe 2,2-diméthylprop-1-yle,
ou l'un de leurs sels, solvates ou solvate de leurs sels.

11. Procédé de production d'un composé de formule (IIa) selon la revendication 7, **caractérisé en ce qu'**un composé de formule 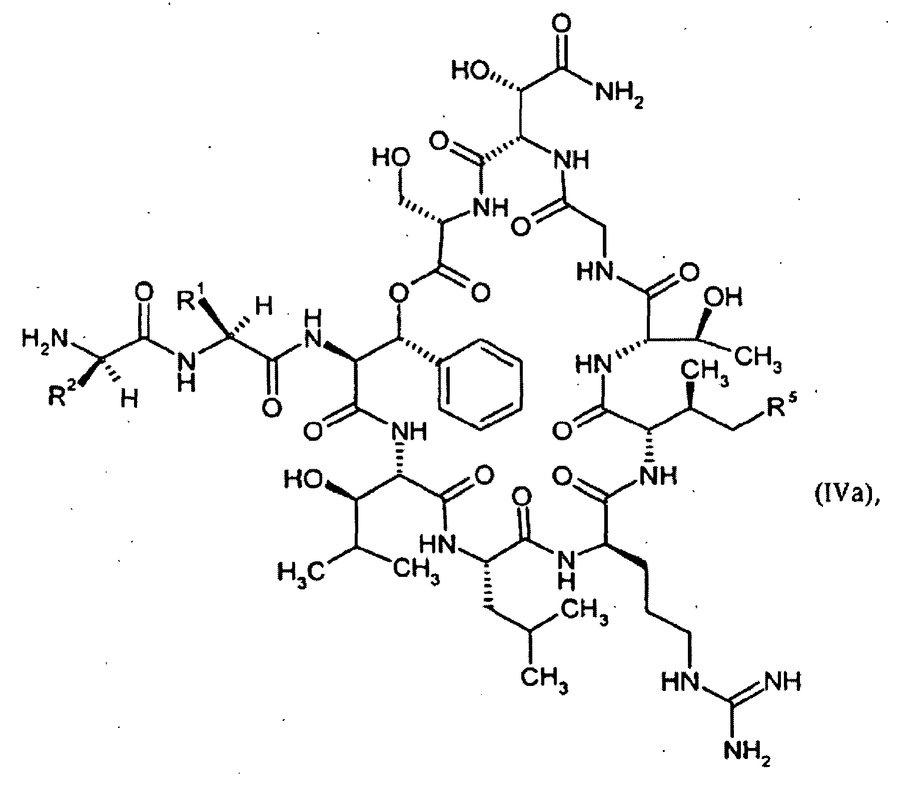 dans laquelle
R¹, R² et R⁵ sont tels que définis dans la revendication 7, est hydrolysé avec un acide dans un solvant approprié.

12. Composé selon l'une des revendications 1 à 5, pour le traitement et/ou la prophylaxie de maladies.

13. Utilisation d'un composé selon l'une des revendications 1 à 5, pour la production d'un médicament destiné au traitement et/ou à la prophylaxie d'infections bactériennes.

14. Médicament contenant un composé selon l'une des revendications 1 à 5, en combinaison avec un adjuvant inerte, non toxique, pharmaceutiquement approprié.

15. Médicament selon la revendication 14, pour le traitement et/ou la prophylaxie d'infections bactériennes.
